(19) Europäisches Patentamt
European Patent Office
Office européen des brevets

(11) Publication number: **0 497 091 A1**

# (12) EUROPEAN PATENT APPLICATION

(21) Application number: **92100095.6**

(22) Date of filing: **05.01.92**

(51) Int. Cl.5: **C07D 493/08**, A61K 31/34, C12P 17/18, C07H 19/01, C12P 19/02, C07C 57/03, A61K 31/71, //(C07D493/08, 319:00,307:00)

The microorganism(s) has (have) been deposited with IMI under number(s) IMI 332962.

(30) Priority: **09.01.91 GB 9100428**
**09.01.91 GB 9100429**
**09.01.91 GB 9100430**
**09.01.91 GB 9100418**
**01.08.91 GB 9116600**
**02.08.91 GB 9116688**
**07.08.91 GB 9117109**

(43) Date of publication of application: **05.08.92 Bulletin 92/32**

(84) Designated Contracting States: **AT BE CH DE DK ES FR GB GR IT LI LU MC NL PT SE**

(71) Applicant: **GLAXO GROUP LIMITED**
**Glaxo House, Berkeley Avenue**
**Greenford, Middlesex UB6 0NN(GB)**

(72) Inventor: **Sidebottom, Philip James**
**Glaxo Group Research Limited, Berkeley Avenue**
**Greenford, Middlesex UB6 0HE(GB)**
Inventor: **Lester, Michael George**
**Glaxo Group Research Limited, Berkeley Avenue**
**Greenford, Middlesex UB6 0HE(GB)**
Inventor: **Procopiou, Panayiotis Alexandrou**
**Glaxo Group Research Limited, Berkeley Avenue**
**Greenford, Middlesex UB6 0HE(GB)**
Inventor: **Watson, Nigel Stephen**
**Glaxo Group Research Limited, Berkeley Avenue**
**Greenford, Middlesex UB6 0HE(GB)**
Inventor: **Bell, Richard**
**Glaxo Group Research Limited, Berkeley Avenue**
**Greenford, Middlesex UB6 0HE(GB)**
Inventor: **Giblin, Gerard Martin Paul**
**Glaxo Group Research Limited, Berkeley Avenue**
**Greenford, Middlesex UB6 0HE(GB)**
Inventor: **Bailey, Esme Joan**
**Glaxo Group Research Limited, Berkeley Avenue**
**Greenford, Middlesex UB6 0HE(GB)**
Inventor: **Kirk, Barrie Edward**
**Glaxo Group Research Limited, Berkeley Avenue**
**Greenford, Middlesex UB6 0HE(GB)**
Inventor: **Smith, Colin**
**Glaxo Group Research Limited, Berkeley Avenue**
**Greenford, Middlesex UB6 0HE(GB)**
Inventor: **Scicinski, Jan Josef**
**Glaxo Group Research Limited, Berkeley Avenue**
**Greenford, Middlesex UB6 0HE(GB)**
Inventor: **Ross, Barry Clive**
**Glaxo Group Research Limited, Park Road**
**Ware, Hertfordsire, SG12 0DG(GB)**

(74) Representative: **Caffin, Lee et al**
**Glaxo Holdings plc Glaxo House Berkeley Avenue**
**Greenford, Middlesex UB6 0NN(GB)**

(54) **Bridged cyclic ketal derivatives.**

(57) Compounds are described of the formula

EP 0 497 091 A1

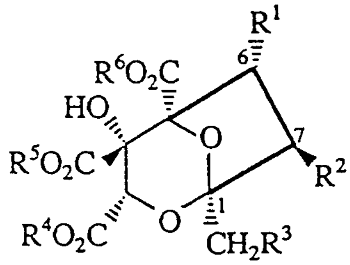

(I)

These compounds inhibit the enzyme squalene synthase and/or are intermediates for the preparation of compounds which inhibit the enzyme squalene synthase. Compounds of the invention may be formulated for use in a variety of conditions where a lowering of the level of blood plasma cholesterol in animals would be beneficial and for use in combating fungal infections in animals.

This invention relates to novel compounds having hypocholesterolemic, hypolipidemic and/or antifungal activity, to processes for their preparation, to pharmaceutical compositions containing them and to their use in medicine, particularly in the treatment and/or prevention of atherosclerosis and associated cardiovascular diseases. The invention also relates to novel compounds which are useful as intermediates for the preparation of compounds having hypocholesterolemic, hypolipidemic and/or antifungal activity.

High levels of blood cholesterol and blood lipids are conditions which are implicated in the onset of vessel wall disease. Methods for effective reduction of plasma cholesterol levels are therefore of high interest. Cholesterol concentrations can be reduced, for example, by lowering the dietary intake of the sterol, by enhancing its metabolism and elimination or by decreasing its rate of biosynthesis. The most effective approaches to lowering physiological cholesterol levels are likely to include inhibition of cholesterol biosynthesis as a component since cholesterol synthesis is subject to feedback regulation, so that decreases in cholesterol levels tend to be compensated for by increased biosynthesis.

One rate-controlling step in the biosynthesis of cholesterol is the formation of mevalonic acid from 3-hydroxy-3-methylglutaryl coenzyme A (HMG CoA) and clinical successes have been achieved with the mevinic acid family of HMG CoA reductase inhibitors in the treatment of hypercholesterolemia. Mevalonic acid, however, is a common precursor of all isoprenyl derivatives, including in animals coenzyme Q, heme A and the dolichols.

The first biosynthetic step which leads exclusively to sterols, the condensation of two farnesyl pyrophosphates to give squalene, is a second site of regulation. The synthesis of squalene from farnesyl pyrophosphate involves an isolable intermediate, presqualene pyrophosphate, and the entire synthetic sequence is catalysed by squalene synthase (farnesyldiphosphate: farnesyldiphosphate farnesyltransferase, EC 2.5.1.21), a membrane-bound enzyme. Agents which act to inhibit the enzyme squalene synthase are therefore potential drugs for the regulation of cholesterogenesis. The use of such agents is attractive as non-steroidal pathways should be minimally affected.

The biosynthesis of ergosterol, the major sterol component of fungal cell membranes, is analogous to that of cholesterol in mammals, including humans, and is thus mediated by the enzyme squalene synthase. Agents which act to inhibit the enzyme squalene synthase in fungal cells are therefore potential drugs for antifungal chemotherapy.

We have now found a group of novel compounds which act as inhibitors of the enzyme squalene synthase and/or are intermediates for the preparation of compounds which act as inhibitors of the enzyme squalene synthase.

Thus, in a first aspect of the present invention, we provide compounds of the general formula (I)

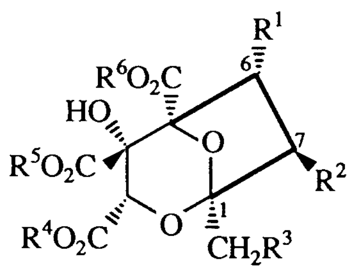

(I)

wherein $R^1$ represents a hydrogen atom or a hydroxyl, acyloxy, carbonate, carbamate or ether group;

$R^2$ represents a hydrogen atom, a hydroxyl group, a group $-OCOR^7$ or a group $-OCO_2R^7$ (where $R^7$ is a group selected from $C_{1-8}$alkyl, aryl, aryl$C_{1-4}$alkyl and $C_{3-8}$cycloalkyl);

$R^3$ represents a group selected from

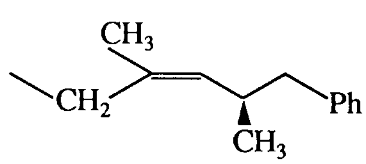

,

$-CH{-}{-}CR^8CR^9R^{10}CHR^{11}(CH_2)_mPh$,

$-CH_2CR^{12}{=}CR^{11}CR^9R^{10}CHR^{11}(CH_2)_nPh$,

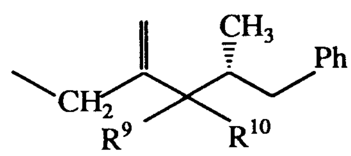

$-CH_2C(CH_3)\overset{E}{=}CHCH(CH_2R^{13})CH_2Ph$, $-CH_2C(CH_2OH)\overset{Z}{=}CHCH(CH_3)CH_2Ph$, $-CH_2C(=CH_2)CH(OH)CH(CH_2OH)CH_2Ph$, $-CH_2C(=CH_2)CH(NHCOCH_3)CH(CH_3)CH_2Ph$, $-CH_2C(CH_2NHCOCH_3)\overset{E}{=}CHCH(CH_3)CH_2Ph$ and

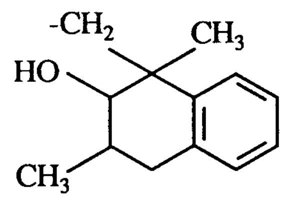

(where the dotted line represents the absence or presence of a single bond, $R^8$ represents a hydrogen atom or a hydroxyl, acyloxy, $C_{1-6}$alkoxy or $C_{1-4}$alkyl group, $R^9$ represents a hydrogen atom and $R^{10}$ represents a hydrogen atom or a hydroxyl, $C_{1-6}$alkoxy or acyloxy group or $CR^9R^{10}$ forms a group C=O, $R^{11}$ represents a hydrogen atom or a $C_{1-4}$alkyl group, $R^{12}$ represents a hydrogen atom or a methyl group, $R^{13}$ represents a hydrogen atom or a hydroxyl group, m represents 1 or 2 and n represents zero or 1);

$R^4$, $R^5$ and $R^6$ may each independently represent a hydrogen atom or a methyl group; and salts thereof;

with the provisos that (1) $R^1$ and $R^2$ cannot both represent hydrogen atoms and (2) when $R_1$ represents a hydrogen atom, a hydroxyl group or an acyloxy group selected from $-OCOCH\overset{E}{=}CHCH(CH_3)(CH_2)_3CH_3$, $-OCOCH\overset{E}{=}CHC(CH_3)\overset{E}{=}CHCH(CH_3)-CH_2CH_3$, or $-OCO-X-CH_2CH(CH_3)CH_2CH_3$ [where X is $-CH\overset{E}{=}CHCH(CH_3)-$, $-CH_2CH(OH)CH(CH_3)-$, $-CH\overset{E}{=}CHC(OH)(CH_3)-$, $-CH_2CH(OH)CH_2-$ or $-CH_2CH_2CH(CH_3)-$)] and $R_2$ represents a hydrogen atom or a hydroxyl group then $R_3$ cannot represent

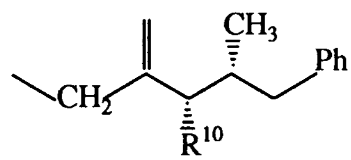

(where $R^{10}$ is a hydroxyl or an acetoxy group), $-CH_2C(CH_3)\overset{E}{=}CHCH(CH_2R^{13})CH_2Ph$, $-CH_2C(CH_2OH)\overset{Z}{=}CHCH(CH_3)CH_2Ph$, $-CH_2C(=CH_2)CH(OH)CH(CH_2OH)CH_2Ph$, $-CH_2C(=CH_2)CH(NHCOCH_3)CH(CH_3)-CH_2Ph$, $-CH_2C(CH_2NHCOCH_3)\overset{E}{=}CHCH(CH_3)CH_2Ph$ or

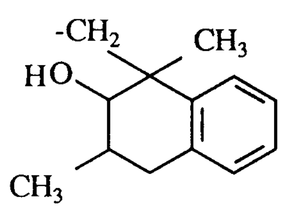

.

It is to be understood that where the configuration of any double bond or chiral centre present in $R^1$, $R^2$ and/or $R^3$ in formula (I) is not defined the present invention is intended to cover all geometrical and optical isomers, including diastereoisomers, of such compounds of formula (I).

It will also be appreciated that compounds of the invention containing a keto group may exist in the

corresponding enolic form.

It is to be understood that when the dotted line in the relevant $R^3$ groupings represents the absence of a single bond $-CH{-}{-}{-}CR^8CR^9R^{10}CHR^{11}(CH_2)_mPh$ and $-CH_2CR^{12}{-}{-}{-}CR^{11}CR^9R^{10}CHR^{11}(CH_2)_nPh$ will represent $-CH_2CHR^8CR^9R^{10}CHR^{11}(CH_2)_mPh$ and $-CH_2CHR^{12}CHR^{11}CR^9R^{10}CHR^{11}(CH_2)_nPh$ respectively, and when the dotted line represents the presence of a single bond $-CH{-}{-}{-}CR^8CR^9R^{10}CHR^{11}(CH_2)_mPh$ and $-CH_2CR^{12}{-}{-}{-}CR^{11}CR^9R^{10}CHR^{11}(CH_2)_nPh$ will represent $-CH{=}CR^8CR^9R^{10}CHR^{11}(CH_2)_mPh$ and $-CH_2CR^{12}{=}CR^{11}CR^9R^{10}CHR^{11}(CH_2)_nPh$ respectively.

Examples of $R^1$ as an acyloxy group include $-OCOCH\overset{E}{=}CHCH(CH_3)(CH_2)_3CH_3$, $-OCOCH\overset{E}{=}CHC(CH_3)\overset{E}{=}CHCH(CH_3)CH_2CH_3$, $-OCO-X-CH_2CH(CH_3)CH_2CH_3$ [where X is $-CH\overset{E}{=}CHCH(CH_3)-$, $-CH_2CH(OH)CH(CH_3)-$, $-CH\overset{E}{=}CHC(OH)(CH_3)-$, $-CH_2CH(OH)CH_2-$ or $-CH_2CH_2CH(CH_3)-$], alkanoyloxy, alkenoyloxy, aroyloxy, arylalkanoyloxy, arylalkenoyloxy, cycloalkanoyloxy, cycloalkylalkanoyloxy and cycloalkylalkenoyloxy.

Examples of $R^1$ as a carbonate group include alkylcarbonates, alkenylcarbonates, arylcarbonates, arylalkylcarbonates, arylalkenylcarbonates, cycloalkylcarbonates, cycloalkylalkylcarbonates and cycloalkylalkenylcarbonates.

When $R^1$ represents a carbamate group this may be, for example, a group $-OCONR^{14}R^{15}$ where $R^{14}$ represents a hydrogen atom or an alkyl group and $R^{15}$ represents a hydrogen atom or a group selected from alkyl, alkenyl, aryl, arylalkyl, arylalkenyl, cycloalkyl, cycloalkylalkyl and cycloalkylalkenyl.

Examples of $R^1$ as an ether group include alkoxy, alkenyloxy, arylalkoxy, arylalkenyloxy, cycloalkyloxy, cycloalkylalkoxy and cycloalkylalkenyloxy.

The term 'alkyl' as part of a group within $R^1$ may be an alkyl chain containing 1-10 carbon atoms optionally substituted by a hydroxyl group, an oxo function (=0) or one or more $C_{1-4}$alkyl groups. Examples of suitable alkyl groups include methyl, ethyl, n-propyl, i-propyl, n-butyl, s-butyl, t-butyl, n-pentyl, n-hexyl, n-heptyl and n-nonyl.

The term 'alkenyl' as part of a group within $R^1$ may be an alkenyl group containing 2-10 carbon atoms and is optionally substituted by one or more $C_{1-4}$alkyl groups. When the alkenyl portion is part of an arylalkenoyloxy group it may represent, for example, an ethenyl group.

The term 'aryl' as a group or part of a group means phenyl or phenyl substituted by one or more moieties including for example halogen atoms, hydroxyl, $C_{1-3}$alkyl and $C_{1-3}$alkoxy groups.

The term 'cycloalkyl' as a group or part of a group means a $C_{3-8}$cycloalkyl group. Examples of suitable cycloalkyl groups include cyclopentyl, cyclohexyl and adamantyl.

The term 'alkyl' within $R^2$ means a straight or branched alkyl chain. Examples of suitable alkyl groups include methyl, ethyl, n-propyl, i-propyl, n-butyl, s-butyl, t-butyl and n-heptyl.

Compounds of formula (I) in which $R^4$, $R^5$ and $R^6$ represent hydrogen atoms or physiologically acceptable cations are generally preferred.

$R^1$ preferably represents an acyloxy, carbonate or ether group, particularly an acyloxy group.

Examples of preferred acyloxy groups within $R^1$ include

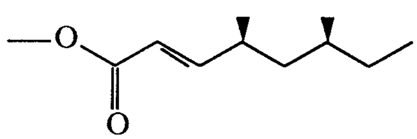 , 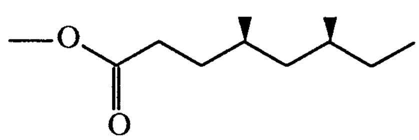 ,

alkanoyloxy, alkenoyloxy, -OCOPh, phenylalkanoyloxy, phenylalkenoyloxy, cycloalkanoyloxy and cycloalkylalkanoyloxy. Particular alkanoyloxy groups within $R^1$ include $-OCO(CH_2)_4COCH_3$, $-OCO(CH_2)_3COCH_3$, $-OCO(CH_2)_6CH_3$, $-OCOCH_3$, $-OCO(CH_2)_8CH_3$ and $-OCO(CH_2)_3CH_3$. Particular alkenoyloxy groups within $R^1$ include $-OCOCH_2CH=CH(CH_2)_3CH_3$. Particular phenylalkanoyloxy groups within $R^1$ include $-OCO(CH_2)_6Ph$. Particular phenylalkenoyloxy groups within $R^1$ include $-OCOCH\overset{E}{=}CHPh$. Particular cycloalkanoyloxy groups within $R^1$ include $-OCOC_6H_{11}$ and -OCOAdamant-1-yl. Particular cycloalkylalkanoyloxy groups within $R^1$ include $-OCO(CH_2)_3C_6H_{11}$.

$R^2$ preferably represents a hydroxyl, acetoxy or $-OCO_2CH_3$ group.

When $R^8$ and/or $R^{10}$ represents an acyloxy group this may be, for example, a $C_{1-6}$alkanoyloxy group and is preferably acetoxy.

$R^3$ preferably represents a group selected from $-CH{-}{-}CR^8CR^9R^{10}CHR^{11}CH_2Ph$ and

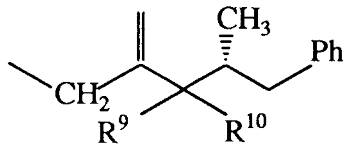

The group -CH--$CR^8$- within $R^3$ may preferably represent a group selected from $-CH=C(CH_3)-$, $-CH_2CH(CH_3)-$, $-CH_2\overline{C}H(OH)-$, $-CH_2C(=0)-$, $-CH_2CH_2-$ and $-CH_2CH(CH_2CH_3)-$.

The group $-CR^9R^{10}-$ within $R^3$ may preferably represent a group selected from -CH(OH)-, -C(=0)-, $-CH_2-$ and $-CH(OCOCH_3)-$.

It is to be understood that this invention covers any combination of the aforementioned particular and preferred groupings.

Particularly preferred compounds of the invention are:

[1S-[1α(4S*,5S*),3α,4β,5α,6α(2E,4R*,6R*),7β]] 1-(4-hydroxy-5-methyl-3-methylene-6-phenylhexyl)-4,6,7-trihydroxy-2,8-dioxabicyclo[3.2.1] octane-3,4,5-tricarboxylic acid, 6-(4,6-dimethyl-2-octenoate);

[1S-[1α[(E),5S*],3α,4β,5α,6α(2E,4R*,6R*),7β]] 1-(3,5-dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyl-2-octenoate);

[1S-[1α[(E),4R*,5S*],3α,4β,5α,6α(2E,4R*,6R*),7β]] 1-(4-hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate);

[1S-[1α[3R*(S*),5S*],3α,4β,5α,6α(2E,4R*,6R*)7β]] 1-(3,5-dimethyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), isomer 1;

[1S-[1α[3R*(S*),4R*,5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 1;

[1S-[1α[3R*(S*),4S*,5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 2;

[1S-[1α[3R*(S*),4R*(S*),5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 3;

[1S-[1α[3R*(S*),4R*(S*),5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 4;

[1S-[1α(3R*S*,5S*),3α,4β,5α,6α(2E,4R*,6R*),7β]] 1-(3-ethyl-5-methyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate);

[1S-[1α[(E),4R*,5S*],3α,4β,5α,6α(4S*,6R*),7β]] 1-(4-acetyloxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyloctanoate);

[1S-[1α(3R*S*,4S*,5S*),3α,4β,5α,6α(4S*,6R*),7β]] 1-(4-acetyloxy-3,5-dimethyl-6-phenylhexyl)-4,6,7-trihydroxy-2,8-dioxabicyclo[3.2.1]octane-3,4,5-tricarboxylic acid, 6-(4,6-dimethyloctanoate);

[1S-[1α(3R*S*,5R*),3α,4β,5α,6α(4S*,6R*),7β]] 1-(3,5-dimethyl-6-phenylhexyl)-4,6,7-trihydroxy-2,8-dioxabicyclo[3.2.1]octane-3,4,5-tricarboxylic acid, 6-(4,6-dimethyloctanoate);

[1S-[1α(3R*S*,4S*,5S*),3α,4β,5α,6α(4S*,6R*),7β]] 1-(3,5-dimethyl-4-hydroxy-6-phenylhexyl)-4,6,7-trihydroxy-2,8-dioxabicyclo[3.2.1]octane-3,4,5-tricarboxylic acid, 6-(4,6-dimethyloctanoate);

[1S-[1α[(E),4R*,5S*],3α,4β,5α,6α(4S*,6R*),7β]] 1-(3,5-dimethyl-4-hydroxy-6-phenyl-2-hexenyl)-4,6,7-trihydroxy-2,8-dioxabicyclo[3.2.1]octane-3,4,5-tricarboxylic acid, 6-(4,6-dimethyloctanoate);

[1S-[1α(3R*(S*),5S*),3α,4β,5α,6α(4S*,6R*),7β]] 1-(3,5-dimethyl-4-oxo-6-phenylhexyl)-4,6,7-trihydroxy-2,8-dioxabicyclo[3.2.1]octane-3,4,5-tricarboxylic acid, 6-(4,6-dimethyloctanoate), 3,4,5-tripotassium salt, isomer 1;

[1S-[1α(4R*,5S*),3α,4β,5α,6α(4S*,6R*),7β]] 1-(4-hydroxy-5-methyl-3-oxo-6-phenylhexyl)-4,6,7-trihydroxy-2,8-dioxobicyclo[3.2.1]octane-3,4,5-tricarboxylic acid, 6-(4,6-dimethyloctanoate);

[1S-[1α(3R*S*,4R*,5S*),3α,4β,5α,6α(4S*,6R*),7β]] 1-(3,4-dihydroxy-5-methyl-6-phenylhexyl)-4,6,7-trihydroxy-2,8-dioxabicyclo[3.2.1]octane-3,4,5-tricarboxylic acid, 6-(4,6-dimethyloctanoate);

[1S-[1α(5R*S*),3α,4β,5α,6α(4S*,6R*),7β]] 1-(5-methyl-4-oxo-6-phenylhexyl)-4,6,7-trihydroxy-2,8-dioxabicyclo-[3.2.1]octane-3,4,5-tricarboxylic acid, 6-(4,6-dimethyloctanoate);

[1S-[1α[3R*(S*)],3α,4β,5α,6α(4S*,6R*),7β]] 1-(3-methyl-4-oxo-6-phenylhexyl)-4,6,7-trihydroxy-2,8-dioxabicyclo[3.2.1]octane-3,4,5-tricarboxylic acid, 6-(4,6-dimethyloctanoate);

[1S-[1α(4R*,5S*),3α,4β,5α,6α(2E,4R*,6R*),7β]] 7-acetyloxy-1-(4-acetyloxy-5-methyl-3-methylene-6-phenylhexyl)-4,6-dihydroxy-2,8-dioxabicyclo[3.2.1]octane-3,4,5-tricarboxylic acid, 6-(4,6-dimethyl-2-octenoate);

[1S-[1α(4R*,5S*),3α,4β,5α,6α(2E,4R*,6R*),7β]] 1-(4-acetyloxy-5-methyl-3-methylene-6-phenylhexyl)-4,6-dihydroxy-7-[(methoxycarbonyl)oxy]-2,8-dioxabicyclo[3.2.1]octane-3,4,5-tricarboxylic acid, 6-(4,6-dimethyl-2-octenoate);

[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-acetate;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-decanoate;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-octanoate;
[1S-[1α(4R*,5S*)3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-pentanoate;
[1S-[1α(4R*,5S*)3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(7-phenylheptanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(4-cyclohexylbutanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(cyclohexanecarboxylate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-[1-(tricyclo[3.3.1.1$^{3,7}$]decyl)acetate];
[1S-[4α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-benzoate;
[1S-[1α(4R*,5S*),3α,4β,5α,6α(2E),7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(3-phenyl-2-propenoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(5-oxo-hexanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(6-oxo-heptanoate);
[1S-[1α(4R*,5S*),3α,4β,5α,6α,7β]] 1-(4-acetyloxy-5-methyl-3-methylene-6-phenylhexyl)-4,7-dihydroxy-6-methoxy-2,8-dioxabicyclo[3.2.1]octane-3,4,5-tricarboxylic acid;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-[(E/Z)-3-octenoate];
and physiologically acceptable salts thereof.

Other preferred compounds of the invention are:
[1S-[1α(4R*,5S*),3α,4β.5α,6α,7β]] 1-(4-acetyloxy-5-methyl-3-methylene-6-phenylhexyl)-4,7-dihydroxy-6-[(-(nonoxycarbonyl)oxy]-2,8-dioxabicyclo[3.2.1]octane-3,4,5-tricarboxylic acid;
[1S-[1α(4R*,5S*)3α,4β,5α,6α,7β]] 1-(4-acetyloxy-5-methyl-3-methylene-6-phenylhexyl)-6-[(butoxycarbonyl)-oxy]-4,7-dihydroxy-2,8-dioxabicyclo[3.2.1]octane-3,4,5-tricarboxylic acid;
and physiologically acceptable salts thereof.

Compounds of formula (I) in which $R^1$ represents a hydroxyl group are particularly useful as intermediates for the preparation of related structures having squalene synthase inhibitory activity.

Compounds of the present invention may form salts with inorganic and organic bases. Physiologically acceptable salts include inorganic base salts such as alkali metal salts (e.g. sodium and potassium salts including the trisodium, dipotassium and tripotassium salts), alkaline earth metal salts (e.g. calcium salts), ammonium salts and amino acid salts (e.g. lysine and arginine salts including the tri-L-lysine salts). Suitable organic base salts include amine salts such as trialkylamine (e.g. triethylamine), dialkylamine (e.g. dicyclohexylamine), optionally substituted benzylamine (e.g. p-bromobenzylamine) and tris(hydroxymethyl)-methylamine salts.

Compounds of the invention have been found to inhibit the enzyme squalene synthase and cholesterol biosynthesis and are therefore of use in medicine, particularly in a variety of conditions where a lowering of the level of blood plasma cholesterol in animals (especially humans) would be beneficial. Examples of such conditions include diseases associated with hypercholesterolemia and hyperlipoproteinemia, especially atherosclerosis and cardiovascular diseases (such as cardiac ischaemic diseases, cerebral ischaemic diseases and peripheral arterial disease).

Compounds of the invention which inhibit squalene synthase may also be of use in combating fungal infections in animals, including humans. For example, they may be useful in the treatment of systemic infections caused by, for example Candida (e.g. Candida albicans, Candida glabrata, Candida parapsilosis and Candida pseudotrop), Cryptococcus neoformans, Aspergillus Sp (e.g. Aspergillus flavus and Aspergillus fumigatus), Coccidioides (e.g. Coccidioides immitis), Paracoccidioides (e.g. Paracoccidioides brasiliensis), Histoplasma (e.g. Histoplasma capsulatum) or Blastomyces (e.g. Blastomyces dermatitidis). They may also be useful in treating topical infections caused by species of Trichophyton, Microsporum or Epidermophyton (e.g. Trichophyton mentographytes, Microsporum canis or Epidermophyton floccosum). They may also be

of use in treating fungal diseases caused by Torulopsis glabrata and Pityrosporum ovale.

The in vitro evaluation of the anti-fungal activity of compounds of the invention can be performed by determining the minimum inhibitory concentration (MIC) which is the concentration of the test compound in a suitable medium at which growth of a particular microorganism fails to occur.

In view of their potential in antifungal therapy, compounds of the invention which inhibit squalene synthase may recommend themselves for the treatment of a variety of fungal infections in human beings and animals. Such infections include mycotic infections such as candidiasis and chronic mucocandidiasis (e.g. thrush and vaginal candidiasis) and skin infections caused by fungi, cutaneous and mucocutaneous candidiasis, dermatophytoses including ringworm and tinea infections, athletes foot, paronychia, pityriasis versicolor, erythrasma, intertrigo, fungal nappy rash, candida vulvitis, candida balanitis and otitis externa. They may also be useful as prophylactic agents to prevent systemic and topical fungal infections. Use as prophylactic agents may, for example, be appropriate as part of a selective gut decontamination regimen in the prevention of infection in immunocompromised patients. Prevention of fungal overgrowth during antibiotic treatment may also be desirable in some disease syndromes or iatrogenic states.

The ability of compounds of the invention to inhibit the enzyme squalene synthase in mammals and fungi may be demonstrated in vitro using [2-$^{14}$C]farnesylpyrophosphate as a substrate under assay conditions similar to those described by S. A. Biller et al. in J. Medicinal Chemistry 31(10), 1869-1871 (1988); [$^{14}$C]squalene is separated from unreacted substrate on thin layer chromatography plates and determined by liquid scintillation counting. The ability of compounds of the invention to inhibit cholesterol biosynthesis may be demonstrated by measuring inhibition from [$^{14}$C]-acetate in liver slices from male Wistar rats using a method similar to that described by Y. Tsujita et al. in Biochem. Biophys. Acta, Volume 877, 50-60 (1986) and modified to include measurement of cholesterol by high performance liquid chromatography (h.p.l.c.).

While it is possible that, for use in therapy, compounds of the invention which inhibit squalene synthase may be administered as the raw chemical, it is preferable to present the active ingredient as a pharmaceutical formulation. The invention thus further provides a pharmaceutical formulation comprising compounds of the invention which inhibits squalene synthase together with one or more pharmaceutically acceptable carriers thereof and, optionally, other therapeutic and/or prophylactic ingredients. The carrier(s) must be 'acceptable' in the sense of being compatible with the other ingredients of the formulation and not deleterious to the recipient thereof.

The compositions of the invention include those in a form especially formulated for oral, buccal, parenteral, implant, rectal, topical, ophthalmic or genito-urinary administration or in a form suitable for administration by inhalation or insufflation.

Tablets and capsules for oral administration may contain conventional excipients such as binding agents, for example, syrup, acacia, gelatin, sorbitol, tragacanth, mucilage of starch or polyvinylpyrrolidone; fillers, for example, lactose, sugar, microcrystalline cellulose, maize-starch, calcium phosphate or sorbitol; lubricants, for example, magnesium stearate, stearic acid, talc, polyethylene glycol or silica; disintegrants, for example, potato starch or sodium starch glycollate; or wetting agents such as sodium lauryl sulphate. The tablets may be coated according to methods well known in the art. Oral liquid preparations may be in the form of, for example, aqueous or oily suspensions, solutions, emulsions, syrups or elixirs, or may be presented as a dry product for constitution with water or other suitable vehicle before use. Such liquid preparations may contain conventional additives such as suspending agents, for example, sorbitol syrup, methyl cellulose, glucose/sugar syrup, gelatin, hydroxyethylcellulose, carboxymethyl cellulose, aluminium stearate gel or hydrogenated edible fats; emulsifying agents, for example, lecithin, sorbitan mono-oleate or acacia; non-aqueous vehicles (which may include edible oils), for example, almond oil, fractionated coconut oil, oily esters, propylene glycol or ethyl alcohol; and preservatives, for example, methyl or propyl p-hydroxybenzoates or sorbic acid. The compositions may also be formulated as suppositories, e.g. containing conventional suppository bases such as cocoa butter or other glycerides.

For buccal administration the composition may take the form of tablets or lozenges formulated in conventional manner.

The composition according to the invention may be formulated for parenteral administration by injection or continuous infusion. Formulations for injection may be presented in unit dose form in ampoules, or in multi-dose containers with an added preservative. The compositions may take such forms as suspensions, solutions, or emulsions in oily or aqueous vehicles, and may contain formulatory agents such as suspending, stabilising and/or dispersing agents. Alternatively the active ingredient may be in powder form for constitution with a suitable vehicle, e.g. sterile, pyrogen-free water, before use.

For administration by inhalation the compositions according to the invention are conveniently delivered in the form of an aerosol spray presentation from pressurised packs with the use of a suitable propellant,

e.g. dichlorodifluoromethane, trichlorofluoromethane, dichlorotetrafluoroethane, carbon dioxide or other suitable gas, or from a nebuliser. In the case of a pressurised aerosol the dosage unit may be determined by providing a valve to deliver a metered amount.

Alternatively, for administration by inhalation the compositions according to the invention may take the form of a dry powder composition, for example a powder mix of the compound and a suitable powder base such as lactose or starch. The powder composition may be presented in unit dosage form in, for example, capsules or cartridges of e.g. gelatin, or blister packs from which the powder may be administered with the aid of an inhaler or insufflator.

The compositions may take the form of a suppository, e.g. containing a conventional suppository base, or a pessary, e.g. containing a conventional pessary base.

The compositions may also be formulated for topical administration in the form of ointments, creams, gels, lotions, shampoos, powders (including spray powders), pessaries, tampons, sprays, dips, aerosols, drops (e.g. eye, ear or nose drops) or pour-ons. Ointments and creams may, for example, be formulated with an aqueous or oily base with the addition of suitable thickening and/or gelling agents. Ointments for administration to the eye may be manufactured in a sterile manner using sterilised components. Pour-ons may, for example, be formulated for veterinary use in oils containing organic solvents, optionally with formulatory agents, e.g. stabilising and solubilising agents. Pessaries and tampons for vaginal insertion may be formulated using conventional techniques and, where appropriate, may contain an effervescent vehicle. Such compositions may also contain other active ingredients such as corticosteroids, antibiotics or antiparasitics as appropriate.

Liquid preparations for intranasal delivery may take the form of solutions or suspensions and may contain conventional excipients such as tonicity adjusting agents, for example, sodium chloride, dextrose or mannitol; preservatives, for example benzalkonium chloride, thiomersal, phenylethyl alcohol; and other formulating agents such as suspending, buffering, stabilising and/or dispersing agents.

Transdermal administration may be affected by the design of a suitable system which promotes adsorption of the active compound through the skin and would typically consist of a base formulation enclosed within an adhesive stick-on patch comprising backing films, membranes and release liners.

The composition according to the invention may also be formulated as a depot preparation. Such long acting formulations may be administered by implantation (for example subcutaneously or intramuscularly) or by intramuscular injection. Thus, for example, a compound of the invention may be formulated with suitable polymeric or hydrophobic materials (for example as an emulsion in an acceptable oil) or ion exchange resins, or as sparingly soluble derivatives, for example, as a sparingly soluble salt.

When the compositions comprise dosage units, each unit will preferably contain 0.001mg to 1000mg, advantageously 0.01mg to 400mg, of active ingredient where a compound of the invention is to be administered orally. The daily dosage as employed for adult human treatment will preferably range from 0.001mg to 5000mg of active ingredient, most preferably from 0.01mg to 2000mg which may be administered in 1 to 4 daily doses, for example, depending on the route of administration and on the condition of the patient and the disease to be treated.

The compound may be administered by intravenous infusion using, for example, up to 50mg/kg/day of the active ingredient. The duration of treatment will be dictated by the rate of response rather than by arbitrary numbers of days.

Compounds of the invention which inhibit squalene synthase may also be used in combination with other therapeutic agents, and the invention thus provides, in a further aspect, a combination comprising a compound of the invention which inhibits squalene synthase together with another therapeutically active agent, such as an inhibitor of 3-hydroxy-3-methylglutaryl coenzyme A (HMG CoA) reductase or another agent which reduces serum cholesterol and/or inhibits cholesterol biosynthesis, for example a bile acid sequestrant or an antihyperlipoproteinemic or antihyperlipemic agent such as probucol, gemfibrozil, clofibrate, dextrothyroxine or its sodium salt, colestipol or its hydrochloride salt, cholestyramine, nicotinic acid, neomycin, p-aminosalicylic acid, aspirin, DEAE-Sephadex, a poly(diallylmethylamine) derivative, an ionene or poly(diallyldimethylammonium) chloride.

The combinations referred to above may conveniently be presented for use in the form of a pharmaceutical formulation and thus pharmaceutical formulations comprising a combination as defined above together with a pharmaceutically acceptable carrier thereof comprise a further aspect of the invention. The individual components of such combinations may be administered either sequentially or simultaneously in separate or combined pharmaceutical formulations.

When a compound of the invention is used in combination with a second therapeutic agent against the same condition the dose of each compound may differ from that when the compound is used alone. Appropriate doses will be readily appreciated by those skilled in the art.

According to another aspect of the present invention, we provide a compound of formula (I) or a physiologically acceptable salt thereof or a pharmaceutical composition comprising a compound of formula (I) or a physiologically acceptable salt thereof as defined above for use in therapy, particularly for the treatment of conditions where a lowering of the level of blood plasma cholesterol in animals (especially humans) would be beneficial, or for the treatment of fungal infections in animals (especially humans).

In a particular aspect of the present invention, we provide a compound of formula (I) or a physiologically acceptable salt thereof or a pharmaceutical composition comprising a compound of formula (I) or a physiologically acceptable salt thereof as defined above for use in the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to a further aspect of the present invention, we provide the use of a compound of formula (I) or a physiologically acceptable salt thereof in the manufacture of a medicament for the treatment of diseases associated with hypercholesterolemia and/or hyperlipoproteinemia, especially atherosclerosis and cardiovascular diseases (such as cardiac ischaemic diseases, cerebral ischaemic diseases and peripheral arterial disease).

According to another aspect of the present invention, we provide the use of a compound of formula (I) or a physiologically acceptable salt thereof in the manufacture of a medicament for the treatment of fungal infections in a human or non-human animal patient.

According to a further aspect of the present invention, we provide a method of treatment of the human or non-human animal body to combat diseases associated with hypercholesterolemia and/or hyperlipoproteinemia, especially atherosclerosis and cardiovascular diseases (such as cardiac ischaemic diseases, cerebral ischaemic diseases and peripheral arterial disease) or to combat fungal diseases, which method comprises administering to said body an effective amount of a compound of formula (I) or a physiologically acceptable salt thereof.

It will be appreciated by those skilled in the art that references herein to treatment extend to prophylaxis as well as the treatment of established conditions or infections.

The compounds of the invention may be prepared by the processes described below.

Thus, a general process (A) for the preparation of compounds of formula (I) in which $R^3$ represents a group $-CH=CR^8CR^9R^{10}CHR^{11}(CH_2)_mPh$ or a group $-CH_2CR^{12}=CR^{11}CR^9R^{10}CHR^{11}(CH_2)_nPh$ where $R^8$ is hydrogen or $C_{1-4}$alkyl comprises reacting a compound of formula (II)

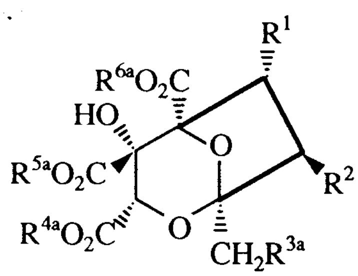

(II)

(wherein $R^{3a}$ represents CHO or $CH_2COR^{12}$ as appropriate, $R^1$ and $R^2$ are as defined previously and $R^{4a}$, $R^{5a}$ and $R^{6a}$ are as defined for $R^4$, $R^5$ and $R^6$ above or are protecting groups) with a compound of formula (IIIa) or (IIIb)

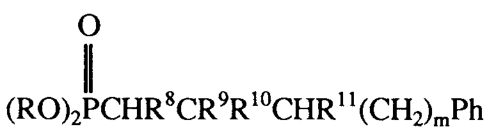

(IIIa)

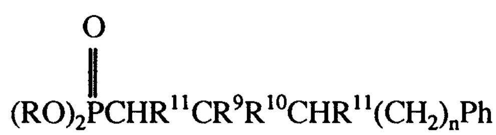

(IIIb)

as appropriate (wherein $R^{11}$ is as defined previously, $R^8$ is hydrogen or $C_{1-4}$alkyl, $CR^9R^{10}$ forms a group C=0 and R represents a $C_{1-6}$alkyl group, e.g. methyl or an aryl group, e.g. phenyl) under Wadsworth-Emmons conditions, followed, where appropriate by converting $CR^9R^{10}$ as a group C=0 to a group $CHR^{10}$

where $R^{10}$ is hydroxy, $C_{1-6}$alkoxy or acyloxy according to the general procedures described hereinafter, and thereafter removing any protecting groups present.

The reaction between compounds (II) and (IIIa) or (IIIb) may conveniently be carried out in an ether solvent (e.g. tetrahydrofuran) in the presence of a strong base such as an alkali metal hydride (e.g. sodium hydride) at a temperature in the range of $0^0$ to $20^0$C.

Another general process (B) comprises converting a compound of formula (I) or a protected derivative thereof to a different compound of formula (I) or a protected derivative thereof, followed, if necessary, by the removal of any protecting groups present. Specific examples of interconversion reactions are described hereinafter.

Compounds of formula (I) in which $R^3$ represents a group $-CH_2CHR^8CR^9R^{10}CHR^{11}(CH_2)_mPh$ or $CH_2CHR^{12}CHR^{11}CR^9R^{10}CHR^{11}(CH_2)_nPh$ may be prepared from the corresponding compounds of formula (I) in which $R^3$ represents a group $-CH=CR^8CR^9R^{10}CHR^{11}(CH_2)_mPh$ or $CH_2CR^{12}=CR^{11}CR^9R^{10}CHR^{11}(CH_2)_nPh$ or protected derivatives thereof under appropriate reducing conditions, for example by catalytic hydrogenation [e.g. hydrogenation in the presence of palladium-on-carbon in a suitable solvent such as an ester (e.g. ethyl acetate) or an alcohol (e.g. ethanol)], followed, where appropriate, by removing any protecting groups present.

Compounds of formulae (IIIa) and (IIIb) are either known compounds or may be prepared by methods analogous to those used to prepare the known compounds of formulae (IIIa) and (IIIb).

Compounds of formula (II) in which $R^{3a}$ represents CHO may be prepared by ozonolysis of a compound of formula (IV)

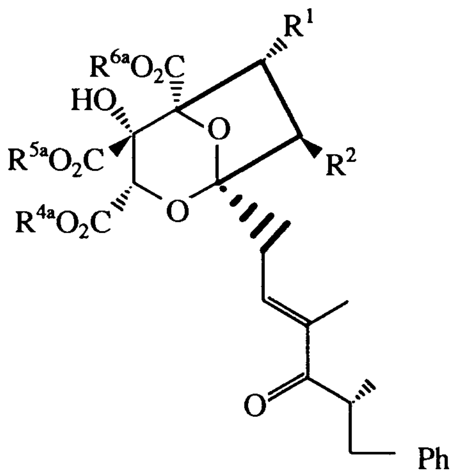

(IV)

(wherein $R^1$, $R^2$, $R^{4a}$, $R^{5a}$ and $R^{6a}$ are as defined previously). Thus, for example, a compound of formula (IV) may conveniently be treated with ozone in a halogenated hydrocarbon solvent (e.g. dichloromethane) at a low temperature (e.g. -70$^0$C to $0^0$C), followed by treatment with either a triarylphosphine such as triphenylphosphine or a dialkyl sulphide such as dimethyl sulphide to provide the desired compound of formula (II).

Compounds of formula (IV) may be prepared by isomerization of a compound of formula (V)

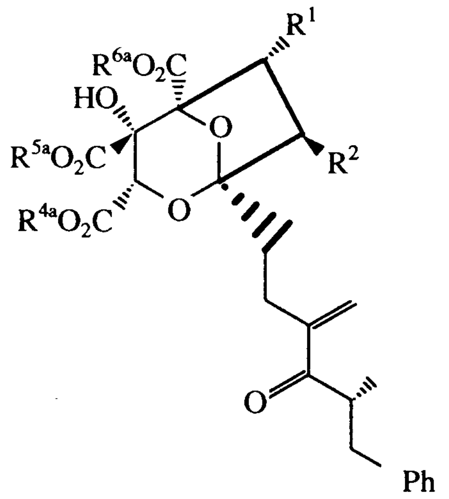

(V)

(wherein $R^1$, $R^2$, $R^{4a}$, $R^{5a}$ and $R^{6a}$ are as previously defined). The isomerization may conveniently be effected by heating a compound of formula (V) with a suitable transition metal catalyst such as rhodium trichloride in an aqueous alcoholic solvent (e.g. aqueous methanol).

Compounds of formula (I) in which $R^3$ represents a group

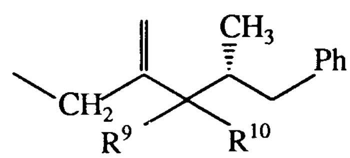

(wherein $CR^9R^{10}$ forms a group C=0) are either compounds of formula (V) or may be prepared from compounds of formula (V) by removing any protecting groups present. Compounds of formula (I) in which $R^9$ is hydrogen and $R^{10}$ is a hydroxyl group may be prepared by reducing the keto group in formula (V) using, for example, sodium borohydride in an alcoholic solvent (e.g. methanol) at a temperature in the range $0^0$ to $20^0$C or using zinc dust in an aqueous ether (e.g. aqueous tetrahydrofuran), followed where necessary by removing any protecting groups present. Compounds of formula (I) in which $R^9$ is hydrogen and $R^{10}$ is an acyloxy group may be prepared from the corresponding compounds in which $R^9$ is hydrogen and $R^{10}$ is a hydroxyl group or protected derivatives thereof by conventional acylation means described hereinafter, followed where necessary by removal of any protecting groups present. Compounds of formula (I) in which $R^9$ is hydrogen and $R^{10}$ is a $C_{1-6}$alkoxy group may be prepared from the corresponding compounds in which $R^9$ is hydrogen and $R^{10}$ is a hydroxyl group or protected derivatives thereof by conventional etherification means described hereinafter, followed where necessary by removal of any protecting groups present.

Compounds of formula (I) in which $R^3$ represents a group

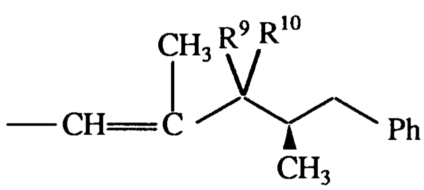

(wherein $CR^9R^{10}$ forms a group C=0) are either compounds of formula (IV) or may be prepared from compounds of formula (IV) by removing any protecting groups present. Compounds of formula (I) in which $R^9$ is hydrogen and $R^{10}$ is a hydroxyl group may be prepared by reducing the keto group in formula (IV) using, for example, sodium borohydride in an alcoholic solvent (e.g. methanol) at a temperature in the range $0^0$ to $20^0$C or using zinc dust in an aqueous ether (e.g. aqueous tetrahydrofuran), followed where necessary

by removing any protecting groups present. Compounds of formula (I) in which $R^9$ is hydrogen and $R^{10}$ is an acyloxy group may be prepared from the corresponding compounds in which $R^9$ is hydrogen and $R^{10}$ is a hydroxyl group or a protected derivative thereof by conventional acylation means as described hereinafter, followed where necessary by removal of any protecting groups present. Compounds of formula (I) in which $R^9$ is hydrogen and $R^{10}$ is a $C_{1-6}$alkoxy group may be prepared from the corresponding compounds in which $R^9$ is hydrogen and $R^{10}$ is a hydroxyl group or protected derivatives thereof by conventional etherification means described hereinafter, followed where necessary by removal of any protecting groups present.

Compounds of formula (I) in which $R^3$ represents a group (wherein $R^8$ is

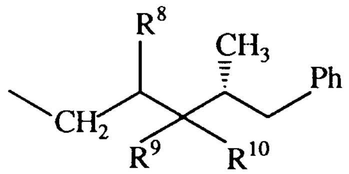

$C_{2-4}$alkyl, $R^9$ is a hydrogen atom and $R^{10}$ is a hydroxyl or acyloxy group or $CR^9R^{10}$ forms a group C=0) may be prepared from compounds of formula (V). Thus, compounds in which $CR^9R^{10}$ represents C=0 may be prepared by treating the compound of formula (V) with a suitable lithium cuprate $LiCu(R^{16})_2$ (where $R^{16}$ is a $C_{1-3}$alkyl group, e.g. methyl) at reduced temperature (e.g. -20$^0$ to +10$^0$C) in an ether solvent (e.g. tetrahydrofuran), followed where necessary by removing any protecting groups present. The resulting keto compounds of formula (I) or protected derivatives thereof may then be converted to the corresponding compounds of formula (I) in which $R^8$ is $C_{2-4}$alkyl, $R^9$ is hydrogen and $R^{10}$ is a hydroxyl, $C_{1-6}$alkoxy or acyloxy group by reduction as described hereinabove, followed where appropriate by etherification or acylation as described hereinafter, followed, where necessary, by removal of any protecting groups present.

Compounds of formula (I) in which $R^3$ represents a group

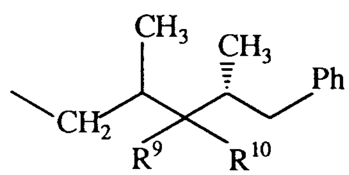

(wherein $R^9$ represents a hydrogen atom and $R^{10}$ represents a hydroxyl, $C_{1-6}$alkoxy or acyloxy group or $CR^9R^{10}$ forms a group C=0) may be prepared from compounds of formula (VI)

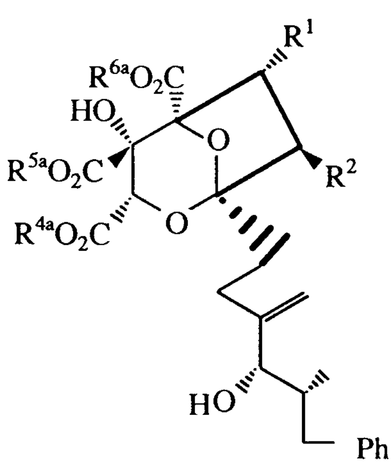

(VI)

(wherein $R^1$, $R^2$, $R^{4a}$, $R^{5a}$ and $R^{6a}$ are as previously defined). Thus, compounds in which $CR^9R^{10}$ represents C=0 may be prepared by treating a compound of formula (VI) with a suitable transition metal reagent such

as a rhodium complex, e.g. $RhCl(PPh_3)_3$ at an elevated temperature (e.g. at reflux), in a suitable solvent such as an aqueous alcohol (e.g. aqueous methanol), or using a suitable metal catalyst such as palladium-on-carbon in an organic solvent, for example an ester or an alcohol at ambient temperature, followed where necessary by removing any protecting groups present. The resulting keto compounds of formula (I) or protected derivatives thereof may then be converted to the corresponding compounds of formula (I) in which $R^9$ is hydrogen and $R^{10}$ is a hydroxyl, $C_{1-6}$alkoxy or acyloxy group by reduction as described hereinabove, followed where appropriate by etherification or acylation as described hereinafter, followed by removal of any protecting groups present.

The aforementioned conversion of $CR^9R^{10}$ as CHOH to $CHR^{10}$ where $R^{10}$ is a $C_{1-6}$alkoxy group may be effected by reaction under conventional conditions for ether formation. Thus, for example, the conversion may conveniently be effected by reaction with a halide $R^{17}$Hal (where Hal is a halogen atom such as bromine or iodine and $R^{17}$ is a $C_{1-6}$alkyl group) preferably in the presence of a suitable base such as an alkali metal hydroxide (e.g. potassium hydroxide), an alkali metal hydride (e.g. sodium hydride) or an alkali metal alkoxide (e.g. potassium tert-butoxide) in a solvent such as an ether (e.g. tetrahydrofuran) or a dialkylamide (e.g. dimethylformamide).

The aforementioned conversion of $CR^9R^{10}$ as CHOH to $CHR^{10}$ where $R^{10}$ is an acyloxy group may conveniently be effected by reaction with a suitable acylating agent under conventional conditions. Thus, for example the conversion may conveniently be effected by reaction with an acyl halide, for example an acyl chloride, in the presence of 4-dimethylaminopyridine with or without a suitable base such as a tertiary amine (e.g.triethyamine) or using an alkali metal carbonate or alkaline earth metal carbonate (e.g. calcium carbonate) in a solvent such as a halogenated hydrocarbon (e.g. dichloromethane).

Compounds of formula (I) in which $R^3$ represents a group

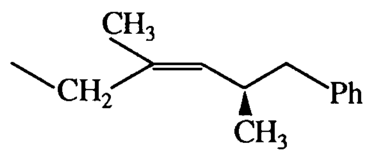

may be prepared from compounds of formula (VI) by catalytic hydrogenolysis using a suitable palladium catalyst, for example palladium-on-barium sulphate in a suitable solvent such as an alcohol (e.g. ethanol), followed where necessary by removing any protecting groups present.

Compounds of formula (I) in which $R^3$ represents

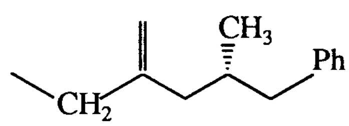

may be prepared from compounds of formula (VII)

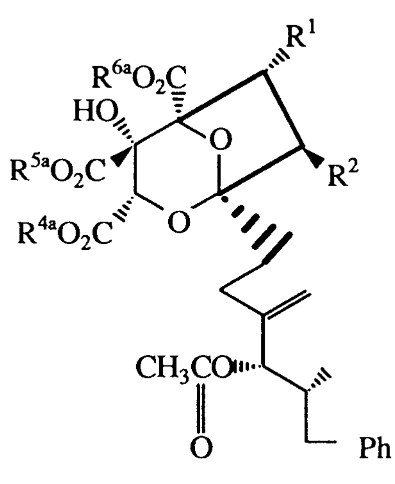

(VII)

(wherein $R^1$, $R^2$, $R^{4a}$ $R^{5a}$ and $R^{6a}$ are as defined previously) by heating the compound of formula (VII) with ammonium formate in the presence of a suitable palladium catalyst such as $(Ph_3P)_2PdCl_2$ and in a suitable solvent such as an ether (e.g. dioxan), followed where necessary by removing any protecting groups present.

Compounds of formula (I) in which $R^3$ represents

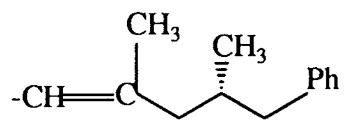

may be prepared from a compound of formula (I) in which $R^3$ represents

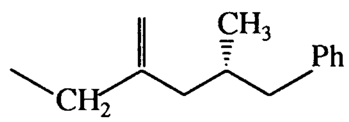

by isomerization using, for example, a hydrogen halide (e.g. hydrogen chloride) in a solvent such as an ether (e.g. dioxan).

Compounds of formula (I) in which $R^3$ represents

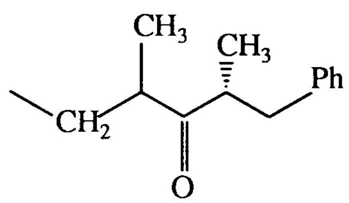

may also be prepared from compounds of formula (I) in which $R^3$ represents

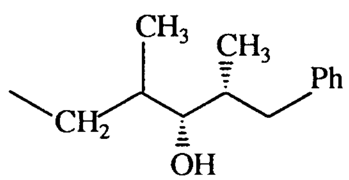

or protected derivatives thereof using a suitable oxidising agent such as pyridinium chlorochromate in a halogenated hydrocarbon solvent (e.g. dichloromethane), followed where necessary by removal of any protecting groups present.

Compounds of formula (I) in which $R^3$ represents

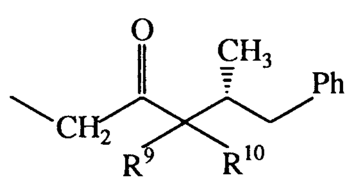

may be prepared from corresponding compounds in which $R^3$ represents

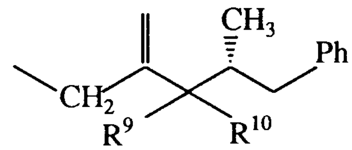

by ozonolysis using for example ozone in a solvent such as a halogenated hydrocarbon (e.g. dichloromethane) at a low temperature (e.g. -70°C to 0°C), followed by treatment with either a triarylphosphine such as triphenylphosphine or a dialkyl sulphide such as dimethyl sulphide, and thereafter where necessary followed by removing any protecting groups present.

Compounds of formula (I) in which $R^3$ represents

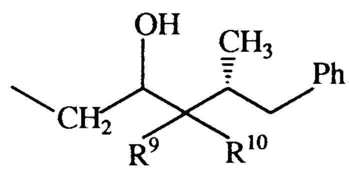

(where $R^9$ and $R^{10}$ are as defined previously except that $CR^9R^{10}$ may not represent C=O) may be prepared from compounds of formula (I) in which $R^3$ represents

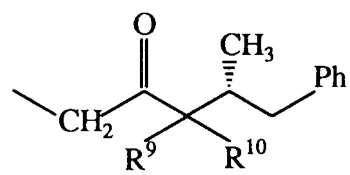

or protected derivatives thereof by reduction, for example using a suitable reducing agent such as sodium borohydride in an appropriate solvent (e.g. an alcohol such as methanol) at reduced temperature (e.g. -10°C to +10°C), followed where necessary by removal of any protecting groups present.

Compounds of formula (I) in which $R^3$ represents a group

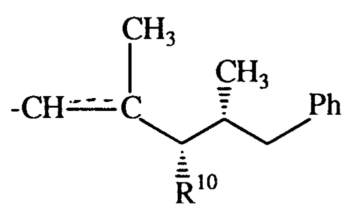

(in which $R^{10}$ is hydrogen, hydroxy or acetoxy) may also be prepared from compounds of formulae (VI) and (VII) as appropriate by catalytic hydrogenation using hydrogen in the presence of a suitable palladium catalyst (e.g. palladium-on-carbon) in a solvent such as ethyl acetate, or a mixture of an alcohol (e.g. ethanol) and a halogenated hydrocarbon (e.g. dichloromethane) in the presence of triethylamine, or by isomerisation using a suitable palladium catalyst as defined above under the conditions described just above, optionally in the presence of hydrogen, followed where appropriate by removing any protecting groups present.

Compounds of formula (II) in which $R^{3a}$ represents $CH_2CHO$ may conveniently be prepared from the aforementioned compounds of formula (I) in which $R^3$ represents

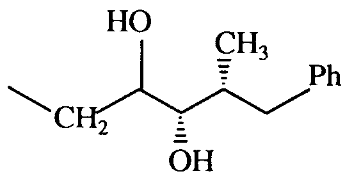

by oxidation using a suitable oxidising agent such as a periodate (e.g. sodium periodate) in an aqueous ether (e.g aqueous tetrahydrofuran) conveniently at a temperature in the range 0°-20°C.

Compounds of formula (II) in which $R^{3a}$ represents $CH_2COCH_3$ may conveniently be prepared from compounds of formula (VIII)

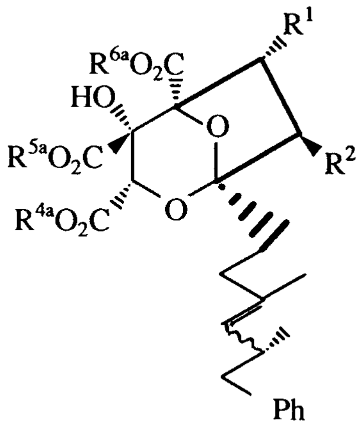

(VIII)

(wherein $R^1$, $R^2$, $R^{4a}$, $R^{5a}$ and $R^{6a}$ are as defined previously) by ozonolysis using for example ozone in a solvent such as a halogenated hydrocarbon (e.g. dichloromethane) at a low temperature (e.g. -70°C to 0°C), followed by treatment with either a triarylphosphine such as triphenylphosphine or a dialkyl sulphide such as dimethyl sulphide.

Compounds of formula (VIII) in which $R^{4a}$, $R^{5a}$ and $R^{6a}$ represent protecting groups may be prepared from the corresponding compounds of formula (VIII) in which $R^{4a}$, $R^{5a}$ and $R^{6a}$ are hydrogen atoms using standard methods of carboxylic acid group protection. Such compounds of formula (VIII) in which $R^{4a}$, $R^{5a}$ and $R^{6a}$ represent hydrogen atoms may conveniently be prepared from compounds of formula (VI) in which $R^{4a}$, $R^{5a}$ and $R^{6a}$ are hydrogen atoms by catalytic hydrogenolysis using palladium-on-barium sulphate in a suitable solvent such as an alcohol (e.g. ethanol).

Compounds of formula (V) may conveniently be prepared from compounds of formula (VI) by oxidation, using for example a suitable oxidising agent such as pyridinium chlorochromate in a halogenated hydrocarbon solvent (e.g. dichloromethane).

It will be appreciated that certain of the above reactions (e.g. hydrogenation, ozonolysis and isomerisation) may not be suitable for compounds in which $R^1$ contains one or two double bonds. In these cases the reaction will be effected on a compound containing an $R^1$ group stable under the conditions of the reaction. The reaction may then be followed by appropriate means described herein to introduce the desired $R^1$ grouping.

Compounds of formulae (VI) and (VII) in which one or more of $R^{4a}$, $R^{5a}$ and $R^{6a}$ represents a protecting group may be prepared from the corresponding carboxylic acid of formula (VI) or (VII) using standard protection methods.

According to a further process (C), compounds of formula (I) in which $R^2$ represents a group $-OCOR^7$ or $-OCO_2R^7$ may be prepared by reacting a compound of formula (IX)

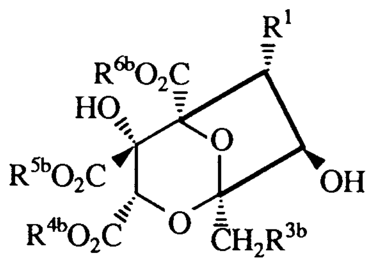

(IX)

(wherein $R^1$ is as defined previously, $R^{3b}$ is as defined for $R^3$ above or is a protected derivative thereof and $R^{4b}$, $R^{5b}$ and $R^{6b}$ are protecting groups) under conventional conditions for ester and carbonate formation, followed by removal of the protecting groups present. Thus, for example, the reaction may conveniently be effected by treating a compound of formula (IX) with a compound $R^7COHal$ or $R^7OCOHal$ as appropriate, wherein Hal represents a halogen atom such as chlorine or bromine.

The reaction with a compound $R^7COHal$ or $R^7OCOHal$ may conveniently be effected in the presence of 4-dimethylaminopyridine with or without a suitable base such as a tertiary amine (e.g. triethylamine) or using an alkali metal carbonate or alkaline earth metal carbonate (e.g. calcium carbonate) in a solvent such as a halogenated hydrocarbon (e.g. dichloromethane).

It will be appreciated that when $R^1$ in the compounds of formula (IX) represents a hydroxyl group this hydroxyl group will also be susceptible to ester and carbonate formation. Thus, in the preparation of compounds of formula (I) in which $R^1$ is a hydroxyl group it may be appropriate to have protected the $R^1$ hydroxyl group in compounds of formula (IX) or utilise a compound of formula (IX) in which $R^1$ is an acyloxy group, and following the reaction to form the 7-position ester or carbonate group remove the protecting group or deacylate as appropriate to provide the desired compound of formula (I) in which $R^1$ is hydroxyl.

Compounds of formula (I) in which $R^2$ represents an acetoxy group may conveniently be prepared from compounds of formula (IX) by reaction with acetic anhydride followed, if desired, by removal of the protecting groups present. The acetylation reaction may conveniently be effected in the presence of a suitable base such as a tertiary amine (e.g. triethylamine) in a solvent such as a halogenated hydrocarbon (e.g. dichloromethane). If excess acetic anhydride, base and 4-dimethylaminopyridine are used the reaction proceeds to provide a 4,7-diacetylated compound of formula (X)

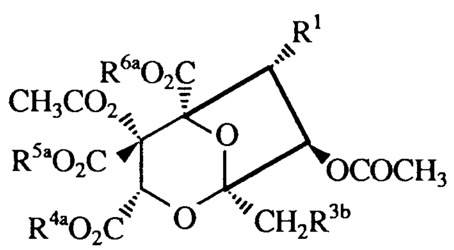

(X)

(wherein $R^1$, $R^{3b}$, $R^{4a}$, $R^{5a}$ and $R^{6a}$ are as defined previously).

Selective deacetylation of a compound of formula (X) in which $R^{4a}$, $R^{5a}$ and $R^{6a}$ are hydrogen atoms may conveniently be achieved by solvolysis in a suitable alcohol such as ethanol at a suitable temperature (e.g. room temperature) to provide the desired compounds of formula (I) in which $R^2$ is an acetoxy group.

According to another process (D), compounds of formula (I) in which $R^1$ represents an acyloxy, carbonate, carbamate or ether group may be prepared from compounds of formula (XI)

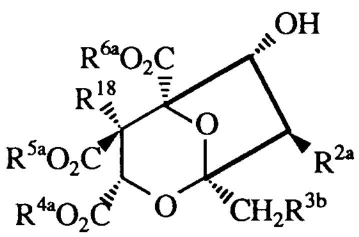

(XI)

(wherein $R^{2a}$ is as defined for $R^2$ above or is a protected hydroxyl group, $R^{3b}$ and $R^{4a}$-$R^{6a}$ are as defined previously and $R^{18}$ is a hydroxyl group or a protected hydroxyl group) by reaction to convert the 6 position hydroxyl group to an acyloxy, carbonate, carbamate or ether group, followed by removal of any protecting groups present.

Thus, for example, the acylation reaction may conveniently be effected by treating a compound of formula (XI) with an acyl halide (e.g. an acyl chloride) in the presence of 4-dimethylaminopyridine with or without a suitable base such as a tertiary amine (e.g. triethylamine) or using an alkali metal carbonate or alkaline earth metal carbonate (e.g. calcium carbonate) in a solvent such as a halogenated hydrocarbon (e.g. dichloromethane).

The reaction to form a carbonate may conveniently be effected by treating a compound of formula (XI) with a suitable haloformate (e.g. chloroformate) in the presence of dimethylaminopyridine with or without a suitable base such as tertiary amine (e.g. triethylamine) or using an alkali metal carbonate or alkaline earth metal carbonate (e.g. calcium carbonate) in a solvent such as a halogenated hydrocarbon (e.g. dichloromethane).

Compounds of formula (I) in which $R^1$ represents a group $-OCONR^{14}R^{15}$ may be prepared from compounds of formula (XI) by reaction with a suitable carbamoylating agent under conventional conditions, followed by removal of the protecting groups present.

Thus, for example, compounds of formula (I) in which $R^1$ represents a group $-OCONR^{14}R^{15}$ (where at least one of $R^{14}$ and $R^{15}$ is a hydrogen atom) may conveniently be prepared by treating a compound of formula (XI) with a suitable isocyanate in a solvent such as a halogenated hydrocarbon (e.g. dichloromethane) at a temperature in the range of $0^0$ to $20^0$C. When $R^1$ represents $-OCONH_2$ a compound of formula (XI) may be reacted with an isocyanate capable of generating the desired compound under appropriate work-up conditions. Suitable isocyanates include, for example, chlorosulphonyl isocyanate and chloroacetyl isocyanate. Compounds in which $R^1$ represents $-OCONHR^{15}$ where $R^{15}$ is other than a hydrogen atom may be prepared using an isocyanate $R^{15}NCO$.

Compounds of formula (I) in which $R^1$ represents a group $-OCONR^{14}R^{15}$ may also be prepared by treating a compound of formula (XI) with a reagent capable of converting the 6-OH group to a group -OCOX (where X is a leaving group such as a halogen atom, e.g. chlorine, or imidazole), followed by treatment with an amine $R^{14}R^{15}NH$ (including ammonia). Suitable reagents capable of effecting the desired conversion of $R^1$ to -OCOX include phosgene and carbonyldiimidazole. The reaction may conveniently be carried out in a solvent such as an ether (e.g. tetrahydrofuran) or a halogenated hydrocarbon (e.g. dichloromethane) at a reduced temperature (e.g. $-40^0$ to $0^0$C). When a reagent such as phosgene is used, the reaction is conveniently carried out in the presence of a non-nucleophilic organic base such as pyridine.

Compounds of formula (I) in which $R^1$ represents a group $-OCONR^{14}R^{15}$ where both $R^{14}$ and $R^{15}$ are other than hydrogen atoms may also be prepared from compounds of formula (XI) by treating (XI) with a reagent $HalCONR^{14}R^{15}$ where Hal is a halogen atom (e.g. chlorine) in a solvent such as a halogenated hydrocarbon (e.g. dichloromethane) in the presence of an organic base (e.g. pyridine).

The reaction to form an ether may conveniently be effected by treating a compound of formula (XI) with a halide (e.g. an alkyl halide) in the presence of a suitable base such as an alkali metal hydroxide (e.g. potassium hydroxide) in a solvent such as a sulphoxide (e.g. dimethylsulphoxide).

If it is desired to prepare compounds of formula (I) in which $R^2$ represents a hydroxyl group from compounds of formula (XI) then it may be necessary to have protected the 7-position hydroxyl group in compounds of formula (XI). Suitable protecting groups and methods of deprotection are described hereinafter.

Compounds of formula (IX) in which $R^1$ represents an acyloxy, carbonate, carbamate or ether group may conveniently be prepared from compounds of formula (XI) in which $R^{2a}$ represents a protected hydroxyl group using the procedure described in process (C) above, followed by removal of the protecting group(s) present.

Compounds of formula (XI) may conveniently be prepared from compounds of formula (XII)

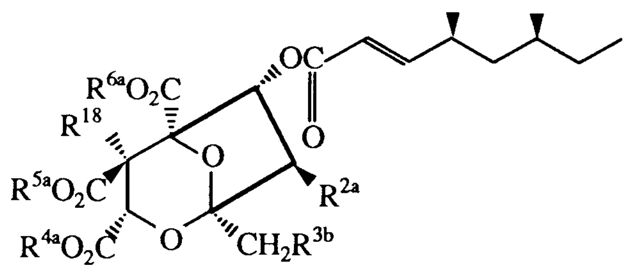

(XII)

(wherein $R^{2a}$, $R^{3b}$, $R^{4a}$-$R^{6a}$ and $R^{18}$ are as defined previously in formula (XI) above) by treating (XII) with a hydroxylamine (e.g. N-methylhydroxylamine hydrochloride) optionally in the presence of a suitable base (e.g. a trialkylamine such as triethylamine) in a solvent such as dimethylformamide.

Compounds of formula (IX) in which $R^1$ represents a hydroxyl group may also be prepared from compounds of formula (XII) in which $R^{2a}$ and $R^{18}$ represent hydroxyl groups, $R^{3b}$ is as defined previously and $R^{4a}$-$R^{6a}$ are protecting groups according to the deacylation procedure described above for the preparation of compounds of formula (XI) from compounds of formula (XII).

Compounds of formula (XII) in which $R^{2a}$ and $R^{18}$ represent protected hydroxyl groups and/or $R^{3b}$ represents a protected $R^3$ group may conveniently be prepared from the corresponding compounds of formula (XII) in which $R^{2a}$ and $R^{18}$ represent hydroxyl groups and/or $R^{3b}$ represents a group $R^3$. Suitable hydroxyl protecting groups are described hereinafter.

Compounds of formula (XII) in which $R^{2a}$ represents a group $-OCOR^7$ or $-OCO_2R^7$ may be prepared from the corresponding compounds of formula (XII) in which $R^{2a}$ represents a hydroxyl group according to the general procedure described hereinabove for the preparation of compounds of formula (I) from compounds of formula (IX).

Compounds of formula (XII) in which $R^{2a}$ and $R^{18}$ represent hydroxyl groups and $R^{3b}$ represents a group $R^3$ may conveniently be prepared from compounds of formula (XIII)

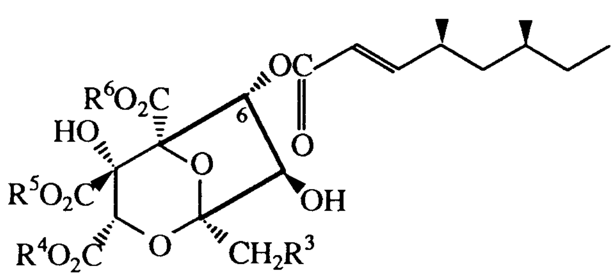

(XIII)

(wherein $R^3$-$R^6$ are as defined in formula (I) above) by standard carboxylic acid protection methods.

Compounds of formula (XI) in which $R^{2a}$ and $R^{18}$ represent hydroxyl groups and $R^{3b}$ is as defined for $R^3$ may also be prepared from compounds of formula (XIV)

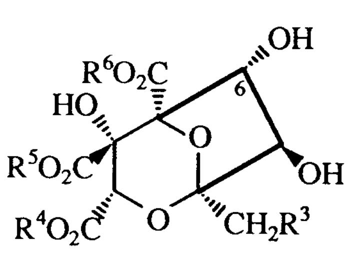

(XIV)

(wherein $R^3$-$R^6$ are as defined in formula (I) above) by standard carboxylic acid protection methods.

Suitable carboxylic acid protecting groups for $R^{4a}$, $R^{5a}$ and $R^{6a}$ and hydroxyl protecting group where

required include any conventional protecting group, for example as described in 'Protective Groups in Organic Chemistry', Ed. J. F. W. McOmie (Plenum Press, 1973) or 'Protective Groups in Organic Synthesis' by Theodora W. Greene (John Wiley and Sons, 1981). Examples of suitable carboxylic acid protecting groups include alkyl groups such as t-butyl, 2-methoxyethoxymethyl or aralkyl groups such as benzyl, diphenylmethyl or p-nitrobenzyl. Examples of suitable hydroxyl protecting groups include groups such as 2-methoxyethoxymethyl.

The protecting groups may be removed using conventional techniques. Thus, an alkyl group such as t-butyl may, for example, be removed under anhydrous acid conditions (for example using hydrogen chloride in a solvent such as an ether, e.g. dioxan). An aralkyl group may conveniently be removed by catalytic hydrogenation using for example a suitable metal catalyst such as palladium-on-carbon. Alternatively, a p-nitrobenzyl group may conveniently be removed using zinc metal and hydrochloric acid in a solvent such as an ether (e.g. tetrahydrofuran or aqueous tetrahydrofuran). A diphenylmethyl group or a 2-methoxyethoxymethyl group may conveniently be removed using aqueous formic acid or trifluoroacetic acid.

Esterification of appropriate compounds of formulae (VI), (VII), (VIII), (XIII) and (XIV) to the corresponding methyl esters may conveniently be effected by treatment with a methylating agent such as a methyl halide (e.g. methyl iodide) or dimethyl sulphate in a suitable organic solvent such as an amide (e.g. dimethylacetamide or preferably dimethylformamide) in the presence of a base such as a bicarbonate (e.g. sodium bicarbonate). The reaction may conveniently be carried out at a temperature ranging from $0^0$ to $100^0C$, preferably $20^0$ to $30^0C$. Alternatively, the esterification may be effected by treatment with an ethereal solution of diazomethane in a suitable solvent such as methanol. The esterification may also be effected by treatment with methanol in the presence of a suitable acid such as a mineral acid (e.g. hydrochloric acid) at about room temperature.

Conversion of one methyl ester of formula (VI) or (VII) or (VIII) or (XIII) or (XIV) to a different methyl ester may be carried out by appropriate esterification/deesterification steps. The deesterification may be effected under standard conditions, for example by base hydrolysis.

Compounds of formulae (VI) and (VII) in which $R^{4a}$, $R^{5a}$ and $R^{6a}$ represent $R^4$, $R^5$ and $R^6$ respectively as defined for compounds of formula (I) above, $R^1$ represents a hydrogen atom, a hydroxyl group or a group selected from $-OCOCH \overset{E}{=} CHCH(CH_3)(CH_2)_3CH_3$, $-OCOCH \overset{E}{=} CHC(CH_3) \overset{E}{=} CHCH(CH_3)CH_2CH_3$, or $-OCO-X-CH_2CH(CH_3)CH_2CH_3$ [where X is $-CH \overset{E}{=} CHCH(CH_3)-$, $-CH_2CH(OH)CH(CH_3)-$, $-CH \overset{E}{=} CHC(OH)(CH_3)-$, $-CH_2CH(OH)CH_2-$ or $-CH_2CH_2CH(CH_3)-$] and $R^2$ represents a hydrogen atom or a hydroxyl group and compounds of formulae (XIII) and (XIV) in which $R^3$ represents

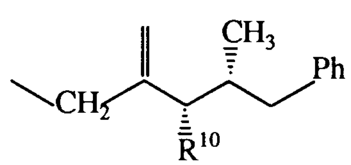

(where $R^{10}$ a hydroxyl or acetoxy group), $-CH_2C(CH_3) \overset{E}{=} CHCH(CH_2R^{13})CH_2Ph$,
$-CH_2C(CH_2OH) \overset{Z}{=} CHCH(CH_3)CH_2Ph$,
$-CH_2C(=CH_2)CH(OH)CH(CH_2OH)CH_2Ph$,
$-CH_2C(=CH_2)CH(NHCOCH_3)CH(CH_3)CH_2Ph$,
$-CH_2C(CH_2NHCOCH_3) \overset{E}{=} CHCH(CH_3)CH_2Ph$ or

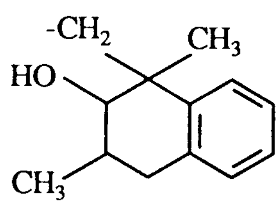 ;

and compounds of formula (VIII) in which $R^{4a}$, $R^{5a}$ and $R^{6a}$ are hydrogen atoms, $R^1$ is hydrogen, hydroxy or an acyloxy group as defined in formula (I) above, $R^2$ is a hydrogen atom or a hydroxyl group and the C-1 side chain double bond is in the E configuration may be prepared according to the fermentation process

described hereinafter or may be prepared from products of the fermentation process by acylation or deacylation at the 6-position as appropriate according to previously described acylation and deacylation methods. Other compounds of formulae (VI), (VII), (XIII) and (XIV) may be prepared from the fermented compounds of formulae (VI), (VII), (XIII) and (XIV) or acylated or deacylated derivatives thereof by modification of the $R^3$ grouping according to the general procedures described hereinabove.

Microorganisms capable of producing a compound of the formula (VI), (VII), (VIII), (XIII) or (XIV) may readily be identified by using a small scale test and analysing a test sample obtained from fermentation of the microorganism using standard methodology.

In particular the microorganism to be conventionally used is a strain of microorganism deposited on 31st May 1989 in the culture collection of Glaxo Group Research Limited, Microbiology Division, Greenford Road, Greenford, Middlesex, England, UB6 0HE (collection number 202 in the World Directory of Collections of Cultures of Microorganisms, 1982; curator: Miss A M Harris) under accession no. C2932 or a mutant thereof. It is to be understood that the above mentioned culture collection centre has given its unreserved and irrevocable consent to the microorganism deposited being made available to any person making a valid request therefor to the culture collection in accordance with Rule 17 of the UK Patents Rules 1982.

The strain deposited at Greenford under accession no. C2932 has also been deposited in the permanent culture collection of the CAB International Mycological Institute, Ferry Road, Kew, Surrey, England. The strain was received by the Institute on 25th May 1989 and was subsequently given the accession no. IMI 332962 and a deposit date of 27th June 1989 (date of confirmation of viability). The deposited strain is identified herein by reference to the Institute accession no. IMI 332962. The characteristics thus far identified for IMI 332962 are given in Example 68 hereinafter.

It will be appreciated that the desired intermediates may also be prepared from a mutant of IMI 332962.

Mutants of the IMI 332962 may arise spontaneously or may be produced by a variety of methods including those outlined in Techniques for the Development of Micro-organisms by H. I. Adler in 'Radiation and Radioisotopes for Industrial Microorganisms', Proceedings of the Symposium, Vienna 1973, p241, International Atomic Energy Authority. Such methods include ionising radiation, chemical methods e.g. treatment with N-methyl-N'-nitro-N-nitrosoguanidine (NTG), heat, genetic techniques, such as recombination and transformation, and selective techniques for spontaneous mutants.

The production of compounds of the formulae (VI), (VII), (VIII), (XIII) and (XIV) by fermentation of a suitable organism may be effected by conventional means i.e. by culturing the organism in the presence of assimilable sources of carbon, nitrogen and mineral salts.

Assimilable sources of carbon, nitrogen and minerals may be provided by either simple or complex nutrients. Sources of carbon will generally include glucose, maltose, starch, glycerol, molasses, dextrin, lactose, sucrose, fructose, galactose, myo-inositol, D-mannitol, soya bean oil, carboxylic acids, amino acids, glycerides, alcohols, alkanes and vegetable oils. Sources of carbon will generally comprise from 0.5 to 10% by weight of the fermentation medium. Fructose, glucose and sucrose represent preferred sources of carbon.

Sources of nitrogen will generally include soya bean meal, corn steep liquors, distillers solubles, yeast extracts, cottonseed meal, peptones, ground nut meal, malt extract, molasses, casein, amino acid mixtures, ammonia (gas or solution), ammonium salts or nitrates. Urea and other amides may also be used. Sources of nitrogen will generally comprise from 0.1 to 10% by weight of the fermentation medium.

Nutrient mineral salts which may be incorporated into the culture medium include the generally used salts capable of yielding sodium potassium, ammonium, iron, magnesium, zinc, nickel, cobalt, manganese, vanadium, chromium, calcium, copper, molybdenum, boron, phosphate, sulphate, chloride and carbonate ions.

Cultivation of the organism will generally be effected at a temperature of from 20 to 40$^0$C preferably from 20 to 35$^0$C, especially around 25 to 28$^0$C, and will desirably take place with aeration and agitation e.g. by shaking or stirring. The medium may initially be inoculated with a small quantity of mycelium and/or spores. The vegetative inoculum obtained may be transferred to the fermentation medium, or to one or more seed stages where further growth takes place before transfer to the principal fermentation medium. The fermentation will generally be carried out in the pH range 3.5 to 9.5, preferably 4.5 to 7.5. It may be necessary to add a base or an acid to the fermentation medium to keep the pH within the desired range. Suitable bases which may be added include alkali metal hydroxides such as aqueous sodium hydroxide or potassium hydroxide. Suitable acids include mineral acids such as hydrochloric, sulphuric or phosphoric acid.

The fermentation may be carried out for a period of 4-30 days, preferably about 7-18 days. An antifoam may be present to control excessive foaming and added at intervals as required. Carbon and/or nitrogen

sources may also be fed into the fermentation medium as required.

Compounds of formulae (VI), (VII), (VIII), (XIII) and (XIV) may be present in both the fermentation liquor and the mycelial fraction, which may conveniently be separated by filtration or centrifugation. The liquor may be optionally thereafter treated with an acid such as sulphuric acid in the presence of an organic solvent until the pH is below pH 6 (e.g. about pH 3).

Compounds of formulae (VI), (VII), (VIII), (XIII) and (XIV) may be separated from the fermentation broth by conventional isolation and separation techniques. It will be appreciated that the choice of isolation techniques may be varied widely.

Compounds of formulae (VI), (VII), (VIII), (XIII) and (XIV) may be isolated and purified by a variety of fractionation techniques, for example adsorption-elution, precipitation, fractional crystallisation, solvent extraction and liquid-liquid partition which may be combined in various ways.

Adsorption onto a solid support followed by elution has been found to be suitable for isolating and purifying compounds of the invention.

Compounds of formulae (VI), (VII), (VIII), (XIII) and (XIV) may be extracted from the cells and the aqueous phase with an appropriate organic solvent such as a ketone (e.g. acetone, methyl ethyl ketone or methyl isobutyl ketone), a halogenated hydrocarbon, an alcohol, a diol (e.g. propane-1,2-diol or butane-1,3-diol) or an ester (e.g. methyl acetate or ethyl acetate). Generally, more than one extraction may be desirable to achieve optimum recovery. The water-immiscible solvent extracts may themselves be extracted with basic aqueous solutions, and after acidification of these basic solutions the desired compounds may be reextracted into water-immiscible organic phase. Removal of the solvent from the organic extracts (e.g. by evaporation) yields a material containing the desired compounds.

Chromatography (including high performance liquid chromatography) may be effected on a suitable support such as silica; a non-functional macroreticular adsorption resin for example cross-linked styrene divinyl benzene polymer resins such as Amberlite XAD-2, XAD-4, XAD-16 or XAD-1180 resins (Rohm & Haas Ltd) or Kastell S112 (Montedison); a substituted styrene-divinyl benzene polymer, for example a halogenated (e.g. brominated) styrene-divinyl benzene polymer such as Diaion SP207 (Mitsubishi); an anion exchanger (e.g. IRA-35 or IRA-68) an organic solvent-compatible cross-linked dextran such as Sephadex LH20 (Pharmacia UK Ltd), or on reverse phase supports such as hydrocarbon linked silica e.g. $C_{18}$-linked silica. An alternative chromatographic means for the purification/separation of compounds of formulae (VI), (VII), (VIII), (XIII) and (XIV) is countercurrent chromatography using a coil extracter such as a multi-layer coil extracter.

Compounds of formulae (VI), (VII), (VIII), (XIII) and (XIV) may also be isolated and purified by the use of a liquid anion exchanger such as LA 2.

When IRA-68 or, particularly, IRA-35 is used as the solid adsorbant the cell extracts may be loaded directly without removal of solvent. The extract may either be loaded directly at about pH3 or at about pH8 following filtration of solid impurities.

Suitable solvents/eluants for the chromatographic purification/ separation of compounds of formulae (VI), (VII), (VIII), (XIII) and (XIV) will, of course, depend on the nature of the column type and support. When using countercurrent chromatography we have found a solvent system comprising ethyl acetate, hexane, methanol and an aqueous acid (e.g. aqueous sulphuric acid) to be particularly suitable. When using an anion exchanger such as IRA-35 the resin may conveniently be washed with aqueous acetone followed by elution with sulphuric acid in aqueous acetone.

The presence of compounds of formulae (VI), (VII), (VIII), (XIII) and (XIV) during the extraction/isolation procedures may be monitored by conventional techniques such as h.p.l.c. or UV spectroscopy or by utilising the properties of the compounds.

Where a compound of formula (VI), (VII), (VIII), (XIII) or (XIV) is obtained in the form solution in an organic solvent, for example after purification by chromatography, the solvent may be removed by conventional procedures, e.g. by evaporation, to yield the required compound. If desired, the compound may be further purified by the aforementioned chromatographic techniques.

Acylation to provide a compound of formula (XIII) may conveniently be effected by treating a corresponding compound of formula (XIV) or a protected derivative thereof with an acid of formula (XV)

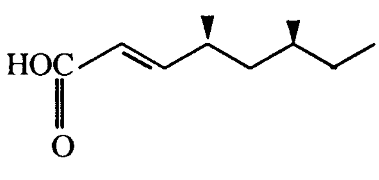

(XV)

or an activated derivative thereof such as the corresponding acid chloride under conventional esterfication conditions followed by removal of any protecting groups present. The acylation reaction may conveniently be carried out in the presence of 4-dimethylaminopyridine with or without a suitable base such as a tertiary amine (e.g. triethylamine) or using an alkali metal carbonate or alkaline earth metal carbon (e.g. calcium carbonate) in a solvent such as a halogenated hydrocarbon (e.g. dichloromethane).

The compound of formula (XV) may conveniently be prepared by hydrolysis of a compound of formula (XIII), for example by base catalysed hydrolysis using a base such as aqueous sodium hydroxide in a solvent such as an alcohol (e.g. methanol).

Deacylation of compounds of formulae (VI), (VII), (VIII) and (XIII) may be effected by acid or base catalysed hydrolysis. Suitable bases include hydroxides such as sodium hydroxide and potassium hydroxide and alkoxides (e.g. methoxides). The base catalysed hydrolysis may take place in water optionally in the presence of an organic co-solvent such as an ether (e.g. tetrahydrofuran) or an alcohol (e.g. methanol) at a temperature in the range of $0^0$ to $100^0$C, preferably at elevated temperature. When an alkoxide is used as the base the reaction may conveniently be effected in an alcohol solvent (e.g. methanol) at a temperature in the range of $0^0$ to $100^0$C. Suitable acids include mineral acids (e.g. hydrochloric acid) and organic acids (e.g. p-toluenesulphonic acid). The acid catalysed hydrolysis may be carried out in water optionally in the presence of an organic co-solvent such as an ether (e.g. dioxan or tetrahydrofuran) or a ketone (e.g. acetone) at a temperature in the range of $0^0$ to $100^0$C, preferably at room temperature.

Compounds of formulae (VI) and (VII) where $R_1$ represents a hydroxyl group may conveniently be prepared from the corresponding compounds where $R_1$ represents an acyloxy group as previously defined in formula (I) using the following procedure. Thus, for example, when $R_1$ represents a substituted 2(E)-octenoyl group this may be converted to a hydroxyl group by treatment with a hydroxylamine (e.g. N-methylhydroxylamine) in the presence of a base (e.g. an organic base such as triethylamine) in a suitable organic solvent such as an amide (e.g. dimethylformamide) at a temperature ranging from, for example, $0^0$ to $50^0$C, preferably $20^0$ to $30^0$C.

It is to be understood that the acylation or deacylation and esterification processes may be combined as sequential or simultaneous reaction steps as appropriate.

Salts of compounds of formula (I) may be conveniently formed by treating a compound of formula (I) with an appropriate salt or base. Thus, for example, salts may conveniently be prepared by treating a compound of formula (I) with a salt or a base selected from sodium or potassium hydroxide, hydrogen carbonate, carbonate or acetate (e.g. potassium hydroxide, potassium hydrogen carbonate, sodium hydrogen carbonate or potassium acetate), ammonium acetate, calcium acetate and L-lysine as appropriate. The salt may, for example, be prepared by adding the appropriate salt or base (if necessary as an aqueous solution) to a solution or suspension of the compound of formula (I) in a suitable solvent such as water and/or a cosolvent such as an alcohol (e.g. methanol), a nitrile (e.g. acetonitrile) or a ketone (e.g. acetone) at temperatures of for example $0^0$C to $80^0$C and conveniently at about room temperature.

The compounds of formulae (II), (IV), (V), (X) and (XV) are novel intermediates and form a further aspect of the present invention. Novel intermediates of formulae (VI), (VII), (VIII), (IX), (XI), (XII), (XIII) and (XIV) form another aspect of the present invention.

The following examples are provided by way of illustrating the invention and are not intended to limit the invention in any way.

Intermediate 1

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxyic acid, 6-(4,6-dimethyl-2-octenoate)[Compound A] and [1S-[1α(4R*,5S*),3α,4β,5α,6α (2E,4R*,6R*),7β]] 1-(4-hydroxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate) [Compound B]

(a) IMI 332962 was grown on agar plates of the following composition:

| | |
|---|---|
| Malt extract (Oxoid L39) | 30g |
| Mycological peptone (Oxoid L40) | 5g |
| Yeast extract (Oxoid L21) | 0.5g |
| Agar (Oxoid No 3) | 20g |
| Distilled water to 1 litre | |

The pH of the medium before autoclaving was in the range of 5.3-5.5. The inoculated plates were incubated at 28$^0$C for 14 days. Several 6mm diameter plugs of agar covered with fungal mycelium were cut from the growing edge of the culture and two plugs were transferred into each of several cryotubes containing 1.6ml of sterile distilled water. The tubes were capped and stored at room temperature until required.

Two agar plugs were used to inoculate each of eight 50ml aliquots of seed medium (A) contained in 250ml Erlenmeyer flasks:

| Seed medium (A): | |
|---|---|
| Peptone (Oxoid L34)<br>Malt extract (Oxoid L39)<br>Glycerol<br>Junlon 110 (Honeywill & Stein Ltd., Wallington, Surrey) | 10g<br>21g<br>40g<br>1g |
| Distilled water to 1 litre | |

The pH of the medium was adjusted to 6.3-6.5 with aqueous sodium hydroxide before autoclaving

The flasks of inoculated seed medium were incubated at 25$^0$C on a shaker platform, which rotated at 250rpm with a 50mm diameter orbital motion, for 5 days.

The contents of the flasks were pooled and homogenised. The homogenised seed culture was used at 3% (v/v) to inoculate 120, 50ml aliquots of fermentation medium (B) in 250ml Erlenmeyer flasks:

| Fermentation medium (B) : | |
|---|---|
| Glycerol<br>Soyabean oil<br>Cottonseed flour (Sigma) | 50g<br>30g<br>10g |
| Distilled water to 1 litre | |

The pH of the medium before autoclaving was in the range 6.1-6.3. The flasks were incubated as above with shaking for 8 days.

The fermentation broth (approximately 6L) from flasks incubated for 8 days was filtered to remove the mycelium and the filtrate adjusted to pH 2.8 with sulphuric acid (20% v/v) and extracted with 3 x 2 volumes of ethyl acetate. The ethyl acetate extracts were bulked and back extracted with 2 x 400ml of aqueous sodium hydrogen carbonate solution (1% w/v). The aqueous back extracts were bulked, adjusted to pH 2.8 as above and re-extracted into 2 x 800ml of ethyl acetate. These extracts were combined and evaporated to dryness to yield a brown oil. This oil was further processed by countercurrent chromatography using an Ito Multi-layer Coil Extractor (P. C. Inc., Potomac, Maryland, USA). The coil used was the standard preparative coil consisting of approximately 70 metres of 2.6mm internal diameter PTFE tubing giving a total volume of about 380ml. The solvent system used was a mixture of ethyl acetate, hexane, methanol and N/100 sulphuric acid (6:5:5:6 by volume). The lower phase was kept stationary. The coil was filled with the lower phase using a Gilson Model 303 pump and a Model 804C Manometric Module (Gilson, Villiers Le Bel, France). The oil (497mg in 4ml of the upper phase + 4ml of the lower phase) was then injected at the "tail" end of the column. The centrifuge was then operated at 800 rev./min. and the mobile (upper) phase pumped at 4ml/min. from the "tail" end of the column. 20ml fractions were collected and monitored by measuring inhibition of squalene synthase.

Consecutive fractions showing activity against squalene synthase were bulked. The earlier fractions were evaporated to dryness to yield the title Compound A (90mg) as a pale yellow oil.

The later fractions were pooled with an additional fraction (46mg, yellow oil) obtained by similar processing of another batch (5L) of fermentation broth produced as described in Example 3 below. The bulked material was further processed by countercurrent chromatography using as solvent a mixture of ethyl acetate, hexane, methanol and N/100 sulphuric acid (3:2:2:3 by volume) with the lower phase kept stationary. The instrument was operated and fractions were collected and monitored as described previously and the mobile (upper phase) was pumped at 5ml/min. The biologically active fractions were bulked and evaporated to druyness to yield title Compound B (33mg) as a pale yellow oil.

(b) The mycelium separated from 6L broth, from flasks incubated for 8 days according to the procedure in part (a) above, was extracted with methanol (2 x 3L) and filtered. The filtrate was concentrated by evaporation to ca. 500ml, adjusted to pH 3.0 with formic acid and extracted with 3 x 500ml of ethyl acetate. The ethyl acetate extracts were bulked and back extracted with 2 x 200ml of sodium hydrogen carbonate solution (1% w/v). The aqueous back extracts were bulked, adjusted to pH 3.0 and re-extracted into 2 x 500ml of ethyl acetate. All the organic fractions were combined and reduced to dryness using a rotary evaporator to yield a brown oil. The oil (578mg) was further processed by high peformance liquid chromatography (HPLC) using a Gilson autopreparative system composed of 3 Gilson solvent delivery pumps (model 303), an 811 Dynamic mixer and an 802C manometric module. The chromatography was carried out on a Dynamax Microsorb C18 (5$\mu$m) semi-preparative column (250 x 10mm). The mobile phase was a gradient composed of acetonitrile and 0.1% v/v formic acid to pH 3.15 with ammonium acetate (1:3 → 4:1 → 1:3) pumped at 2.8-5.6ml/min with a run time of 65 minutes. This method was repeated 16 times. 13 x 4.95 minute fractions were collected and monitored by measuring inhibition of squalene synthase. Fraction number 5 from each run was bulked, acidified to pH 3.0 with formic acid and extracted with 2 x 100ml ethyl acetate. The organic phase was removed and evaporated to dryness to yield the title Compound A (172mg) as a pale yellow oil.

(c) (i) Eight 0.5ml aliquots from a 5 day old fermentation carried out as in part (a) above were used to inoculate eight 50ml aliquots of seed medium (A) contained in 250ml Erlenmeyer flasks. The flasks were incubated at 25$^0$C on a shaker platform, which rotated at 250rpm with a 50mm diameter orbital motion, for 4 days. The contents of the flasks were pooled and homogenised.

The homogenised seed culture was used at 3% (v/v) to inoculate 120, 50ml aliquots of fermentation medium (B) in 250ml Erlenmeyer flasks. The flasks were incubated with shaking as above for 10 days.

(c) (ii) Homogenised seed culture prepared as in part (c)(i) above were used at 3% (v/v) to inoculate two fermentation vessels, each of 5 litres capacity, containing 3 litres of fermentation medium (B). The inoculated medium was maintained at 25$^0$C and agitated with two six bladed turbine impellers (70mm diameter) rotating at 500 rpm. The culture was aerated by sparging with sterile air at 3 Lpm. Provision was made for control of excessive foaming of the culture by the addition of silicone antifoam (Dow Corning 1520). The contents of the two culture vessels were combined after 11 days growth and further processed by countercurrent chromatography according to the procedure in part (a) above to give the title Compound A (137mg); 500MHz proton nmr in deutero-methanol includes signals at about $\delta$ 0.84-0.90 (m,9H), 1.03 (d,7,3H), 1.09-1.19 (m,2H), 2.10 (s,3H), 2.24 (m,1H), 2.34 (m,1H), 2.68 (dd,13,6,1H), 4.04 (d,2,1H), 4.97 (s,1H), 5.02 (s,1H), 5.08 (d, 5,1H), 5.27 (s,1H), 5.80 (d,16,1H), 6.31 (d,2,1H), 6.85 (dd,16,8,1H), 7.14 (t,7,1H), 7.19 (d,7,2H), 7.26 (t,7,2H); composite pulse decoupled 125.75 MHz carbon-13 nmr in deutero-methanol includes peaks at about $\delta$ 172.5 (0), 172.1(0), 170.1(0), 168.5(0), 166.5 (0), 157.6 (1), 147.7 (0), 141.6 (0), 130.2 (1), 129.3 (1), 126.9 (1), 119.8 (1), 111.5 (2), 106.8 (0), 91.1 (0), 82.5 (1), 81.0 (1), 80.1 (1), 76.6 (1), 75.6 (0), 44.4 (2), 40.9 (2), 37.7 (1), 35.6 (1), 34.9 (2), 33.1 (1), 30.8 (2), 26.5 (2), 20.9 (3), 20.5 (3), 19.2 (3), 14.1 (3), 11.4 (3).

(d) (i) Frozen stocks of inoculum were prepared from a 5 day old fermentation carried out as in part (a) above. Samples of culture were centrifuged for 10 min and the mycelium resuspended to the original volume in 15% glycerol and 0.01% Tween 80. The mycelium was spun down and resuspended again before being distributed in 1.8ml amounts in plastic tubes and stored at $^-20^0$C. Eight 0.5ml aliquots of frozen inoculum were used to inoculate eight 50ml aliquots of seed medium (A) contained in 250ml Erlenmeyer flasks. The flasks were incubated at 25$^0$C on a shaker platform, which rotated at 250rpm with a 50mm diameter orbital motion, for 4 days. The contents of the seed flasks were pooled and used at 3% (v/v) to inoculate 120 50ml aliquots of fermentation medium (B) in 250 ml Erlenmeyer flasks. The flasks were incubated with shaking as above for 9 days.

(d) (ii) The contents of 4 final stage flasks grown as in part (d)(i) above were pooled after 7 days incubation and homogenised to provide the seed for 120 50ml aliquots of fermentation medium (B) which were incubated for 8 days as in parts (c)(i) and (d)(i) above. The fermentation broth (approximately 6L) from flasks incubated for 8 days was filtered to remove the mycelium. The filtrate was adjusted to pH 2.8 with sulphuric acid (20% v/v) and extracted into ethyl acetate, back extracted into sodium hydrogen carbonate and re-extracted into ethyl acetate at pH 2.8 as described in part (a) above. The ethyl acetate extract was concentrated under reduced pressure to a yellow oil which was dissolved in methanol (10ml). This solution was evaporated to 3ml and applied to a column (32 x 2.5cm) of ODS-3 (Whatman Partisil Bioprep 40, 75 Angstrom, slurry packed in acetonitrile-water, 20:80). The column was eluted with a stepwise gradient of a mixture of acetonitrile and water, increasing the proportion of acetonitrile as follows : 1:4, 3:7, 2:3, 1:1, 3:2. Fractions were monitored by HPLC and those containing the title Compound A were evaporated to remove acetonitrile. The resulting aqueous suspensions were pooled

and freeze dried overnight to yield the title Compound A (59mg) as an off-white solid. Compound B may also be prepared as a solid using a similar freeze drying technique.

(e) The procedure in part (d)(i) was followed except that the pooled seed flasks were used at 3% (v/v) to inoculate 4 litres of seed medium (A) in a 7L fermenter. The culture was incubated with agitation as above at 500rpm for 2 days with the culture aerated at 4L/min. 1.2L of the culture was removed and used to inoculate a 70L fermenter filled with 40L seed medium (A). The culture was incubated as above at 500rpm for 2 days with the culture aerated at 40L/min. 15L of the culture was removed and added to a 780L fermenter filled with 500L fermentation medium (C).

| Fermentation medium (C) : | |
|---|---|
| Fructose | 50g |
| Soyabean oil | 30g |
| Cottonseed flour (sigma) | 20g |
| Natural pH | |

The culture was incubated with shaking as above at 200rpm for 450h with the culture aerated at 500L/min and fed at 120h with a 50% (w/v) solution of fructose at 5g/L/day increasing to 7.5g/L/day at 162h. Analysis of the broth at 450h indicated a yield of the title Compound A of 1056 mg/L.

The above procedure was repeated on a reduced scale but replacing fructose with other sources of carbon selected from glucose, galactose, sucrose, maltose, lactose, myo-inositol, D-mannitol and soyabean oil. Analysis of the broth from each experiment at 450h indicated a substantial titre of the title Compound A.

Compound A prepared according to the above procedures was consistent with a product having the following characterising features:

Approximate molecular weight 690; -FAB mass spectrometry [M-H]-689.2789; +FAB mass spectrometry [M+Na)+ 713.2753; Molecular formula $C_{35}H_{46}O_{14}$.

500 MHz proton nmr spectrum in deutero-chloroform [δ values with multiplicities, coupling constants ($H_z$) and integration values in parenthesis]:0.79 to 0.85 (m,9H), 1.00 (d,7,3H), 1.04 to 1.15 (m,2H), 2.09 (s,3H), 2.40 (m,1H), 2.69 (dd,13,5,1H), 4.05 (s,1H), 4.94 (s,1H), 4.96 (s,1H), 5.06 (d,4,1H), 5.30 (s,1H), 5.78 (d,16,1H), 5.92 (s,1H), 6.88 (dd,16,8,1H), 7.11 (d,7,2H), 7.14 (t,7,1H), 7.24 (t,7,2H).

Composite pulse decoupled 125.75MHz carbon-13 nmr spectrum in deutero-chloroform [δ values with the number of attached protons in parenthesis]:171.5(0), 171.0 (0), 169.1 (0), 167.0 (0), 166.7 (0), 157.9 (1), 145.4 (0), 140.1 (0), 128.9 (1), 128.1 (1), 125.8 (1), 117.8 (1), 111.4 (2), 105.8 (0), 88.5 (0), 81.6 (1), 80.7 (1), 79.3 (1), 75.1 (1), 74.2 (0), 42.9 (2), 39.7 (2), 36.7 (1), 34.2 (1), 33.6 (2), 31.6 (1), 29.4 (2), 25.4 (2), 20.9 (3), 19.8 (3), 18.8 (3), 13.5 (3), 10.9 (3).

Compound B prepared according to the above procedure was consistent with a product having the following characterising features:

Approximate molecular weight 648; -FAB mass spectrometry [M-H]-647.2708; +FAB mass spectrometry [M+Na}+ 671; Molecular formula $C_{33}H_{44}O_{13}$; electron impact (EI) mass spectrometry gave the following fragment ions: m/z 91, 170, 497.

500 MHz proton nmr spectrum in deutero-methanol [δ values with multiplicities, coupling constants ($H_z$) and integration values in parenthesis] : 0.80 to 0.90 (m,9H), 1.03 (d,7,3H), 1.05 to 1.19 (m,2H), 2.28 (m,1H), 2.37 (dd,13,9,1H), 2.76 (dd,13,5,1H), 3.92 (d,5,1H), 4.08 (d,2,1H), 5.00 (s,1H), 5.10 (s,1H), 5.27 (s,1H), 5.78 (d,16,1H), 6.31 (d,2,1H), 6.84 (dd,16,8,1H), 7.13 (t,7,1H), 7.21 (d,7,2H), 7.24 (t,7,2H).

Composite pulse decoupled 125.75MHz carbon-13 nmr spectrum in deutero-methanol [δ values with the number of attached protons in parenthesis] : 172.4 (0), 170.1 (0), 168.4 (0), 166.5 (0), 157.6 (1), 152.0 (0), 142.5 (0), 130.2 (1), 129.2 (1), 126.6 (1), 119.8 (1), 110.7 (2), 107.1 (0), 91.0 (0), 82.3 (1), 81.0 (1), 78.6 (1), 76.6 (1), 75.6 (0), 44.4 (2), 41.2 (2), 39.0 (1), 35.6 (1), 35.1 (2), 33.1 (1), 30.8 (2), 26.0 (2), 20.5 (3), 19.2 (3), 14.0 (3), 11.4 (3).

Intermediate 2

[1S-[1α(4R*,5S*),3α,4β,5α,6α(2E,4R*,6R*),7β]] 1-(4-Hydroxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)methyl] ester

To a stirred solution of freeze dried Compound B of Intermediate 1 (2.50g) and triethylamine (1.78ml) in

N,N-dimethylformamide (20ml) was added 4-nitrobenzyl bromide (2.75g) and the resulting mixture left to stir at room temperature for 22h. The mixture was diluted with 2M aqueous hydrochloric acid (450ml) and extracted with ethyl acetate (3x150ml). The extracts were combined, washed with saturated aqueous sodium bicarbonate (2x150ml) and brine (2x150ml), dried over magnesium sulphate and evaporated to a foam. Flash column chromatography on silica gel (Merck 9385, 700ml) eluting with ethyl acetate:cyclohexane (1:2) gave the title compound (2.98g) as a cream-yellow foam; δ ($CDCl_3$) includes 0.77-0.90(m,9H,$CH_3$), 1.02-(d,3H,J=7Hz,$CH_3$), 4.98-5.38(m,Ar$CH_2$,C=$CH_2$,3H, 5.48(d,1H,J=15Hz,CH=$CHCO_2$), 5.86(broad s,1H,6H), 6.85(dd,1H,J=15,7Hz,CH=$CHCO_2$), 7.10-7.28(m,aromatic protons of phenyl ring), 7.37-7.52(m,6H,2 and 6 protons of 4-nitrophenyl rings), 8.08-8.23(m,6H,3 and 5 protons of 4-nitrophenyl rings).

Intermediate 3

[1S-[1α(5S*),3α,4β,5α,6α(2E,4R*,6R*),7β]] 1-(5-Methyl-3-methylene-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)-methyl] ester

To a solution of Intermediate 2 (2.85g) in dichloromethane (75ml) was added a solution of pyridinium chlorochromate (874mg) in dichloromethane (75ml) and the resulting mixture left to stir at room temperature for 19h. The mixture was diluted with diethyl ether (100ml) and filtered through kieselguhr in vacuo. The filtrate was evaporated to a foam. Flash column chromatography on silica gel (Merck 9385, 700ml) eluting with ethyl acetate:cyclohexane (1:3) gave the title compound (2.47g) as a cream foam; δ ($CDCl_3$) includes 0.79-0.87(m,6H,$CH_3$), 1.01(d,3H,J=7Hz,$CH_3$), 1.08(d,3H,J=7Hz,$CH_3$), 5.07-5.39(m,Ar$CH_2$,3H), 5.47-(d,1H,J=15Hz,CH=$CHCO_2$), 5.83 and 6.02(2s,2H,C=$CH_2$), 5.88(d,1H,J=2Hz,6H), 6.85-(dd,J=15,7Hz,CH=$CHCO_2$), 7.08-7.29(m,aromatic protons of phenyl ring), 7.41-7.54 (m,6H,2 and 6 protons of 4-nitrophenyl rings), 8.09-8.24(m,6H,3 and 5 protons of 4-nitrophenyl rings).

Intermediate 4

[1S-[1α(4R*,5S*),3α,4β,5α,6α(2E,4R*,6R*),7β]] 1-(4-Hydroxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris-(diphenylmethyl) ester

A solution of freeze dried Compound B of Intermediate 1 (2.00g) in dichloromethane (26ml) under nitrogen was treated with a 0.86M solution of diphenyldiazomethane in dichloromethane (13ml) dropwise over 20min. The resulting mixture was left to stir at room temperature for 22h. The mixture was evaporated to a foam. Flash column chromatography on silica gel (Merck 9385, 700ml) eluting with ethyl acetate:cyclohexane (1:4) gave the title compound (2.51g) as a white foam; δ ($CDCl_3$) includes 0.81-0.88-(m,9H,$CH_3$), 1.00(d,3H,J=7Hz,$CH_3$), 4.96(d,1H,J=15Hz, CH=$CHCO_2$), 5.00 and 5.07(2s,2H,C=$CH_2$), 5.33-(s,1H,3H), 5.79(d,1H,J=2Hz,6H), 6.68(dd,1H,J=15,7Hz,CH=$CHCO_2$), 6.79,6.85 and 6.93(3s,3H,$Ph_2CH$), 7.09-7.33(m,aromatic protons).

Intermediate 5

[1S-[1α(5S*),3α,4β,5α,6α(2E,4R*,6R*),7β]] 1-(5-Methyl-3-methylene-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris(diphenylmethyl) ester

A solution of Intermediate 4 (2.41g) in dichloromethane (100ml) was treated with pyridinium chlorochromate (679mg) and the resulting mixture left to stir at room temperature for 18h. The mixture was diluted with diethyl ether (100ml) and filtered through Kieselguhr in vacuo. The filtrate was evaporated to a foam. Flash column chromatography on silica gel (Merck 9385, 700ml) eluting with ethyl acetate:cyclohexane (1:8) gave the title compound (1.34g) as a white foam; δ ($CDCl_3$) includes 0.82-0.92(m,6H,$CH_3$), 0.99-(d,3H,J=7Hz,$CH_3$), 1.08(d,3H,J=7Hz,$CH_3$), 3.48(sextet,1H,J=7Hz, $CH_2CH(CH_3)CO$), 4.93-(d,1H,J=15Hz,CH=$CHCO_2$), 5.34(s,1H,3H), 5.79 and 5.93(2s, 2H,C=$CH_2$), 5.82(d,1H,J=2Hz,6H), 6.67-(dd,1H,J=15,7Hz,CH=$CHCO_2$), 6.81,6.83 and 6.92(3s,3H,$Ph_2CH$), 7.10-7.36(m,aromatic protons).

Intermediate 6

[1S-[1α[(E),5S*],3α,4,5α,6α(2E,4R*,6R*),7β]] 1-(3,5-Dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyl-2-octenoate), 3,4,5-tris(diphenylmethyl) ester

A solution of Intermediate 5 (1.00g) in methanol:water (10:1) (100ml) was treated with rhodium (III) chloride trihydrate (69mg) and the resulting mixture refluxed under nitrogen for 6h then allowed to cool to room temperature over 73h. The mixture was evaporated to a gum. Flash column chromatography on silica gel (Merck 9385, 300ml) eluting with ethyl acetate:cyclohexane (1:4) gave a pale-yellow gum. Further flash column chromatography on silica gel (Merck 9385, 50ml) eluting with ethyl acetate:cyclohexane (1:4) gave the title compound (84mg) as a colourless gum; δ ($CDCl_3$) includes 0.79-0.88(m,6H,$CH_3$), 0.98-(d,6H,J = 7Hz,$CH_3$), 3.49(sextet,1H,J = 7Hz,$CH_2CH(CH_3)CO$), 4.69(d,1H,J = 15Hz,CH = $CHCO_2$), 5.34-(s,1H,3H), 5.82(d,1H,J = 2Hz,6H), 6.69(dd,1H,J = 15,7Hz,CH = $CHCO_2$), 6.82,6.84 and 6.94(3s,3H,$Ph_2CH$), 7.03-7.33(m,aromatic protons).

Intermediate 7

[1S-[1α[(E),5S*],3α,4β,5α,6α(2E,4R*,6R*),7β]] 1-(3,5-Dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)-methyl] ester

A solution of Intermediate 3 (1.15g) in methanol:water (10:1) (88ml) was treated with rhodium (III) chloride trihydrate (115mg) and the resulting mixture refluxed under nitrogen for 6h then allowed to cool to room temperature over 20h. The mixture was evaporated to a gum. Flash column chromatography on silica gel (Merck 9385, 300ml) eluting with ethyl acetate:cyclohexane (1:4 then 1:2) gave the title compound - (659mg) as a cream-coloured foam; δ ($CDCl_3$) includes 0.78-0.87 (m,$CH_3$), 3.44(sextet,1H,J = 7Hz,$CH_2CH$-($CH_3$)CO), 5.08-5.38(m,$ArCH_2OCO$,3H), 5.43(d,1H,J = 15Hz,CH = $CHCO_2$), 5.86(d,1H,J = 2Hz,6H), 7.08-7.28-(m,aromatic protons of phenyl ring), 7.37-7.48 (m,6H,2 and 6 protons of 4-nitrophenyl rings), 8.09-8.19-(m,6H,3 and 5 protons of 4-nitrophenyl rings).

Intermediate 8

[1S-[1α[(E),4R*,5S*],3α,4β,5α,6α(2E,4R*,6R*),7β]] 1-(4-Hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)methyl] ester [Compound 1] and [1S-[1α[(E),4S*,5S*],3α,4β,5α,6α(2E,4R*,6R*),7β]] 1-(4-hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)methyl] ester [Compound 2]

A solution of Intermediate 7 (630mg) in methanol (15ml) was cooled to ~3°C and treated with sodium borohydride (45mg). The resulting mixture was left to stir at room temperature for 1h then treated with 2M aqueous hydrochloric acid (1ml) and the methanol removed in vacuo. The remainder was diluted with further 2M aqueous hydrochloric acid (60ml) and extracted with ethyl acetate (2x20ml). The summed extracts were washed with water (20ml) and brine (20ml), dried over magnesium sulphate and evaporated to a foam. Preparative thin layer chromatography on silica gel (Whatman PK6F, 20x20cm plates, 1000μm layer) using ethyl acetate:cyclohexane (1:1,5 elutions, then 4:3, 2 elutions) as eluent gave title Compound 2 (29mg) as a clear, colourless gum; δ ($CDCl_3$) includes 0.62(d,3H, J = 7Hz,$CH_3$), 0.74-0.90(m,$CH_3$), 0.96-(d,3H,J = 7Hz,$CH_3$), 5.07-5.37(m,$ArCH_2OCO$,3H, 5.43(d,1H,J = 15Hz,CH = $CHCO_2$), 5.57(t,1H,J = 7Hz,CH = C-($CH_3$)), 5.87(d,1H,J = 2Hz,6H), 6.83(dd,1H,J = 15,7Hz,CH = $CHCO_2$), 7.12-7.31(m,aromatic protons of phenyl ring), 7.37-7.56(m,6H,2 and 6 protons of 4-nitrophenyl rings), 8.07-8.25(m,6H,3 and 5 protons of 4-nitrophenyl rings). Title Compound 1 (40mg) was also isolated as a clear, colourless gum; δ ($CDCl_3$) includes 0.72- 0.89(m,9H,$CH_3$), 0.96(d,3H,J = 7Hz,$CH_3$), 5.06-5.38(m,$ArCH_2OCO$,3H), 5.43(d,1H, J = 15Hz,CH = $CHCO_2$), 5.67(t,1H,J = 7Hz,CH = C($CH_3$)), 5.88(d,1H,J = 2Hz,6H), 6.80(dd, 1H,J = 15,7Hz CH = $CHCO_2$), 7.09-7.29(m,aromatic protons of phenyl ring), 7.37- 7.56(m,6H,2 and 6 protons of 4-nitrophenyl rings), 8.07-8.25(m,6H,3 and 5 protons of 4-nitrophenyl rings).

Intermediate 9

[1S-[1α(3R*S*,5S*),3α,4β,5α,6α(2E,4R*,6R*),7β]] 1-(3,5-Dimethyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)-

methyl] ester

A solution of Intermediate 2 (675mg) in methanol (180ml) and water (30ml) was treated with Wilkinsons catalyst (68mg) and refluxed under nitrogen for 6 hours. The solvent was evaporated and the residue, together with some crude material prepared similarly (59mg) was chromatographed on silica (Merck 7734, 72g) eluting with chloroform : acetone 10:1. The required fractions were combined and evaporated to give the title compound (504mg) as a 4:3 ratio of epimers; δ($CDCl_3$) includes 0.8-0.9 (m,$CH_3$), 1.05 (m,$CH_3$) 2.95 (m,$CH_3CHCO$), 3.21 and 3.26 (2d,J3Hz,OH), 3.82 and 3.88 (2s,4OH, 3.98 and 4.08 (2t,J2Hz,7H), 5.08-5.4 (3H,aryl $CH_2$), 5.48 (2d,J16Hz,OCOCH = CH), 5.82 (2d,6H), 6.85 (2dd,J9 and 16Hz,OCOCH = CH), 7.1-7.3 ($C_6H_5$),7.45(2 and 6 protons of 4-nitrophenyl rings), 8.15 (3 and 5 protons of 4-nitrophenyl rings).

Intermediate 10

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris(1,1-dimethylethyl) ester

Freeze-dried Compound A of Intermediate 1 (7.5g) in dry dichloromethane (32ml) were heated at reflux, under nitrogen and treated dropwise over 20 min with N,N-dimethylformamide di-tert.butyl acetal (31.3ml). The mixture was heated under reflux for 1h when a further addition of N,N-dimethylformamide di-tert.butyl acetal (7.21ml) was made over 3 min. The mixture was heated under reflux for a further 4h and was then allowed to cool to room temperature, diluted with diethyl ether (200ml) and washed with brine (3x100ml). The organic phase was dried ($MgSO_4$) and evaporated to give foam. This was subjected to flash chromatography on silica gel (Merck 9385, 1100ml) eluting with ethyl acetate:cyclohexane (1:6). Fractions which contained the major component were combined and evaporated to give the title compound (6.55g) as a cream-yellow foam; δ ($CDCl_3$) includes 1.43(s,$Me_3C$-), 1.48(s,$Me_3C$-), 1.60(s,$Me_3C$-), 2.08(s,$CH_3CO_2$-), 2.93(d,J-3Hz,7-OH), 4.00(broad s,7-H), 4.08(s,4-OH), 4.95(bs,C = $CH_2$), 5.05(s,3-H), 5.11(d,J-5Hz,$CH_3CO_2CH$), 5.77(d,J-15Hz,CH = CH.CHMe), 6.01(d,J-2Hz,6-H), 6.91(dd,J-15Hz,7Hz,CH = CH.CHMe) and 7.10-7.30(m,aromatic protons).

Intermediate 11

[1S-[1α(5S*),3α,4β,5α,6α(2E,4R*,6R*),7β]] 1-(5-Methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), tris(1,1-dimethylethyl) ester

Intermediate 10 (336mg) in dioxan (5ml) was treated with ammonium formate (49mg) and bis-(triphenylphosphine) palladium (II) chloride (27mg). The mixture was stirred and heated under reflux, under nitrogen for 4 hours. Further quantities of ammonium formate (52mg) and bis(triphenylphosphine) palladium (II) chloride (27mg) were then added and the mixture heated under reflux for a further 18 hours. The mixture was allowed to cool and was added to water (100ml) and partitioned with ethyl acetate (2x80ml). The organic extracts were combined, dried ($MgSO_4$) and evaporated under reduced pressure to give a gum. This was subjected to flash silica gel (Merck 9385, 200ml) chromatography, eluting with cyclohexane:ethyl acetate (4:1). Fractions which contained the major component were combined and evaporated to give the title compound (237mg) as a pale yellow foam; δ ($CDCl_3$) includes 0.75-0.83(m,3-$CH_3$ groups), 1.03-(d,J = 7.5Hz,CH = CH.$CHCH_3$), 1.46(s,$Me_3C$-), 1.48(s,$Me_3C$-), 1.59(s,$Me_3C$-), 2.90(d,J = 2.5Hz,7-OH),4.03(m,7-H), 4.07(s,4-OH), 4.74 and 4.81(2bs,C = $CH_2$), 5.30(s,3-H), 5.76(d,J = 16Hz,CH = CH.CHMe), 6.90-(dd,J = 16Hz,8Hz,CH = CH.CHMe), 7.08-7.30(m,aromatic protons).

Intermediate 12

1S-[1α(3R*S*,5S*),3α,4β,5α,6α(2E,4R*,6R*),7β]] 1-(3-Ethyl-5-methyl-4-oxo-6-phenylhexyl)-4,6,7-trihydroxy-2,8-dioxabicyclo[3.2.1]octane-3,4,5-tricarboxylic acid 6-(4,6-dimethyl-2-octenoate), 3,4,5-tris(diphenylmethyl) ester

Intermediate 5 (250mg) in dry tetrahydrofuran (3ml) was added dropwise to a stirred solution of lithium dimethylcuprate prepared from copper (I) iodide (100mg) and methyllithium (1.4M, 0.74ml) in dry tetrahydrofuran (5ml) at -10°C under nitrogen in dry tetrahydrofuran, under nitrogen at such a rate that the temperature did not exceed -10°C. When the addition was complete the resulting yellow suspension was

stirred at -10°C for 30 min when a saturated aqueous solution of ammonium chloride (20ml, adjusted to pH8 by the addition of 0.880 ammonia solution) was added and the resulting mixture was stirred at 0°C for 20 min. The mixture was extracted with ether (2x50ml) and the organic extracts were combined, dried ($MgSO_4$) and evaporated to give a foam. The major component was isolated by preparative layer chromatography (Whatman PF6 20x20cm plates) developing three times with cyclohexane:ethyl acetate (3:1). The major component was eluted with ethyl acetate to give, after filtration and evaporation of the solvent, the title compound (140mg) as a colourless foam; δ ($CDCl_3$) includes 0.66(t,J=7.5Hz,C(=0).CH.$CH_2CH_3$), 0.98-(d,J=7Hz,CH=CH.CH$CH_3$), 1.02(d,J=7Hz,C(=0).CH$CH_3$), 2.80-3.04(m,CH(Et)C(=O)CH($CH_3$)), 3.19-(d,J=2.5Hz,7-OH), 3.88(s,4-OH), 3.96(m,7-H), 4.96(d,J=15Hz,CH=CH.CHMe), 5.29(s,3-H), 5.77-(d,J=2.5Hz,6-H), 6.66(dd,J=15Hz,8.75Hz,CH=CH.CHMe), 6.80(s,$Ph_2$CH), 6.82(s,$Ph_2$CH), 6.91(s,$Ph_2$CH), 7.05-7.33(m,aromatic protons).

Intermediate 13

[1S-[1α[(Z/E),5S*],3α,4β,5α,6α(4S*,6R*),7β]] 1-(3,5-Dimethyl-6-phenyl-3-hexenyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of freeze dried Compound B of Intermediate 1 (10.0g) in ethanol (1000ml) was hydrogenated for 70mins using 5% palladium on barium sulphate catalyst (800mg). The catalyst was removed by filtration through Kieselguhr and the bed washed with more ethanol. The combined filtrates were evaporated to dryness to give a mixture. This mixture was combined with further material prepared as above and the combined mixture (18.61g) in dry N,N-dimethylformamide (220ml) was treated with triethylamine (11.58g) and 4-nitrobenzyl bromide (24.71g) and stirred at room temperature for 22h. The reaction mixture was then diluted with ethyl acetate (2000ml) and extracted with 2N hydrochloric acid (3x400ml). The organic layer was dried and evaporated and the residue purified by silica gel chromatography (Merck 9385) eluting with cyclohexane : ethyl acetate mixtures. Appropriate fractions were combined and evaporated, and the residue purified by silica gel chromatography (Merck 9385) eluting with cyclohexane/ethyl acetate mixtures. Appropriate fractions were combined to give a sample of the title compound (18.79g) and a portion was further purified by silica gel chromatography (Merck 9385) eluting with cyclohexane : ethyl acetate mixtures to give the title compound as a foam; δ ($CDCl_3$) includes 1.66-1.55 (2s,-$CH_2$-$CH_3$C= of both E/Z isomers) 3.90 (t,J=2Hz,7H), 4.82 and 4.98 (2d,J=10Hz,-MeC=CH-CHMe-) 5.82 (d,J=2Hz, 6H), 7.04-7.30 (m,aromatic protons phenylhexenyl sidechain) 7.4-8.25 (m, aromatic protons p-nitro benzyl); approximate molecular weight 1040.1; +FAB mass spectrometry [M + $NH_4$]+ 1057.

Intermediate 14

[1S-[1α(3R*S*,4S*,5S*),3α,4β,5α,6α(4S*,6R*),7β]] 1-(4-Hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)-methyl] ester

A solution of Example 13 (1g) in dimethylformamide (15ml) was treated with p-nitrobenzyl bromide (1.3g) and triethylamine (0.84ml) and the mixture was stirred at 20°C for 18h. The reaction mixture was diluted with ethyl acetate and poured onto hydrochloric acid (2M; 100ml). The organic phase was washed with 2MHCl, $NaHCO_3$, $H_2O$, dried ($MgSO_4$) and chromatographed on silica gel (Merck 7734, 60g) eluting with ethyl acetate:cyclohexane (1:1) to give the title compound (743mg); δ($CDCl_3$) includes 0.7-0.9(m,$CH_3$), 4.03(broad singlet,7H), 5.1-5.4(m,Ar$CH_2$O,and 3H), 5.8(broad singlet,6H), 7.1-7.3(aromatic protons of phenyl ring), 7.4-7.52(2 and 6 protons of 4-nitrophenyl rings), 8.1-8.24(3 and 5 protons of 4-nitrophenyl rings).

Intermediate 15

[1S[1α(3R*S*,5S*),3α,4β,5α,6α(4S*,6R*),7β]] 1-(3,5-Dimethyl-4-oxo-6-phenylhexyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of Intermediate 14 (1.15g) in dichloromethane (40ml) was stirred with pyridinium chlorochromate (400mg) for 3h at 20°C. The solvent was removed under reduced pressure and the residue was partitioned between ethyl acetate and 2M hydrochloric acid. The organic solution was washed with 2MHCl,

brine, dried and chromatographed on silica gel (Merck 7734, 35g) eluting with ethyl acetatecyclohexane (1:1) to give the title compound (902mg); δ(DMSO-$d_6$) includes 0.8-0.9(m,$CH_3$), 1.03-1.12(m,$CH_3CHCO$), 4.02 and 4.07(2d,J5Hz,7H), 5.1-5.45(m,3H,Ar$CH_2$O), 6.30(d,J5Hz,6H), 6.41 and 6.43(2s,7OH), 6.81(s,4OH), 7.2-7.4(aromatic protons of phenyl ring), 7.55 and 7.64(2d,J8Hz, 2 and 6 protons of 4-nitrophenyl rings), 8.14, 8.19 and 8.24(3d,J8Hz,3 and 5 protons of 4-nitrophenyl rings).

Intermediate 16

[1S-[1α(4R*,5S*),3α,4β,5α,6α(4S*,6R*),7β]] 1-(4-Hydroxy-5-methyl-3-methylene-6-phenylhexyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of freeze dried Compound B of Intermediate 1 (10.0g) in ethanol (1000ml) was hydrogenated for 70mins using 5% palladium on barium sulphate catalyst (800mg). The catalyst was removed by filtration through Kieselguhr and the bed washed with more ethanol. The combined filtrates were evaporated to dryness to give a mixture. This mixture was combined with further material prepared as above and the combined mixture (18.61g) in dry N,N-dimethylformamide (220ml) was treated with triethylamine (11.58g) and 4-nitrobenzyl bromide (24.71g) and stirred at room temperature for 22h. The reaction mixture was then diluted with ethyl acetate (2000ml) and extracted with 2N hydrochloric acid (3 x 400ml). The organic layer was dried and evaporated and the residue purified by silica gel chromatography (Merck 9385) eluting with cyclohexane : ethyl acetate mixtures. Appropriate fractions were combined and evaporated to give a sample of the title compound (18.79g); δ ($CDCl_3$) includes 3.97-4.07 (m,4-OH,7H and CH(OH)$CHCH_3$), 5.12 (s,C=$CH_2$), 5.18-5.31 (m,Ar$CH_2$), 5.35 (s,3H), 5.81 (d,J=2Hz,6H), 7.1-7.3, 7.37-7.53 and 8.09-8.25 (3m,aromatic protons).

Intermediate 17

[1S-[1α(4R*,5S*),3α,4β,5α,6α(4S*,6R*),7β]] 1-(4-Hydroxy-5-methyl-3-oxo-6-phenylhexyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of Intermediate 16 (165mg) in dichloromethane (60ml) was ozonolysed at -70°C for 15min. The ozonide was reduced with triphenylphosphine (70mg) and the mixture was allowed to warm to 20°C. The solution was concentrated and chromatographed on silica gel (Merck 7734, 25g) eluting with ethyl acetate-cyclohexane (1:1) to give the title compound (127mg); δ($CDCl_3$) includes 0.7-0.88(m,$CH_3$), 4.02-(broad triplet,7H), 4.15(dd,J2.5 and 4Hz,CH(OH)CO), 5.15-5.30(m,3H,Ar$CH_2$O), 5.79(d,J2Hz,6H), 7.19-7.28-(aromatic protons of phenyl ring), 7.43,7.44 and 7.49(3d,J8Hz,2 and 6 protons of 4-nitrophenyl rings), 8.14,8.17 and 8.20(3d,J8Hz,3 and 5 protons of 4-nitrophenyl rings).

Intermediate 18

[1S-[1α(3R*S*,4R*,5S*),3α,4β,5α,6α(4S*,6R*),7β]] 1-(3,4-Dihydroxy-5-methyl-6-phenylhexyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)-methyl] ester

A solution of Intermediate 17 (492mg) in methanol (9ml) was cooled in an ice-bath and then treated with sodium borohydride (35mg). After 0.5h the reaction was quenched by addition of 2M hydrochloric acid. The methanol was removed under reduced pressure and the residue was partitioned between 2M hydrochloric acid and ethyl acetate. The organic solution was washed with $NaHCO_3$ solution, brine, dried ($MgSO_4$) and chromatographed on silica gel (Merck 7734, 25g) eluting with ethyl acetate-cyclohexane (1:1→3:1) to give the title compound (300mg); δ($CDCl_3$) includes 0.75-0.9(m,$CH_3$), 4.03 and 4.05(2 broad singlets, 7H), 5.1-5.3(m,Ar$CH_2$O), 5.35 and 5.39(2s,3H), 5.85(broad singlet,6H), 7.1-7.3(aromatic protons of phenyl ring), 7.38-7.5(2 and 6 protons of 4-nitrophenyl rings), 8.08-8.24(3 and 5 protons of 4-nitrophenyl rings).

Intermediate 19

[1S-[1α(2E,5S*),3α,4β,5α,6α(4S*,6R*),7β]] 1-(3,5-Dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl]

ester

Pyridinium chlorochromate (1.7g) was added to a solution of Intermediate 16 (4.9g) in dichloromethane (50ml) and the mixture was stirred at 20°C for 4h. The solvent was removed under reduced pressure, and the residue was partitioned between ethyl acetate and water. The organic phase was washed with $H_2O$, 2M HCl, brine, dried and chromatographed on silica gel (Merck 7734; 150g) eluting with ethyl acetate : cyclohexane (1:1) to give a foam which was dissolved in methanol (200ml). Rhodium trichloride trihydrate (200mg) in water (25ml) was added to the methanol solution, and the mixture was heated to reflux for 7h under nitrogen. The mixture was concentrated under reduced pressure and then diluted with ethyl acetate. The organic phase was washed with $H_2O$, 2M HCl, brine, dried and chromatographed on silica gel (Merck 7734; 100g) eluting with ethyl acetate: cyclohexane (1:1) to give the title compound (2.4g); δ($CDCl_3$) includes 0.79-0.87(m,$CH_3$), 1.80 (s,$CH_3C=C$), 5.38 (s,3-H), 5.81 (d,J=2Hz,6-H), 6.83 (t,J=7Hz,CH=C), 7.1-7.3 (aromatic protons of phenyl ring), 7.36-7.46(2 and 6 protons of 4-nitrophenyl rings), 8.1-8.2 (3 and 5 protons of 4-nitrophenyl rings).

Intermediate 20

[1S-[1α,3α,4β,5α,6α(4S*,6R*),7β]] 1-(2-Oxoethyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of Intermediate 19 (2.38g) in dichloromethane (130ml) was cooled to -70°C and ozonolysed for 1h. The ozonide was reduced with triphenylphosphine (888mg) and the mixture was allowed to warm to 20°C. The solution was concentrated and chromatographed on silica gel (Merck 7734; 100g) eluting with ethyl acetate : dichloromethane (1:9) to give the title compound (834mg); δ($CDCl_3$) includes 0.75-0.86 (m,$CH_3$), 2.10 (t,J=8Hz,$CH_2CO_2$), 3.04 and 3.43 each (d,J=18Hz,$CH_2CHO$), 3.94 (s,4-OH), 4.09 (d,J=2Hz,7-H),4.20 (t,J=2Hz,7-H), 5.49 (s,3-H), 6.10 (d,J=2Hz,6-H), 7.42, 7.48, 7.49 each (d,J=8Hz, 2 and 6 protons of 4-nitrophenyl rings), 8.16, 8.19, 8.21 each (d,J=8Hz, 3 and 5 protons of 4-nitrophenyl rings), 9.81 (s,CHO); analysis found: C,56.34;H,5.08;N,4.41; $C_{42}H_{45}N_3O_{19}$ requires C,56.31;H,5.06;N,4.69 %

Intermediate 21

[1S-[1α(2E),3α,4β,5α,6α(4S*,6R*),7β]] 1-(4-Oxo-6-phenyl-2-hexenyl)-4,6,7-trihydroxy-2,8-dioxabicyclo[3.2.1]-octane-3,4,5-tricarboxylic acid, 6-(4,6-dimethyloctanoate), 3,4,5-tris[(4-nitrophenyl)methyl] ester

Dimethyl 2-oxo-4-phenylbutylphosphonate (92mg) in tetrahydrofuran (3ml) was added to a suspension of sodium hydride (60% oil dispersion; 15mg) in tetrahydrofuran (1ml) under nitrogen. When the evolution of hydrogen subsided a solution of Intermediate 20 (250mg) in tetrahydrofuran (5ml) was added and the solution was stirred at 20°C for 1h. 2M hydrochloric acid was added and the mixture was extracted with ethyl acetate. The organic phase was washed with $NaHCO_3$ 2M HCl, brine, dried, and chromatographed on silica gel (Merck 7734; 15g) eluting with ethyl acetate : dichloromethane (1:9 increasing to 1:3) to give the title compound (218mg); δ($CDCl_3$) includes 0.77-0.88 (m,$CH_3$), 2.12 (t,J=7Hz,$CH_2CO_2$), 3.84 (s,4-OH), 4.00 (br.s,7-H), 5.79 (d,J=2Hz,6-H), 6.26 (d,J=16Hz, CH=CHCO), 6.91 (dt,J=16 and 7Hz,CH=CHCO), 7.16-7.3 (aromatic protons of phenyl ring), 7.44 and 7.49 each (d,J=9Hz,2 and 6 protons of nitrophenyl rings), 8.15, 8.17 and 8.20 each (d,J=9Hz,3 and 5 protons of nitrophenyl rings).

Intermediate 22

[1S-[1α(2E,5R*S*),3α,4β,5α,6α(4S*,6R*),7β]] 1-(5-Methyl-4-oxo-6-phenyl-2-hexenyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

Dimethyl 3-methyl-2-oxo-4-phenylbutylphosphonate (110mg) in tetrahydrofuran (3ml) was added to a suspension of sodium hydride (60% oil dispersion; 15mg) in tetrahydrofuran (0.5ml) under nitrogen. When the evolution of hydrogen subsided a solution of Intermediate 20 (250mg) in tetrahydrofuran (10ml) was added and the solution was stirred at 20°C for 1h. Dilute hydrochloric acid was added and the mixture was extracted with ethyl acetate. The organic phase was washed with 2M HCl, $NaHCO_3$, brine, dried and chromatographed on silica gel (Merck 7734, 25g) eluting with ethyl acetate : dichloromethane (1:9) to give the title compound (156mg); δ($CDCl_3$) includes 0.75-0.87 (m,$CH_3$), 1.06 (d,J=6Hz,$COCHCH_3$), 2.13

(t,J = 8Hz,$CH_2CO_2$), 3.87 (s,4-OH), 4.00 (m,7-H), 5.80 (d,J = 2Hz,6-H), 6.29 (d,J = 16Hz,CH = CHCO), 6.92 (dt,J = 16 and 7Hz,CH = CHCO), 7.1-7.27 (aromatic protons of phenyl ring), 7.44 and 7.48 each (d,J = 8Hz,2 and 6 protons of nitrophenyl rings), 8.11-8.21 (3 and 5 protons of nitrophenyl rings).

Intermediate 23

[1S-[1α(2E),3α,4β,5α,6α(4S*,6R*),7β]] 1-(3-Methyl-4-oxo-6-phenyl-2-hexenyl)-4,6,7-trihydroxy-2,8-dioxabicyclo[3.2.1]octane-3,4,5-tricarboxylic acid, 6-(4,6-dimethyloctanoate), tris[(4-nitrophenyl)methyl] ester

A solution of phosphonic acid [(1R,S)1-methyl-2-oxo-4-phenylbutyl]dimethyl ester (97mg) in dry tetrahydrofuran (3ml) was added to a stirred suspension of sodium hydride (60% dispersed in oil, 15mg) in dry tetrahydrofuran (1ml) under nitrogen. After 3 min a solution of Intermediate 20 (250mg) in dry tetrahydrofuran (15ml) was added and the resulting solution was stirred, under nitrogen, at room temperature for 45min. The mixture was poured into 2N hydrochloric acid and partitioned with ethyl acetate (2x100ml). The organic extracts were combined and washed with 2N hydrochloric acid, aqueous saturated sodium hydrogen carbonate solution, brine and then dried ($MgSO_4$). Evaporation of the solvent gave a gum which was chromatographed on silica gel (250g) eluting with dichloromethane ethyl acetate mixtures. Fractions which contained the appropriate component were combined and evaporated to give the title compound (117mg); δ ($CDCl_3$) includes 1.82(s,C = $CH_3$). C( = 0)), 2.10(t,J = 7Hz,OC( = 0)$CH_2CH_2$),3.22-(d,J = 2Hz,7-OH), 3.85(s,4-OH), 4.00(s,fine splitting,7-H), 5.80(d,J<2Hz,6-H), 6.81(t,J = 5Hz,$CH_2$.CH = C($CH_3$)), 7.10-7.54(m,aromatic protons), 8.08-8.25(m,aromatic protons).

Intermediate 24

[1S-[1α(2E),3α,4β,5α,6α(4S*,6R*),7β]] 1-(4-Oxo-7-phenyl-2-heptenyl)-4,6,7-trihydroxy-2,8-dioxabicyclo[3.2.1]-octane-3,4,5-tricarboxylic acid, 6-(4,6-dimethyloctanoate),3,4,5-tris[(4-nitrophenyl)methyl] ester

Dimethyl 2-oxo-5-phenylpentylphosphonate (97mg) in tetrahydrofuran (3ml) was added to a suspension of sodium hydride (60% oil dispersion; 15mg) in tetrahydrofuran (1ml) under nitrogen. When the evolution of hydrogen subsided a solution of Intermediate 20 (250mg) in tetrahydrofuran (5ml) was added and the solution was stirred at 20°C for 0.5h. Dilute hydrochloric acid was added and the mixture was extracted with ethyl acetate. The organic phase was washed with 2M HCl, $NaHCO_3$, brine, dried and chromatographed on silica gel (Merck 7734, 25g) eluting with ethyl acetate : dichloromethane (1:9→1:3) to give the title compound (200mg); δ($CDCl_3$) includes 0.77-0.88 (m,$CH_3$), 2.12 (t,J = 7Hz,$CH_2CO_2$), 3.86 (s,4-OH), 4.01 (br.t,7-H), 5.34 (s,3-H), 5.80 (d,J = 2Hz,6-H), 6.25 (d,J = 16Hz,CH = CHCO), 6.89 (dt,J = 16 and 7Hz,CH = COCH), 7.12-7.3 (aromatic protons of phenyl ring), 7.45,7.46 and 7.50 each (d,J = 8Hz,2 and 6 protons of nitrophenyl rings), 8.15, 8.18 and 8.20 each (d,J = 8Hz,3 and 5 protons of nitrophenyl rings); analysis found: C,60.95;H,5.56;N,3.89; $C_{53}H_{57}N_3O_{19}$ requires C,61.20;H,5.52;N,4.04 %

Intermediate 25

[1S-[1α,3α,4β,5α,6α(4S*,6R*),7β]] 1-(3-Oxopropyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of Intermediate 17 (6.927g) in methanol (128ml) was cooled to 0°C and treated with sodium borohydride (500mg). The mixture was stirred at 0°C for 30min when 2N hydrochloric acid (50ml) was added slowly. The methanol was evaporated and a further addition of 2N hydrochloric acid (100ml) was made. The mixture was partitioned with ethyl acetate (2x200ml) and the organic extracts were combined, washed with saturated aqueous sodium hydrogen carbonate solution, brine and dried ($MgSO_4$). Evaporation of the solvent gave a foam which was chromatographed on silica gel (Merck Kieselgel 60, 300g) eluting with cyclohexane:ethyl acetate (1:1) and ethyl acetate. Fractions which contained the major component were combined and evaporated to give a white foam (2.541g) (hereinafter referred to as Intermediate C).

A portion (2.343g) of this material was dissolved in tetrahydrofuran (60ml) and the solution was stirred and treated with a solution of sodium metaperiodate (1g) in water (28ml). The mixture was stirred at room temperature for 24h when the tetrahydrofuran was evaporated and the cloudy aqueous phase was diluted with water (100ml) and extracted with ethyl acetate (2x150ml). The organic extracts were combined, dried ($MgSO_4$) and evaporated to give a yellow oil. This was chromatographed on silica gel (Merck Keiselgel 60, 300g) eluting with ethyl acetate:cyclohexane (3:1). Fractions which contained a major component of

intermediate polarity were combined and evaporated to give the title compound (336mg) as a white foam; δ ($CDCl_3$) includes 2.13(t,J=7.5Hz,$OCCH_2CH_2$), 2.69-2.96(m,$CH_2CHO$), 3.21(d,J=3Hz,7-OH), 3.81(s,4-OH), 4.05(m,7-H), 5.79(d,J=2.5Hz,6-H)), 7.38-7.58 and 8.10-8.27(2m,aromatic protons), 9.81(s,CHO); analysis found:C,56.59; H,5.29; N,4.67%; $C_{43}H_{47}N_3O_{19}$(909.8) requires C,56.76; H,5.21; N,4.62%.

Fractions which contained the more polar major component were combined and evaporated to give a colourless foam, whose pmr spectrum resembled that of an authentic sample of Intermediate 26 hereinafter.

Intermediate 26

[1S-[1α,3α,4β,5α,6α(4S*,6R*),7β]]1-(3-Oxopropyl)-4,6,7-trihydroxy-2,8-dioxabicyclo[3.2.1]octane-3,4,5-tricarboxylic acid, 6-(4,6-dimethyloctanoate),3-methylester, 4,5-bis[(4-nitrophenyl)methyl] ester

A portion (200mg) of Intermediate C (from Intermediate 25 experiment above) was dissolved in tetrahydrofuran (5ml) and the solution was stirred and a solution of sodium metaperiodate (50mg) in water (1.5ml) was added. The resulting solution was stirred at room temperature for 4h when a further addition of sodium metaperiodate (15mg) in water (0.5ml) was made. The mixture was stirred at room temperature for 24h when a further addition of sodium metaperiodate (20mg) in water (0.2ml) was made. The mixture was stirred at room temperature for 18h and was then poured into water (50ml) and extracted with ethyl acetate (2x100ml). The organic extracts were combined, dried ($MgSO_4$) and evaporated to give a gum. This was chromatographed on silica gel (Merck Kieselgel 60, 100g) eluting with cyclohexane:ethyl acetate (3:2). Fractions which contained the major component were combined and evaporated to give the title compound (99mg); δ ($CDCl_3$) includes 2.15(t,J=7.5Hz,OC(=0)$CH_2CH_2$), 2.68-2.99(m,$CH_2CH_2CHO$), 3.16(bs,7-OH), 3.68(s,$CO_2CH_3$), 3.77(s,7-H), 4.04(s,4-OH), 5.81(d,J<2Hz,6-H), 7.50(d,J=8.75Hz,aromatic protons), 7.54-(d,J=8.75Hz,aromatic protons), 8.19(2d,J=8.75Hz,aromatic protons), 9.83(s,CHO); analysis found: C,57.99; H,5.97; N,3.30%; $C_{37}H_{44}N_2O_{17}$ (788.7) requires C,57.89; H,5.99; N,3.33%.

Intermediate 27

[1S-[1α,3α,4β,5α,6α(4S*,6R*),7β]] 1-(3-Oxobutyl)-4,6,7-trihydroxy-2,8-dioxabicyclo[3.2.1]octane-3,4,5-tricarboxylic acid, 6-(4,6-dimethyloctanoate), 3,4,5-tris[(4-nitrophenyl)methyl] ester

A solution of Intermediate 13 (1.72g) in dichloromethane (300ml) was cooled to -70°C and subjected to ozonolysis for 10 minutes to give a blue colour. Nitrogen was then bubbled in and after a further 14 minutes triphenylphosphine (1.3g) was added and the solution was allowed to reach 21°C. It was then evaporated to an oil which was chromatographed on silica (Merck 7734; 300g) eluting with chloroform: acetone 7:1 then 6:1. The required fractions were combined and evaporated to give the title compound as a white foam (1.39g), containing triphenylphosphine oxide (2mol equiv); ν($CHBr_3$) 1769 and 1744 (ketone and ester C=0), 1523 and 1347$cm^{-1}$ ($NO_2$); δ($CDCl_3$) includes 0.75-0.9 (m,$CH_3$), 2.16 (s,$CH_3CO$), 3.75 (d,J3Hz,7-OH), 3.93 (s,4-OH),4.09 (t,J3Hz,7-H), 5.15-5.36(aryl $CH_2$,3-H), 5.89 (d,J2Hz,6-H), 7.45 (2 and 6 protons of 4-nitrophenyl rings), 8.15 (3 and 5 protons of 4-nitrophenyl rings).

Intermediate 28

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), tris-(diphenylmethyl) ester.

To a stirred solution of the freeze-dried Compound A of Intermediate 1 (10.65g) in dichloromethane (250ml) was added dropwise over 20 minutes a 0.5M solution of diphenyldiazomethane. Addition of diphenyldiazomethane was continued until a pale pink colour remained. The solution was stirred at 20°C for 3hr and evaporated to dryness under reduced pressure to afford a foam.

This was purified (two batches of 12.4g each) by flash chromatography on Merck 9385 Kieselgel (2x250g; petrol : ethyl acetate = 5:1 eluant). The appropriate fractions were combined and evaporated to dryness under reduced pressure to provide the title compound (17.6g) as a colourless foam; δ ($CDCl_3$) includes 0.80-1.05 (m,$CH_3$, 12 protons), 2.06 (s,$OCOCH_3$), 3.25 (d,J2Hz,7OH), 3.92 (s,4OH), 3.95 (t,J2Hz,7H), 4.96 (d,J12Hz,CH=CHCH($CH_3$)), 4.99 (m,=$CH_2$), 5.1 (d, J4Hz, $CHOCH_3$), 5.34 (s,3H), 5.81 (d,J2Hz,6H), 6.68 (d,dJ8Hz,12Hz,CH=CHCH($CH_3$)), 6.81(s), 6.82 (s), 6.87 (s), ($CHPh_2$) and 7.05-7.35 (m,aromatic protons);

Analysis Found: C,74.4;H,6.4;
$C_{74}H_{76}O_{14}$ requires : C,74.7;H,6.4%.

Intermediate 29

[1S-[1α(4R*,5S*),3α,4β,5α,6α,(2E,4R*,6R*),7β]] 1-(4-Acetyloxy-5-methyl-3-methylene-6-phenylhexyl)-4,6-dihydroxy-7-[(2-methoxyethoxy) methoxy]-2,8-dioxabicyclo[3.2.1]octane-3,4,5-tricarboxylic acid, 6-(4,6-dimethyl-2-octenoate), tris(diphenylmethyl) ester

To a cooled ($5^0$C) stirred solution of Intermediate 28 (476mg) in dichloroethane (5ml) was added N,N-diisopropylethylamine (258mg) and 2-methoxyethoxymethyl chloride (250mg) keeping the temperature below $5^0$C by means of an ice/salt bath. The solution was allowed to warm to $20^0$C over 30 minutes and then heated to reflux for 6hr. The solution was cooled ($20^0$C) and further N,N-diisopropylethylamine (129mg) and 2-methoxyethoxymethyl chloride (125mg) added. The resulting solution was heated to reflux for 16hr. The cooled solution was diluted with ethyl acetate (200ml), and washed sequentially with 2N hydrochloric acid (2x30ml), water (30ml), saturated sodium bicarbonate (2x30ml), water (30ml) and brine (30ml), dried ($MgSO_4$) and evaporated to dryness, under reduced pressure, to leave a gum. This was purified by flash chromatography on Merck '9385' Kieselgel (64g; petrol : ethyl acetate = 5:1 eluant). The appropriate fractions were combined and evaporated to dryness, under reduced pressure, to furnish a solid (441mg).

A sample (380mg) of the solid was further purified by flash chromatography on Merck '9385' Kieselgel (36g; dichloromethane : ethyl acetate = 45:1 eluant). The appropriate fractions of the more polar product were combined and evaporated to dryness, under reduced pressure to afford the title compound (284mg) as a colourless gum; δ ($CDCl_3$) includes 0.79-0.97 (m,$CH_3$,12 protons), 2.08 (s,$OCOCH_3$), 3.3 (s,$OCH_2CH_2OCH_3$), 3.43 (m,$OCH_2CH_2OCH_3$), 3.67 (m,$OCH_2CH_2OCH_3$), 3.89 (s,4OH), 4.06 (broad s,7H), 4.77 (d,J6Hz,$OCH_2OCH_2CH_2$), 4.84 (d,J12Hz,CH=CHCH($CH_3$)), 4.96 (d,J6Hz,$OCH_2OCH_2CH_2$), 5.0 (broad s,=$CH_2$), 5.15 (d,J4Hz,CHOAc), 5.34(s,3H), 6.33 (broad s,6H), 6.65 (d,dJ7Hz,J12Hz,CH=CHCH($CH_3$)), 6.65 (s), 6.83 (s), 6.85 (s) ($CHPh_2$), 7.05-7.32 (m,aromatic protons);
Analysis Found: C,72.4; H,6.6%;
$C_{78}H_{84}O_{16}.0.25CH_2Cl_2$(1298.8) requires : C,72.4;H,6.6%.

Intermediate 30

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, tris (diphenylmethyl) ester

A solution of Intermediate 29 (4.77g) in N,N-dimethylformamide (30ml) was stirred at 20°C, and N-methylhydroxylamine hydrochloride (623mg) and triethylamine (1.57ml) were added. The resulting suspension was stirred for 18 hours, and then partitioned between ethyl acetate (300ml) and 2N-hydrochloric acid (100ml). The organic phase was washed with 2N-hydrochloric acid (100ml), water (3x100ml), and brine (100ml), and dried (magnesium sulphate), and evaporated to dryness. The residue was purified by flash chromatography on Merck '9385' Kieselgel (250g, petrol : ethyl acetate = 2:1 eluant) to give the title compound (3.76g) as a colourless foam; δ($CDCl_3$) includes 0.81 (d,J7Hz $CH_3$), 2.05 (s,$OCOCH_3$), 3.15-3.35 (m), 3.40-3.55 (m), ($OCH_2CH_2O$), 3.24 (s,$OCH_3$), 3.86 (s,4OH), 3.88 (broad s,7H), 4.05 (d,J3Hz,6OH),4.68 and 4.74 (ABq,J8Hz,$OCH_2O$), 5.00 (broad s,=$CH_2$), 5.10-5.18 (m,6H,CHOAc), 5.13 (s,3H, 6.58 (s), 6.80 (s), 6.87 (s), ($CHPh_2$), 7.0-7.3 (m,aromatic protons);
Analysis Found: C,72.7;H,6.1;
$C_{68}H_{68}O_{15}$ (1125.2) requires C,72.6;H,6.1%.

Intermediate 31

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris(1,1-dimethylethyl) ester

Freeze-dried Compound A of Intermediate 1 (7.5g) in dry dichloromethane (32ml) was heated at reflux, under nitrogen and treated dropwise over 20 min with N,N-dimethylformamide di-tert.butyl acetal (31.3 ml). The mixture was heated under reflux for 1h when a further addition of N,N-dimethylformamide di-tert.butyl acetal (7.21ml) was made over 3 min. The mixture was heated under reflux for a further 4h and was then

allowed to cool to room temperature, diluted with diethyl ether (200ml) and washed with brine (3x100ml). The organic phase was dried ($MgSO_4$) and evaporated to give a red-brown foam (10.75g). This was subjected to flash chromatography on silica gel (Merck 9385, 1100ml) eluting with ethyl acetate:cyclohexane (1:6). Fractions which contained the major component were combined and evaporated to give the title compound (6.55g) as a cream-yellow foam; $\nu_{max}$ ($CHBr_3$) ca 3400-3600 (OH), 1755 (ester C=0), 1730 (ester C=0) and 1250cm$^{-1}$ (ester C=0); $\delta$ ($CDCl_3$) includes 1.43(s,$Me_3$C-), 1.48(s,$Me_3$C-), 1.60-(s,$Me_3$C-), 2.08(s,$CH_3CO_2$-), 2.93(d,J=3Hz,7-OH), 4.00(broad s,7-H), 4.08(s,4-OH), 4.95(bs,C=$CH_2$), 5.05-(s,3-H), 5.11(d,J=5Hz,$CH_3CO_2$CH), 5.77 (d,J=15Hz,CH=CH.CHMe), 6.01(d,J=2Hz,6-H), 6.91-(dd,J=15Hz,7Hz,CH=CH.CHMe) and 7.10-7.30(m,aromatic protons).

Intermediate 32

[1S-[1α(4R*,5S*),3α,4β,5α,6α(2E,4R*,6R*),7β]]7-Acetyloxy-1-(4-acetyloxy-5-methyl-3-methylene-6-phenylhexyl)-4,6-dihydroxy-2,8-dioxabicyclo[3.2.1]octane-3,4,5-tricarboxylic acid, 6-(4,6-dimethyl-2-octenoate), 3,4,5-tris(1,1-dimethylethyl) ester

A solution of Intermediate 31 (0.38g) in dry dichloromethane (3ml) was treated with triethylamine (0.61ml) and acetic anhydride (0.083ml) with stirring under nitrogen and the mixture stirred for 24h at room temperature. An additional portion of acetic anhydride (0.20ml) was added, and stirring continued for another 18h at room temperature and 2h at reflux. A further portion of acetic anhydride (0.15ml) was added and after a further 1h at reflux the mixture was allowed to cool. The mixture was diluted with dichloromethane (50ml) and the resultant solution washed three times with saturated aqueous sodium bicarbonate solution (20ml), dried and evaporated to give a gum. This material was chromatographed using a silica gel column eluting with ethyl acetate-cyclohexane (1:5) and appropriate fractions were combined and evaporated to give the title compound (0.39g) as a pale yellow gum; $\delta$ ($CDCl_3$) values include 1.43, 1.48 and 1.66(3s,3xC($CH_3$)$_3$), 2.10 and 2.14(2s,$CH_3CO_2$), 4.11(s,OH), 4.92-5.02(m,C=$CH_2$), 4.96(s,($CH_3$)$_3$$O_2$CCH), 5.10-5.18(m,$CH_3CO_2$CH), 5.78(d,J16Hz,$O_2$CCH=CH), 6.45(d,J2Hz,CHO$_2$CCH=CH), 6.92(dd,J16 and 8Hz,$O_2$CCH=CH).

Intermediate 33

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 3,4,5-tris[(4-nitrophenyl)methyl] ester

A solution of the freeze dried Compound A of Intermediate 1 (1g), p-nitrobenzyl bromide (1.26ml) and triethylamine (0.63ml) in dimethylformamide (10ml) was stirred at 21°C for 23h and then partitioned between ethyl acetate (100ml) and 2M-hydrochloric and (100ml). The aqueous phase was extracted with ethyl acetate (50ml). Combined organic extracts were wasted with 2M-hydrochloric acid (100ml), water and brine (2x100ml each), dried ($MgSO_4$), and evaporated to a gum. This was chromatographed on silica (Merck 7734, 200g) eluting with chloroform:acetone 15:1. The required fractions were combined and evaporated to give the title compound as a white foam (758mg), $\delta$ ($CDCl_3$) includes 0.8-0.9(m,$CH_3$), 1.0-(d,J6Hz,$CH_3$CH=CH), 2.08(s,OCOCH$_3$), 3.25(d,J2Hz,7OH), 3.90(s,4OH), 4.05(t,J2Hz,7H), 4.99-(d,J7Hz,C=$CH_2$), 5.05-4.9(CHOAc, arylCH$_2$,3H), 5.49(d,J16Hz,OCOCH=CH), 5.85(d,J2Hz,6H), 6.85(dd,J9 and 16Hz,OCOCH=CH), 7.05-7.3($C_6H_5$), 7.45(2 and 6 protons of 4-nitrophenyl rings), 8.15(3 and 5 protons of 4-nitrophenyl rings).

Intermediate 34

[1S-[1α(4R*,5S*),3α,4β,5α,6α(2E,4R*,6R*),7β]] 1-(4-Acetyloxy-5-methyl-3-methylene-6-phenylhexyl)-4,7-diacetyloxy-6-hydroxy-2,8-dioxabicyclo[3.2.1]octane-3,4,5-tricarboxylic acid, 6-(4,6-dimethyl-2-octenoate), 3,4,5-tris[(4-nitrophenyl)methyl] ester

A solution of Intermediate 33 (180mg) in dichloromethane (1ml), acetic anhydride (1ml) and triethylamine (2ml) was treated with 4-dimethylaminopyridine (53mg) and kept at 21°C for 2.5 days, then it was partitioned between ethyl acetate (50ml) and 2M-hydrochloric acid (50ml). The organic phase was washed with further acid (50ml). Combined acidic washes were extracted with ethyl acetate (50ml). Combined organic extracts were washed with saturated sodium bicarbonate solution and brine (2x50ml

each), dried ($MgSO_4$) evaporated to an oil. This was chromatographed on silica (Merck 7734, 26g) eluting with cyclohexane: ethyl acetate 2:1. The required fractions were combined and evaporated to give the title compound as a yellow foam (174mg); δ($CDCl_3$) includes 0.75-0.9 (m,$CH_3$), 0.95 (d,J6Hz,$CH_3CH=CH$), 1.95 and 2.15 (s,4 and 7- $OCOCH_3$), 2.10 (s,$OCOCH_3$), 4.9-5.3 (m,CHOAc,aryl $CH_2$,7H,3H,C=$CH_2$), 5.55 (d,J16Hz,OCOCH=CH), 6.50 (d,J2Hz,6H), 6.85 (dd,J9 and 16Hz,OCOCH=CH), 7.1-7.5 ($C_6H_5$ and 2 and 6 protons of 4-nitrophenyl rings), 8.1-8.25 (3 and 5 protons of 4-nitrophenyl rings).

Intermediate 35

1S-[1α(4R*,5S*),3α,4β,5α,6α(2E,4R*,6R*),7β]] 1-(4-Acetyloxy-5-methyl-3-methylene-6-phenylhexyl)-4,6-dihydroxy-7-[(methoxycarbonyl)oxy]2,8-dioxabicyclo[3.2.1]octane-3,4,5-tricarboxylic acid, 6-(4,6-dimethyl-2-octenoate), tris (diphenylmethyl) ester.

A solution of Intermediate 28 (892 mg) and 4-dimethylaminopyridine (183.3 mg) in dichloromethane (1.5 ml) was cooled to 0°C under nitrogen. To this cold solution was added methyl chloroformate (64 μl) with vigorous stirring. The resulting solution was allowed to warm to room temperature and was subsequently stirred for 2 hours. The solvent was removed from the reaction mixture under reduced pressure leaving a white residue which was separated between ethyl acetate (100 ml) and water (50 ml). The organic phase was washed with water (2 x 25 ml) and saturated brine (30 ml) and dried over $MgSO_4$. The solvent was then removed under reduced pressure to yield a foam. This was purified by flash column chromatography on Merck Kieselgel 60 (80 g) with 2% ethyl acetate/dichloromethane as eluent. Appropriate fractions were combined and the solvent was removed under reduced pressure to yield the title compound as a white foam, (990.4 mg); δ ($CDCl_3$) includes 0.76-0.89 (m, $CH_3$, 9 protons). 0.93 (d,J=7 Hz, $CH_3$), 2.07 (s, $OCOCH_3$), 3.83 (s, $OCO_2CH_3$), 3.89 (s, 4 OH), 4.86 (d, J=16 Hz, OCOCH=CH), 5.01 (s, broad =$CH_2$), 5.08 (d, J=2 Hz, 7H), 5.14(d, J=5 Hz, CHOAc), 5.26 (s, 3H), 6.33 (d, J=2 Hz, 6H), 6.64 (dd, J=8.5 Hz, 16 Hz, CH=CHCH(Me)), 6.74 (s, $CHPh_2$), 6.84 (s, $CHPh_2$), 7.05-7.35 (m, aromatic protons).
Analysis Found: C, 72.93%; H,6.26%, $C_{76}H_{78}O_{16}$(1247.46) requires C, 73.18%; H, 6.30%.

Intermediate 36

[1S-[1α(4R*,5S*),3α,4β,5α,6α,7β]] 1-(4-Acetyloxy-5-methyl-3-methylene-6-phenylhexyl)-4,6-dihydroxy-7-[-(methoxycarbonyl)oxy]-2,8-dioxabicyclo[3.2.1]octane-3,4,5-tricarboxylic acid, tris (diphenylmethyl) ester.

To a stirred solution of Intermediate 35 (400 mg) and N-methyl hydroxylamine hydrochloride (53.6 mg) in N,N-dimethylformamide (1.5 ml) was added triethylamine (134 μl) with stirring. The resulting reaction mixture was then stirred under nitrogen at room temperature for 21 hours. The reaction mixture was separated between ethyl acetate (50 ml) and 1N hydrochloric acid (25 ml) and the organic phase washed with water (2 x 25 ml) and saturated brine (25 ml). The solution was then dried over $MgSO_4$ and the solvent removed under reduced pressure to yield a solid. This was then purified by flash column chromatography on Merck Kieselgel 60 (20 g) with elution by 25% ethyl acetate/petroleum ether. Appropriate fractions were combined and the solvent removed under reduced pressure to yield the title compound as a white foam (329.8 mg); δ ($CDCl_3$) includes 0.83 (d, J=6.5 Hz, -$CH_3$), 2.04 (s, $OCOCH_3$), 3.74 (s, 4-0H), 3.80 (s, $OCO_2CH_3$), 4.8 (d, J=2Hz, 7H), 4.99 (s, broad, =$CH_2$), 5.02(s, 3H), 5.10 (d, J=4.5 Hz, CHOAc), 5.23 (dd, J=2 Hz, 4.5 Hz, 6H), 6.61 (s, $CHPh_2$), 6.82 (s, $CHPh_2$), 6.86 (s, $CHPh_2$), 7.03-7.31 (m, aromatic protons);
Analysis Found: C, 71.67%; H, 5.38%;
$C_{66}H_{62}O_{15}$.0.5 EtOAc requires C, 71.69%; H,5.84%.

Intermediate 37

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), tris[(2-methoxyethoxy)ethyl] ester

To a stirred solution of the freeze dried Compound A of Intermediate 1 (2.00g) in dichloromethane (20ml) at 0°C, diisopropyethylamine (2.44ml) followed by 2-methoxyethoxymethyl chloride (1.60ml) was added. The reaction was stirred at 20°C for 2h. Water (50ml) and ethyl acetate (50ml) were added, and after separation, the aqueous was re-extracted with ethyl acetate (2x50ml). Washing of the combined organics with brine (100ml) and drying followed by evaporation of the solvent gave an oil which was subjected to

flash chromatography (4% methanol in chloroform) to give the title compound (2.79g) as a pale yellow oil; δ-($CD_3OD$) includes 0.80-0.90 (9H,m,3x$CH_3$), 1.03 (3H,d,J = 7Hz,$CH_3$), 4.05(1H,d,J = 2Hz,7-H), 4.22 (1H,s,4-OH), 4.98 (2H,d, = $CH_2$), 5.09 (1H,d,J = 5Hz,CHOAc), 5.30 (1H,s,3H), 5.38 (2H,d,J = 11Hz,$OCH_2O$), 5.43 (2H,s,$OCH_2O$), 5.54 (2H,s,$OCH_2O$), 5.77 (1H,d,J = 16Hz,CH = $CHCO_2$), 5.88 (1H,d,J = 2Hz,6-H), 6.88 (1H,dd,J = 16,9Hz,CH = $CHCO_2$), 7.10-7.30 (5H,m,Ph);
Analysis Found: C,57.84%,H,7.58%;
$C_{47}H_{70}O_{20}$ requires C,57.88%; H, 7.07%.

Intermediate 38

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, tris[(2-methoxyethoxy)ethyl] ester

To a stirred solution of Intermediate 37 (500mg) in N,N-dimethylformamide (3ml) at 20°C, triethylamine (70μl) was added followed by N-methylhydroxylamine hydrochloride (46mg). The reaction was stirred for 16h and the solvent was then removed under reduced pressure. The residue was subjected to flash chromatography (2-10% methanol in chloroform) to give the title compound as a yellow oil (170mg); δ-($CD_3OD$) includes 0.83 (3H,d,J = 7Hz $CH_3$), 2.08 (3H,s,OAc), 3.35, 3,37, 3,40 (9H,3s,3x$OCH_3$), 4.17 (1H,d,J = 2Hz,7-H), 4.98, 5.03 (2H,2s, = $CH_2$), 5.15 (1H,d,J = 2Hz,6-H), 5.18 (1H,s,3H), 5.28 (1H,d,J = 5Hz,$OCH_2O$), 5.37 (2H,s,$OCH_2O$), 5.46 (1H,d,J = 5Hz,$OCH_2O$), 5.53 (1H,d,J = 5Hz,$OCH_2O$), 5.62 (1H,d,J = 5Hz,$OCH_2O$), 7.10-7.34 (5H,m,Ph).

Intermediate 39

[1S-[1α(4R*,5S*),3α,4β,5α,6α,7β]] 1-(4-Acetyloxy-5-methyl-3-methylene-6 phenylhexyl)-4,6,7-trihydroxy-2,8-dioxabicyclo[3.2.1]octane-3,4,5-tricarboxylic acid, tris(diphenylmethyl) ester

To a solution of Intermediate 28 (120mg) in dry N,N-dimethylformamide (1ml) was added N-methylhydroxylamine hydrochloride (17mg) and triethylamine (42μl). After stirring at room temperature for 16h the reaction mixture was diluted with ethyl acetate (50ml), washed with 2M hydrochloric acid (50ml), water (4x50ml) and brine (50ml), and dried ($Na_2SO_4$). The solvent was evaporated and the residue purified by flash chromatography (16g, Merck '9385' silica gel). Elution with ethyl acetate : petroleum ether (1:2) afforded the title compound as a white foam (97mg); δ($CDCl_3$) values include 0.88 (3H,d,J7Hz,$CH_3$), 2.08 (3H,s,$OCOCH_3$), 3.80 (1H,s,4-OH), 4.04 (1H,dd,J5Hz,J2Hz,7-H), 4.97-5.07 (4H,m, = $CH_2$,CHOAc,6-H), 5.27 (1H,s,3H), 6.72, 6.80 and 6.85 (3H,3s,3x$CHPh_2$), 7.02-7.30 (35H,m,aromatic protons);
Analysis Found: C,73.90;H,5.89;
$C_{64}H_{60}O_{13}$ requires C,74.10;H,5.85%.

Intermediate 40

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid,6,7-bis-octanoate, tris(diphenylmethyl) ester [Compound 1], [1S-[1α(4R*,5S*), 3α,4β,5α,6α,7β]] 1-(4-acetyloxy-5-methyl-3-methylene-6-phenylhexyl)4,6,7-trihydroxy-2,8-dioxabicyclo[3.2.1]octane-3,4,5-tricarboxylic acid, 7-octanoate, tris(diphenylmethyl) ester [Compound 2] and [1S-[1α-(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-octanoate, tris(diphenylmethyl) ester [Compound 3]

A solution of Intermediate 39 (519mg) and 4-dimethylaminopyridine (128mg) in dry dichloromethane (10ml) was stirred at 0°C, and octanoyl chloride (0.85ml) was added. The solution was stirred at 0°C for 20 minutes, and then partitioned between ethyl acetate (50ml) and 2N-hydrochloric acid (25ml). The organic phase was washed with water, and brine (25ml of each), and dried (magnesium sulphate), and evaporated to dryness. The residue was purified by flash chromatography on Merck '9385' Kieselgel (60g, petrol : ethyl acetate = 4:1 eluant) to give (in order of elution from the column):
Compound 1 (250mg); δ ($CDCl_3$) includes 0.8-0.9 (m,$CH_3$,9 protons), 1.1-1.3 (m,$OCOCH_2CH_2(CH_2)_4Me$), 2.06 (s,$OCOCH_3$) 3.93 (s,OH), 5.01 (broad s, = $CH_2$), 5.13 (d,J5Hz,CHOAc), 5.21 (broad s,3H,7H), 6.29 (broad s,6H), 6.68 (s), 6.85 (s), 6.87 (s), ($CHPh_2$), 7.05-7.3 (m,aromatic protons); Compound 2 (27mg); δ -($CDCl_3$) includes 0.8-0.9 (m,$CH_3$,6 protons), 1.15-1.35 (m,$OCOCH_2CH_2(CH_2)_4Me$), 2.05 (s,$OCOCH_3$), 2.72 (broad s,6OH), 3.80 (s,4OH), 4.85 (d,J2Hz,7H), 4.95 (s,3H, 5.00 (broad s, = $CH_2$), 5.10 (d,J5Hz CHOAc), 5.14

(narrow m,6H), 6.58 (s), 6.82 (s), 6.86 (s), (CHPh$_2$), 7.0-7.3 (m,aromatic protons); and Compound 3 (100mg) having a similar n.m.r. spectrum to an authentic sample prepared in Intermediate 15 hereinafter.

Intermediate 41

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-octanoate, tris(diphenylmethyl) ester

A solution of Intermediate 39 (104mg) in dry dichloromethane (5ml) was stirred at 20°C, and octanoyl chloride (0.5ml of 1M-solution in dichloromethane) and Calofort U ($CaCO_3$, 100mg) were added. The resulting suspension was stirred for 40 hours, then diluted with dichloromethane (25ml), washed with 2 N-hydrochloric acid (25ml), and dried (magnesium sulphate), and evaporated to dryness. The residue was purified by flash chromatography on Merck '9385' Kieselgel (20g, petrol : ethyl acetate = 4:1 eluant) to give the title compound (57mg) as a colourless oil; δ ($CDCl_3$) includes 0.8-0.95 (m,$CH_3$,6 protons), 1.2-1.4 (m,$OCOCH_2CH_2(CH_2)_4Me$), 1.55-1.8 (m,$OCOCH_2CH_2(CH_2)_4Me$), 2.06 (s,$OCOCH_3$), 3.20 (broad s,7OH), 3.90 (broad s,7H), 3.93 (s,4OH), 5.00 (broad s, =$CH_2$), 5.10 (d,J5Hz,CHOAc), 5.33 (s,3H, 5.75 (broad s,6H, 6.80 (s), 6.85 (s), 6.92 (s), (CHPh$_2$), 7.0-7.3 (m,aromatic protons).

Intermediate 42

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate),7-acetate, tris-(diphenylmethyl) ester

A solution of Intermediate 28 (476mg) in dry dichloromethane (15ml) was stirred at 20°C and acetyl bromide (74mg) and 4-dimethylaminopyridine (73mg) were added. The resulting solution was stirred for 18 hours, then more acetyl bromide (0.06ml) and 4-dimethylaminopyridine (98mg) were added, and stirring was continued for a further 24hours. The mixture was partitioned between ethyl acetate (100ml) and 2N-hydrochloric acid (50ml), and the organic phase was washed with water, and brine (50ml of each), and dried (magnesium sulphate), and evaporated to dryness. The residue was purified by flash chromatography on Merck '9385' Kieselgel (40g, petrol : ethyl acetate = 4:1 eluant) to give the title compound (334mg) as a colourless foam; δ ($CDCl_3$) includes 0.80-0.95 (m,$CH_3$,12 protons), 2.06 (s,$OCOCH_3$), 2.13 (s,$OCOCH_3$), 3.90 (s,OH), 4.88 (d,J16Hz,OCOCH=CH), 5.01 (broad s,=$CH_2$), 5.12 (d,J5Hz,CHOAc), 5.23 (s,3H), 5.28 (broad s,7H), 6.32 (broad s, 6H) 6.65 (dd, J8,16Hz,CH=CHCH(Me)), 6.71(s), 6.85 (s), 6.88 (s), (CHPh$_2$), 7.1-7.4 (m,aromatic protons).

Intermediate 43

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate), 7-octanoate, tris(diphenylmethyl) ester

A solution of Intermediate 28 (357mg), octanoyl chloride (0.4ml of 1M-solution in dichloromethane) and 4-dimethylaminopyridine (49mg) in dry dichloromethane (10ml) was stirred at 20°C for 18 hours. The solution was diluted with ethyl acetate (100ml), then washed with 2N-hydrochloric acid, water and brine (50ml of each), and dried (magnesium sulphate), and evaporated to dryness. The residue was purified by flash chromatography on Merck '9385' Kieselgel (40g, petrol : ethyl acetate = 4:1 eluant) to give the title compound (340mg) as a colourless foam; δ ($CDCl_3$) includes 0.80-0.95 (m,$CH_3$,15 protons), 1.2-1.4 (m,$OCOCH_2CH_2(CH_2)_4Me$), 1.6 (m,$OCOCH_2CH_2(CH_2)_4Me$), 2.39 (m,$OCOCH_2(CH_2)_5Me$), 2.06 (s,$OCOCH_3$), 3.91 (s,OH), 4.88 (d,J16Hz,OCOCH=CH), 5.01 (broad s,=$CH_2$), 5.12 (d,J5Hz CHOAc), 5.24 (s,3H), 5.29 (broad s,7H), 6.30 (broad s,6H), 6.63 (dd,J8,16Hz,CH=CHCH(Me)), 6.72 (s), 6.85 (s), 6.88 (s), (CHPh$_2$), 7.05-7.30 (m,aromatic protons).

Intermediate 44

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-acetate, tris(diphenylmethyl) ester

A solution of Intermediate 42 (324mg) in N,N-dimethylformamide (5ml) was stirred at 20°C, and N-methylhydroxylamine hydrochloride (44mg) and triethylamine (0.11ml) were added. The resulting suspension was stirred for 24hours, and then partitioned between ethyl acetate (50ml) and 2N-hydrochloric acid (25ml). The organic phase was washed with water (3x25ml), and brine (25ml), and dried (magnesium sulphate), and evaporated to dryness. The residue was purified by chromatography on Merck '9385' Kieselgel (30g, petrol : ethyl acetate = 2:1 eluant) to give the title compound (233mg) as a colourless foam; δ ($CDCl_3$) includes 0.83 (d,J8Hz,$CH_3$), 2.06 (s), 2.08 (s), ($OCOCH_3$), 2.71 (d,J3Hz,6OH), 3.77 (s,4OH), 4.85 (broad s,7H), 4.97 (s,3H), 4.99 (s,=$CH_2$), 5.09 (d,J5Hz,CHOAc), 5.16 (narrow m,6H), 6.58 (s), 6.82 (s), 6.86 (s), ($CHPh_2$), 7.0-7.3 (m,aromatic protons).

Intermediate 45

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-octanoate, tris(diphenylmethyl) ester

A solution of Intermediate 43 (300mg) in N,N-dimethylformamide (5ml) was stirred at 20°C, and N-methylhydroxylamine hydrochloride (38mg) and triethylamine (0.096ml) were added. The resulting suspension was stirred for 18 hours, and then partitioned between ethyl acetate (50ml) and 2 N-hydrochloric acid (25ml). The organic phase was washed with water(3x25ml), and brine (25ml), and dried (magnesium sulphate), and evaporated to dryness. The residue was purified by chromatography on Merck '9385' Kieselgel (30g, petrol : ethyl acetate = 4:1 eluant) to give the title compound (190mg) as a colourless foam; δ ($CDCl_3$) includes 0.8-0.9 (m,$CH_3$,6 protons), 1.2-1.3 (m,$OCOCH_2CH_2(CH_2)_4Me$), 2.05 (s,$OCOCH_3$), 2.65 (broad s,6OH), 3.78 (s,4OH, 4.85 (broad s,7H), 4.96 (s,3H), 5.00 (s,=$CH_2$), 5.10 (d,J5Hz,CHOAc), 5.13 (narrow m,6H), 6.60(s), 6.82(s), 6.86(s), ($CHPh_2$), 7.05-7.3 (m,aromatic protons).

Intermediate 46

[1S-[1α(4R*,5S*),3α,4β,5α,6α,7β]] 1-(4-Acetyloxy-5-methyl-3-methylene-6-phenylhexyl)-4,6-dihydroxy-7-[-(methoxyethoxy)methoxy)-2,8-dioxabicyclo[3.2.1]octane-3,4,5-tricarboxylic acid, 6-pentanoate, tris-(diphenylmethyl) ester

Intermediate 30 (600mg) was stirred (15h) at room temperature with pentanoyl chloride (96mg),4-dimethylaminopyridine (13mg) and triethylamine (270mg) in dichloromethane (25ml). After this time more 4-dimethylaminopyridine (13mg) and pentanoyl chloride (32mg) were added and the mixture stirred for a further 2h. Ethyl acetate (250ml) was then added and the organic solution washed with hydrochloric acid (2N, 100ml), water (100ml), saturated aqueous sodium bicarbonate solution (100ml) and finally brine (100ml). The organic phase was dried over anhydrous sodium sulphate and evaporated to an oil which was subjected to flash column chromatography on silica gel eluting with ethyl acetate : cyclohexane mixtures to give the title compound (466mg) as a white foam; δ($CDCl_3$) includes 2.05 (s,-$OCOCH_3$), 3.28 (s,-$CH_2OCH_3$), 3.92 (s,4-OH), 3.95 (d,J=1Hz,7-H), 4.75 and 4.89 (2d,J=7Hz,-$OCH_2O$-), 5.02 (s,C=$CH_2$), 5.14 (d,J=5Hz,-CH-OAc), 5.32 (s,3-H), 6.30 (d,J=1Hz,6-H), 6.59, 6.82 and 6.86 (3s,$Ph_2CH$-) and 7.02-7.35 (m,aromatic protons).

Intermediate 47

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(7-phenylheptanoate), tris-(diphenylmethyl) ester

Intermediate 30 (600mg) was stirred (15h) at room temperature with 7-phenylheptanoyl chloride (0.2ml), 4-dimethylaminopyridine (26mg) and triethylamine (270mg) in dichloromethane (25ml). After this time more 4-dimethylaminopyridine (26mg) and 7-phenyl heptanoyl chloride (0.1ml) were added and the mixture stirred for a further 2h. Ethyl acetate (250ml) was then added and the organic solution washed with hydrochloric acid (2N,100ml), water (100ml), aqueous saturated sodium bicarbonate soln (100ml) and finally brine (100ml). The organic phase was dried over sodium sulphate and evaporated to an oil which was subjected to flash column chromatography on silica gel eluting with ethyl acetate : cyclohexane mixtures to give the title compound (533mg) as a foam; δ ($CDCl_3$) includes 2.06 (s,-$OCOCH_3$), 3.28 (s,-$CH_2OCH_3$), 3.89 (s,4-OH), 4.00 (d,J=1Hz,7-H), 4.75 and 4.89 (2d,J=7Hz,-$OCH_2O$-), 5.02 (s,-CH=$CH_2$), 5.12 (d,J=5Hz,-CHOAc), 5.31 (s,3-H), 6.30 (d,J = 1Hz,6-H), 6.58,6.84,6.86 (3s,$PhCH_2$-), 7.02-7.36 (m,aromatic protons); Analysis Found: C74.0%,H6.9%;$C_{81}H_{84}O_{16}$ requires C74.1%,H6.5%.

Intermediate 48

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(4-cyclohexylbutanoate), tris(diphenylmethyl) ester

To a solution of Intermediate 30 (600mg) in dichloromethane (20ml) was added triethylamine (0.22ml), 4-cyclohexanebutyryl chloride (154mg) and 4-dimethylaminopyridine (6.6mg) and the mixture stirred at room temperature for 23h. After 20h, further 4-dimethylaminopyridine (6.6mg), and after 21h, further triethylamine (0.11ml) and 4-cyclohexanebutyryl chloride (77mg) were added. The reaction mixture was diluted with ethyl acetate (250ml) and washed successively with 0.5N aqueous hydrochloric acid (2x80ml), water (80ml), saturated aqueous sodium bicarbonate (80ml) and brine (80ml). The organic phase was dried ($MgSO_4$) and evaporated to a gum which was subjected to flash column chromatography on silica gel (Merck 9385, 350ml) eluting with ethyl acetate:cyclohexane (1:5) to give the title compound (478mg) as an opaque, cream gum; δ ($CDCl_3$) includes 0.84(d,3H,J = 7Hz,$CH_3$), 2.07(s,3H,$O_2CCH_3$), 3.30(s,3H,$OCH_3$), 3.91-(s,1H,4OH), 4.00(d,1H,J = 2Hz,7H), 4.76 and 4.90(2d,2H,J = 7Hz,$OCH_2O$), 5.02(broad s,2H,C = $CH_2$), 5.13-(d,1H,J = 7Hz, $CHO_2CCH_3$), 5.32(s,1H,3H), 6.30(d,1H,J = 2Hz,6H), 6.61,6.84,6.85(3s,3H,$Ph_2CH$), 7.01-7.43-(m,aromatic protons).

Intermediate 49

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(cyclohexanecarboxylate), tris(diphenylmethyl) ester

To a solution of Intermediate 30 (300mg) in dichloromethane (10ml) was added triethylamine (0.11ml), cyclohexanecarbonyl chloride (59mg) and 4-dimethylaminopyridine (3.3mg) and the mixture stirred at room temperature for 21h. After 18.5h, further cyclohexanecarbonyl chloride (30mg) and triethylamine (57μl) were added. The reaction mixture was diluted with ethyl acetate (150ml) and washed successively with 0.5N aqueous hydrochloric acid (2x50ml), water(50ml), saturated aqueous sodium bicarbonate (50ml) and brine (50ml). The organic phase was dried ($MgSO_4$) and evaporated to a gum which was subjected to flash column chromatography on silica gel (Merck 9385, 100ml) eluting with ethyl acetate:cyclohexane (1:4) to give the title compound (271mg) as an opaque cream-yellow gum; δ ($CDCl_3$) includes 0.84-(d,3H,J = 7Hz,$CH_3$), 2.06(s,3H,$O_2CCH_3$), 3.29(s,3H,$OCH_3$), 3.87(s,1H,4OH), 3.98(d,1H,J = 2Hz,7H), 4.76 and 4.88(2d,2H,J = 7Hz,$OCH_2O$), 5.02(broad s,2H,C = $CH_2$), 5.14(d,1H,J = 7Hz,$CHO_2CCH_3$), 5.36(s,1H,3H), 6.35-(d,1H,J = 2Hz,6H), 6.58,6.83,6.88(3s,3H,$Ph_2CH$), 7.03-7.36(m,aromatic protons).

Intermediate 50

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-[1-(tricyclo[3.3.1.1$^{3,7}$]-decyl)acetate], tris(diphenylmethyl) ester

To a solution of Intermediate 30 (400mg) in dichloromethane (10ml) was added triethylamine (0.15ml), 1-adamantaneacetyl chloride (115mg) and 4-dimethylaminopyridine (4.4mg) and the mixture stirred at room temperature for 23h. The reaction mixture was diluted with ethyl acetate (150ml) and washed successively with 0.5N aqueous hydrochloric acid (2x50ml), water(50ml), saturated aqueous sodium bicarbonate (50ml) and brine (50ml). The organic phase was dried ($MgSO_4$) and evaporated to a foam which was subjected to flash column chromatography on silica gel (Merck 9385, 150ml) eluting with ethyl acetate:cyclohexane (1:5) to give the title compound (386mg) as a clear, colourless gum; δ ($CDCl_3$) includes 0.85(d,3H,J = 7Hz,$CH_3$), 2.07(s,3H,$O_2CCH_3$), 3.28(s,3H,$OCH_3$), 3.89(s,1H,4OH), 4.04(d,1H,J = 2Hz,7H), 4.76 and 4.94-(2d,2H,J = 7Hz,$OCH_2O$), 5.03(broad s,2H,C = $CH_2$), 5.14(d,1H,J = 7Hz,$CHO_2CCH_3$), 5.34(s,1H,3H), 6.30-(d,1H,J = 2Hz,6H), 6.58,6.83,6.86(3s,3H,$Ph_2CH$), 7.03-7.43(m,aromatic protons).

Intermediate 51

[1S-[1α(4R*,5S*),3α,4β,5α,6α,7β]] 1-(4-Acetyloxy-5-methyl-3-methylene-6-phenylhexyl-4,6-dihydroxy-7-[(2-methoxyethoxy)methoxy]-2,8-dioxabicyclo[3.2.1]octane-3,4,5-tricarboxylic acid, 6-benzoate, tris-(diphenylmethyl) ester

To a solution of Intermediate 30 (600mg) in dichloromethane (20ml) was added triethylamine (0.37ml), benzoyl chloride (0.16ml) and 4-dimethylaminopyridine (13mg) and the mixture stirred at room temperature for 21h. After 19h, further triethylamine (0.11ml) and 4-dimethylaminopyridine (33mg) were added. The reaction mixture was diluted with ethyl acetate (150ml) and washed successively with 0.5N aqueous hydrochloric acid (2x75ml), water (75ml), saturated aqueous sodium bicarbonate (75ml) and brine (75ml). The organic phase was dried ($MgSO_4$) and evaporated to a foam which was subjected to flash column chromatography on silica gel (Merck 9385, 420ml) eluting with ethyl acetate:cyclohexane (1:4) to give the title compound (575mg) as a white, solidified foam; δ ($CDCl_3$) includes 0.85(d,3H,J=7Hz,$CH_3$), 2.05-(s,3H,$O_2CCH_3$), 3.23(s,3H,$OCH_3$), 3.89(s,1H,4OH), 4.15(d,1H,J=2Hz,7H), 4.82 and 4.98-(2d,2H,J=7Hz,$OCH_2O$), 5.03(broad s,2H,C=$CH_2$), 5.14(d,1H,J=7Hz,$CHO_2CCH_3$), 5.43(s,1H,3H), 6.54-(d,1H,J=2Hz,6H), 6.63,6.84,6.85(3s,3H,$Ph_2CH$), 7.02-7.63(m,aromatic protons).

Intermediate 52

[1S-[1α(4R*,5S*),3α,4β,5α,6α(2E),7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(3-phenyl-2-propenoate), tris(diphenylmethyl) ester

To a solution of Intermediate 30 (300mg) in dichloromethane (10ml) was added triethylamine (0.19ml), cinnamoyl chloride (113mg) and 4-dimethylaminopyridine (6.6mg) and the mixture stirred at room temperature for 24h. After 16h, further triethylamine (0.11ml), cinnamoyl chloride (45mg) and 4-dimethylaminopyridine (33.0mg) were added. The reaction mixture was diluted with ethyl acetate (150ml) and washed successively with 0.5N aqueous hydrochloric acid (2x50ml), water (50ml), saturated aqueous sodium bicarbonate (50ml) and brine (50ml). The organic phase was dried ($MgSO_4$) and evaporated to a foam which was subjected to flash column chromatography on silica gel (Merck 9385, 150ml) eluting with ethyl acetate:cyclohexane (1:4) to give the title compound (303mg) as an opaque cream gum; δ ($CDCl_3$) includes 0.85(d,3H,J=7Hz,$CH_3$), 2.06(s,3H,$O_2CCH_3$), 3.29(s,3H,$OCH_3$), 3.93(s,1H,4OH), 4.12(broad s,1H,7H), 4.80 and 4.97(2d,2H,J=7Hz,$OCH_2O$), 5.03(broad s,2H,C=$CH_2$), 5.15(d,1H,J=7Hz,$CHO_2CCH_3$), 5.39(s,1H,3H), 5.66(d,1H,J=15Hz,CH=CHPh), 6.46(broad s,1H,6H), 6.63,6.86 and 6.92(3s,3H,$Ph_2CH$), 7.03-7.44(m,aromatic protons), 7.48(d,1H,J=15Hz,CH=CHPh).

Intermediate 53

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(5-oxo-hexanoate), tris-(diphenylmethyl) ester

Intermediate 30 (1.125g) was dissolved in acetone (40ml) and 1,3-dicyclohexylcarbodiimide (515mg) and 4-dimethylaminopyridine (30mg) were added. The reaction mixture was allowed to stand at room temperature for 20h. It was then filtered and the filtrate was evaporated to dryness. The gum was dissolved in dichloromethane and chromatographed on silica (40-63μ) using ethyl acetate-cyclohexane 1:3 and ethyl acetate 1:2 as solvent to give the title compound (1.15g). A sample of the title compound was purified by preparative plate chromatography using ethyl acetate-cyclohexane 1:7 as solvent. Elution with ethyl acetate gave an analytical sample of the title compound; δ ($CDCl_3$) includes 0.83(d,J=6Hz,$CH_3$), 2.08 and 2.09-(2s,$COCH_3$ and $OCOCH_3$), 3.30(s,$OCH_3$), 3.90(s,4OH), 4.01(s,7H), 4.74 and 4.90(2d,J=6Hz,$OCH_2O$), 5.02-(s,=$CH_2$), 5.14(d,J=4Hz,CHOAc), 5.31(s,3H), 6.30(s,6H), 6.61(s,$CHPh_2$), 6.82,6.83(2s,$CHPh_2$), 7.05-7.3-(aromatic protons);
Analysis Found: C,71.65; H,6.3,
$C_{74}H_{76}O_{11}$ requires C,71.8; H,6.2%.

Intermediate 54

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 6-(6-oxo-heptanoate), tris-

(diphenylmethyl) ester

Intermediate 30 (1g) was dissolved in dichloromethane (70ml, dried over molecular sieves 3A) and 1,3-dicyclohexylcarbodiimide (520mg) and 4-dimethylaminopyridine (30mg) were added. The reaction mixture was allowed to stand at room temperature for 24h. It was then filtered and the filtrate was evaporated to dryness and purified by preparative plate chromatography using ethyl acetate-dichloromethane 1:9 as solvent. Elution with ethyl acetate gave the title compound (560mg); δ ($CDCl_3$) includes 0.88(d,J=6Hz,$CH_3$), 2.1 and 2.16(2s,$COCH_3$ and $OCOCH_3$), 3.34(s,$OCH_3$), 3.92(s,4OH), 4.05(s,7H), 4.78,4.93(2d,J=6Hz,$OCH_2O$), 5.06(s,=$CH_2$), 5.16(d,J=5Hz,CHOAc), 5.35(s,3H), 6.33(s,6H), 6.63(s,$CHPh_2$), 6.87.6.88(2s,$CHPh_2$), 7.05-7.35(aromatic protons);
Analysis Found: C,71.6; H,6.5,
$C_{75}H_{78}O_{17}$ requires C,72.0; H,6.3%.

Intermediate 55

[1S-[1α(4R*,5S*),3α,4β,5α,6α,7β]] 1-(4-Acetyloxy-5-methyl-3-methylene-6-phenylhexyl)-4-hydroxy-6-[-(methoxycarbonyl)oxy]-7-[(2-methoxyethoxy)methoxy]-2,8-dioxabicyclo[3.2.1]octane-3,4,5-tricarboxylic acid, tris(diphenylmethyl) ester.

A solution of Intermediate 30 (405mg) and 4-dimethylaminopyridine (88mg) in dichloromethane (1.6ml) was cooled to 0°C under nitrogen. Methyl chloroformate (28μl) was added with vigorous stirring and the resulting reaction mixture allowed to warm to room temperature with subsequent stirring for 45 minutes. The solvent was removed from the reaction mixture under reduced pressure and the residue separated between ethyl acetate (60 ml) and 1N HCl (25 ml). The organic layer was then washed with $H_20$ (2 x 20 ml) and saturated brine (20 ml), dried over $MgSO_4$ and evaporated under reduced pressure to yield the title compound (376.9mg) as a white foam; δ ($CDCl_3$) includes 0.86 (d,J=6.5 Hz, $CH_3$), 2.08 (s, $OCOCH_3$), 3.31 (s, $OCH_2CH_2OCH_3$), 3.60 (s, $OCO_2CH_3$), 3.85 (s, 4OH), 4.11 (s, 7H), 4.75. 4.92 (ABq, J=44 Hz. -$OCH_2O$-), 5.00 (s,=$CH_2$), 5.12 (d,J=5 Hz, CHOAc), 5.29 (s, 3H), 6.18 (s, 6H), 6.64 (s, $CHPh_2$), 6.83 (s, $CHPh_2$), 6.85 (s, $CHPh_2$), 7.0-7.4(m, aromatic protons).
Analysis found: C,70.7; H,6.06;
$C_{70}H_{70}O_{17}$ requires : C,71.05; H,5.96%

Intermediate 56

[1S-[1α(4R*,5S*),3α,4β,5α.6α,7β]] 1-(4-Acetyloxy-5-methyl-3-methylene-6-phenylhexyl)-4-hydroxy-6-[-(nonoxycarbonyl)oxy]-7-[(2-methoxyethoxy)methoxy]-2,8-dioxabicyclo[3.2.1]octane-3,4,5-tricarboxylic acid, tris(diphenylmethyl) ester.

A solution of Intermediate 30 (405.1mg) and 4-dimethylaminopyridine (88mg) in dichloromethane (1.5 ml) was cooled to 0°C under nitrogen. To this cold solution was added nonyl-chloroformate (2.4 ml of a 0.45M solution in dichloromethane) with vigorous stirring. The resulting reaction mixture was allowed to warm to room temperature and was subsequently stirred for 16 hours. The solvent was removed from the reaction mixture under reduced pressure and the residue separated between ethyl acetate (50 ml) and 1N HCl (25 ml). The organic phase was washed with water(2 x 15 ml) and saturated brine (20ml), and then dried over $MgSO_4$. The solvent was then removed under reduced pressure to yield a gum which was purified by flash column chromatography on Merck Kieselgel 60 (40 g) with 20% ethyl acetate/petroleum ether as eluent. The appropriate fractions were combined and the solvent removed under reduced pressure to yield the title compound, (347.3mg) as a white foam; δ ($CDCl_3$) includes 0.83 (d, J=6.75 Hz, $CH_3$), 0.89 (t, J=6.5 Hz, $OCO_2(CH_2)_8CH_3$), 1.28 (s, broad, $OCO_2CH_2(CH_2)_7CH_3$), 2.06 (s, $OCOCH_3$), 3.31 (s, -$OCH_2CH_2OCH_3$), 3.42 (t, J=4.5 Hz, $OCO_2CH_2(CH_2)_7CH_3$), 3.83 (s, 4 OH), 4.12 (d, J=1.5 Hz, 7H), 4.76, 4.93 (ABq, J=44 Hz, -$OCH_2O$-), 5.10 (d, J=5 Hz CHOAc), 5.27 (s, 3H, 6.18 (d, J=1.5 Hz, 6 H), 6.58 (s, $CHPh_2$), 6.82 (s, $CHPh_2$), 6.84 (s, $CHPh_2$), 7.0-7.31 (m, aromatic protons).
Analysis Found: C,72.35; H,6.82;
$C_{78}H_{86}O_{17}$ requires : C,72.31; H,6.69%.

Intermediate 57

[1S-[1α(4R*,5S*)3α,4β,5α,6α,7β]] 1-(4-Acetyloxy-5-methyl-3-methylene-6-phenylhexyl)-6-[(butoxycarbonyl)-

oxy]-4-hydroxy-7-[(2-methoxyethoxy)methoxy]-2,8-dioxabicyclo[3.2.1]octane-3,4,5-tricarboxylic acid, tris-(diphenylmethyl) ester

Intermediate 30 (600mg) was stirred (16h) at room temperature with n-butyl chloroformate (217.5mg), triethylamine (270mg) and 4-dimethyl aminopyridine (109mg) in dichloromethane (25ml). Ethyl acetate (250ml) was then added and the organic solution washed with hydrochloric acid (2N, 100ml), water (100ml), aqueous sodium bicarbonate soln (saturated, 100ml) and finally brine (100ml). The organic phase was dried over anhydrous sodium sulphate and evaporated to an oil which was subjected to flash column chromatography on silica gel eluting with ethyl acetate : cyclohexane mixtures gave the title compound (527mg) as a foam; δ($CDCl_3$) includes 2.06 (s,-$OCOCH_3$), 4.12 (d,J=1Hz,7-H), 4.75 and 4.94 (2d,J=7Hz,-$OCH_2O$-), 4.98 (s,-C=$CH_2$), 5.10 (d,J=5Hz,-CH-OAc), 5.26 (s,3-H), 6.18 (d,J=1Hz,6-H), 6.59, 6.80 and 6.84 (3s,$PhCH_2$-), 7.00-7.36 (m,aromatic protons). Analysis found C71.4%,H6.4%;$C_{73}H_{76}O_{17}$ requires C71.6%,H6.3%.

Intermediate 58

[1S-[1α(4R*,5S*),3α,4β,5α,6α,(2E,4R*,6R*),7β]] 1-(4-Acetyloxy-5-methyl-3-methylene-6-phenylhexyl)-6-hydroxy-4,7-bis[(2-methoxyethoxy)methoxy]-2,8-dioxabicyclo[3.2.1]octane-3,4,5-tricarboxylic acid, 6-(4,6-dimethyl-2-octenoate), tris(diphenylmethyl) ester

To a cooled (5°C) stirred solution of Intermediate 28 (476mg) in dichloroethane (5ml) was added N,N-diisopropylethylamine (258mg, 0.35ml) and 2-methoxyethoxymethyl chloride (250mg, 0.228ml) keeping the temperature below 5°C by means of an ice/salt bath. The solution was allowed to warm to 20°C over 30 minutes and then heated to reflux for 6hr. The solution was cooled (20°C) and further N,N-diisopropylethylamine (129mg, 0.175ml) and 2-methoxyethoxymethyl chloride (125mg, 0.114ml) added. The resulting solution was heated to reflux for 16hr. The cooled solution was diluted with ethyl acetate (200ml), and washed sequentially with 2N hydrochloric acid (2x30ml), water (30ml), saturated sodium bicarbonate (2x30ml), water (30ml) and brine (30ml), dried ($MgSO_4$) and evaporated to dryness, under reduced pressure, to leave a gum (576mg).

The gum was purified by flash chromatography on Merck '9385' Kieselgel (64g; petrol : ethyl acetate = 5:1 eluant). The appropriate fractions were combined and evaporated to dryness, under reduced pressure, to furnish a solid (441mg).

A sample (380mg) of the solid was further purified by flash chromatography on Merck '9385' Kieselgel (36g; dichloromethane : ethyl acetate = 45:1 eluant). The appropriate fractions of the more polar product were combined and evaporated to dryness, under reduced pressure to afford the title compound (23mg) as a colourless gum; δ ($CDCl_3$) includes 0.75-0.93 (m,$CH_3$,12 protons), 2.05 (s,$OCOCH_3$), 3.05 (m,4$OCH_2CH_2OCH_3$), 3.09 (s,4$OCH_3$), 3.29 (s,7$OCH_3$), 3.45(m,4$OCH_2CH_2OCH_3$, 7$OCH_2CH_2OCH_3$), 3.65 (m,7$OCH_2CH_2OCH_3$), 4.01 (d,J2Hz,7H), 4.72-4.95 (m,4,7$OCH_2OCH_2CH_2OCH_3$,CH=CHCH($CH_3$)), 5.01 (broad s,=$CH_2$), 5.13 (d,J4Hz, CHOAc), 5.37 (s,3H), 6.41 (broad s,6H), 6.61 (dd,J7Hz,12Hz,CH=CHCH-($CH_3$)), 6.7 (broad s), 6.8 (s), 6.86 (s) ($CHPh_2$), 7.04-7.35 (m, aromatic protons);
Analysis Found: C,71.7; H,6.9
$C_{82}H_{92}O_{18}$ requires : C,72.1; H,6.8%.

Intermediate 59

[1S-[1α(4R*,5S*),3α,4β,5α,6α,7β]] 1-(4-Acetyloxy-5-methyl-3-methylene-6-phenylhexyl)-6-hydroxy-4,7-bis[(2-methoxyethoxy)methoxy]-2,8-dioxabicyclo[3.2.1]octane-3,4,5-tricarboxylic acid, tris(diphenylmethyl) ester

To a stirred solution of Intermediate 58 (632mg) in anhydrous N,N-dimethylformamide (5ml) at room temperature under nitrogen was added N-methylhydroxylamine hydrochloride (77mg) and triethylamine (0.19ml). The resultant mixture was stirred for 66 hours at room temperature under nitrogen. The reaction mixture was separated between water (150ml) and ethyl acetate (500ml). The organic phase was washed with water (4x150ml) and saturated brine (1x100ml). The organic phase was dried over anhydrous $MgSO_4$ and the solvent was removed under reduced pressure to yield an off-white viscous oil (667mg). This material was purified by flash chromatography on Merck Kieselgel 60 (34g) packed with cyclohexane eluted in a gradient to cyclohexane, ethyl acetate (1:1). The appropriate fractions were combined and the solvent removed under reduced pressure to yield the title compound as a white foam (536mg); δ ($CDCl_3$) includes 0.84 (d,7.5Hz,3H,$CH_3$), 2.06 (s,3H,$OCOCH_3$), 3.02 (s,3H,4$OCH_2CH_2OCH_3$), 3.20 (s,3H,7$OCH_2CH_2OCH_3$), 3.85 (d,2.25Hz,1H,7CH), 4.29 (brd,6.25Hz,1H,6OH), 5.00 (brs,2H,=$CH_2$), 5.08-5.11 (m,2H,CHOAc and 3CH),

6.63 (s,1H,CHPh$_2$), 6.74 (s,1H,CHPh$_2$), 6.88 (s,1H,CHPh$_2$), 6.96-7.39ppm (m,35H aromatic protons); Analysis Found: C,71.34; H,6.29
$C_{72}H_{76}O_{17}$ requires : C,71.27; H,6.29%.

Intermediate 60

[1S-[1α(4R*,5S*),3α,4β,5α,6α,7β]] 1-(4-Acetyloxy-5-methyl-3-methylene-6-phenylhexyl)-6-methoxy-4,7-bis[(2-methoxyethoxy) methoxy]-2,8-dioxabicyclo[3.2.1]octane-3,4,5-tricarboxylic acid, tris(diphenylmethyl) ester

Powdered potassium hydroxide (47mg) was vigorously stirred at room temperature with dimethylsulphoxide (3ml) for 15 minutes. A solution of Intermediate 59 (511mg) in dimethylsulphoxide (4ml) was added and the mixture treated immediately with iodomethane (0.53ml). The resultant mixture was stirred at room temperature for 4½ hours.

The reaction mixture was separated between ethyl acetate (250ml) and water (100ml). The organic phase was washed with water (4x100ml) and saturated brine (1x100ml). The organic phase was dried with anhydrous $MgSO_4$ and the solvent was removed under reduced pressure. The residue was purified by flash chromatography using Merck Kiesselgel 60 (47g) packed with cyclohexane eluted in a gradient to cyclohexane, ethyl acetate (2:3). The appropriate fractions were combined and the solvent was removed under reduced pressure to yield the title compound, as a white solid (97mg) δ ($CDCl_3$) includes 0.84 (d,J7.5Hz,3H,CH$_3$), 2.06 (s,3H,OCOCH$_3$), 2.84 (s,3H,6OCH$_3$), 3.03 (s,3H,4OCH$_2$CH$_2$OCH$_3$), 3.31 (s,3H,7OCH$_2$CH$_2$OCH$_3$), 3.99 (d,J2.2Hz,1H,7CH), 5.00 (s,2H,=CH$_2$), 5.1 (d,J6.25Hz,1H,CHOAc), 5.14 (s,1H,3CH), 6.71 (brs,1H,CHPh$_2$), 6.83 (s,1H,CHPh$_2$), 7.03-7.39 (m,35H,aromatic protons);

Intermediate 61

[1S-[1α(4R*,5S*),3α,4β,5α,6α,7β]] 1-(4-Acetyloxy-5-methyl-3-methylene-6-phenylhexyl)-6-[(aminocarbonyl)-oxy]-4-hydroxy-7-[(2-methoxyethoxy)methoxy]-2,8-dioxabicyclo[3.2.1]octane-3,4,5-tricarboxylic acid, 3,4,5-tris(diphenylmethyl) ester

A solution of Intermediate 30 (250mg) in dichloromethane (5ml) was cooled to 0°C and treated with chlorosulphonylisocyanate (40μL). The mixture was stirred for 1h at 20°C and then aqueous saturated sodium bicarbonate solution was added. The mixture was stirred for 2.5h and then the dichloromethane was removed under reduced pressure. The residue was diluted with ethyl acetate and the organic phase was washed with $NaHCO_3$, 2M HCl, brine, dried and evaporated to dryness to give the title compound (258mg); δ($CDCl_3$)(V175432) includes 0.85 (d,J=7Hz,CH$_3$CH), 2.06 (s,AcO), 3.30 (s,OCH$_3$), 3.93 (s,4-OH), 4.09 (d,J=2Hz,7-H), 4.75 and 4.99 (2d,J=8Hz,OCH$_2$O), 5.02 (s,C=CH$_2$), 5.14 (d,J=6Hz,CHOAc), 5.30 (s,3-H), 6.17 (d,J=2Hz,6-H), 6.62 and 6.85 (2s, CHPh$_2$), 7.05-7.35 (aromatic protons).

Intermediate 62

[1S-[1α(4R*,5S*),3α,4β,5α,6α,7β]] 1-(4-Acetyloxy-5-methyl-3-methylene-6-phenylhexyl)-4,6-dihydroxy-7-[(2-methoxyethoxy)methoxy]-2,8-dioxabicyclo[3.2.1]octane-3,4,5-tricarboxylic acid, 3,4,5-tris(diphenylmethyl) ester, 6-[(E/Z)-3-octenoate]

A solution of Intermediate 30 (1.5g) in dichloromethane (25ml) and triethylamine (674mg) was treated with 4-dimethylaminopyridine (33mg) and octenoyl chloride (301mg) (prepared from 2-octenoic acid and thionyl chloride at reflux for 2h, followed by evaporation of excess thionyl chloride). The mixture was stirred at room temperature for 24h when further quantities of the octenoyl chloride (500mg), 4-dimethylaminopyridine (66mg) and triethylamine (3ml) were added. The mixture was stirred for a further 24h, diluted with ethyl acetate (250ml) and washed with 3N hydrochloric acid (3x200ml), saturated aqueous sodium bicarbonate solution (2x200ml) and brine (200ml). The organic phase was dried ($MgSO_4$) and evaporated to give a brown foam. This was chromatographed on silica gel (Merck 7734, 200g) eluting with cyclohexane:ethyl acetate (4:1). Appropriate fractions were combined and evaporated to give the title compounds as a pale brown gum (477mg); δ($CDCl_3$) includes 2.06 (s,OCCH$_3$), 3.28 (s,CH$_2$OCH$_3$), 3.87 (s,4-OH), 4.00 (s,7-H), 5.01 (s,=CH$_2$), 5.12 (d,J=5Hz,CHOCOCH$_3$), 5.32 (s,3-H, 6.62 and 6.63 (2d,J<2Hz,6-H,E/Z isomers), 7.00-7.35 (m,aromatic protons).
Analysis Found: C,72.47;H,6.62%
$C_{76}H_{80}O_{16}.\frac{1}{2}H_2O$ requires: C,72.53;H,6.49%.

Intermediate 63

[1S-[1α(4R*,5S*),3α,4β,5α,6α,7β]] 1-(4-Acetyloxy-5-methyl-3-methylene-6-phenylhexyl)-6-[[(ethylamino)-carbonyl]oxy]-4-hydroxy-7-[(2-methoxyethoxy)methoxy]-2,8-dioxabicyclo[3.2.1]octane-3,4,5-tricarboxylic acid, 3,4,5-tris(diphenylmethyl) ester

A solution of Intermediate 30 (515mg) in dichloromethane (3ml) and triethylamine (1ml) was treated with 4-dimethylaminopyridine (12mg) and ethyl isocyanate (0.25ml). The mixture was heated to 60°C for 2h and then stirred at 20°C for 66h. The solution was diluted with dichloromethane and washed with 2M HCl (x4), dried, and chromatographed on silica gel (Merck 7734; 75g) eluting with dichloromethane and then ethyl acetate:dichloromethane (1:19) to give the title compound (237mg); $\nu_{max}$ ($CHBr_3$) 3422 (N-H and O-H), 1732cm$^{-1}$ (C=O); δ($CDCl_3$) includes 0.8-0.9(m,$CH_3$), 0.93(t,J7Hz,$CH_3CH_2N$), 2.07(s,AcO), 3.3(s,$OCH_3$), 3.96(s,4-OH), 4.10(d,J<2Hz,7-H), 5.13(d,J5Hz,CHOAc), 5.3(s,3-H), 6.18(d,J<2Hz,6-H), 6.62 and 6.84-(s,$CHPh_2$), 7.02-7.4(m,$C_6H_5$).

Intermediate 64

[1S-[1α(4R*,5S*),3α,4β,5α,6α,7β]] 1-(4-Acetyloxy-5-methyl-3-methylene-6-phenylhexyl)-4-hydroxy-7-[(2-methoxyethoxy)methoxy]-6-[[methylamino)carbonyl]oxy]-2,8-dioxabicyclo[3.2.1]octane-3,4,5-tricarboxylic acid, 3,4,5-tris(diphenylmethyl) ester

A solution of Intermediate 30 (500mg) in dichloromethane (10ml) and pyridine (5ml) was added to phosgene (2ml) at 0°C. The mixture was diluted with dichloromethane (15ml) and stirred for 1h allowing the temperature to rise to 15°C. Excess methylamine gas was condensed into the reaction mixture at -50°C and then water (15ml) was added. The mixture was stirred vigorously and allowed to warm to 20°C. The two phases were separated and the organic phase was washed with aqueous HCl, water, dried and chromatographed on silica gel (Merck 7734; 30g) eluting with ethyl acetate:cyclohexane (1:3) increasing to (9:11) to give the title compound (180mg); $\nu_{max}$ ($CHBr_3$) 3430 (N-H and O-H), 1733 (C=O, esters), 1681cm$^{-1}$ (C=O, carbamate); δ ($CDCl_3$) includes 0.85(d,J7Hz,$CHCH_3$), 2.08(s,AcO), 2.45(d,J6Hz,$NHCH_3$), 3.31(s,$OCH_3$), 3.99(d,J<2Hz,7-H), 5.02(s,=$CH_2$), 5.13(d,J5Hz, CHOAc), 5.3(s,3-H), 6.17(d,J<2Hz,6-H), 6.66, 6.82 and 6.85(3s,$CHPh_2$), 7.03-7.45(m,$C_6H_5$).

Example 1

[1S-[1α(4S*,5S*),3α,4β,5α,6α(2E,4R*,6R*),7β]] 1-(4-Hydroxy-5-methyl-3-methylene-6-phenylhexyl)-4,6,7-trihydroxy-2,8-dioxabicyclo[3.2.1]octane-3,4,5-tricarboxylic acid, 6-(4,6-dimethyl-2-octenoate)

To a stirred solution of Intermediate 3 (250mg) in water:tetrahydrofuran (1:2) (15ml) at room temperature was added zinc dust (1.00g) then 2M aqueous hydrochloric acid, dropwise, until the pH remained constant at ca. 1.0. The mixture was filtered in vacuo and extracted with ethyl acetate (3x10ml). The extracts were combined, washed with 2M aqueous hydrochloric acid (3x10ml) and brine (10ml), dried over magnesium sulphate, and evaporated to a gum. This was purified by preparative hplc on a Spherisorb ODS-2 (20x250mm) column eluting with 43% (acetonitrile:water(95:5) containing 0.15ml/l conc. $H_2SO_4$): 57% (water containing 0.15ml/l conc. $H_2SO_4$). Appropriate fractions from each run were combined, the acetonitrile removed in vacuo (bath temperature <40$^0$C) and the remainder extracted with ethyl acetate. The combined extracts were washed with water, dried over magnesium sulphate and evaporated to give the title compound (20mg) as a clear, colourless gum; [ α]$^{23}$D + 2.03$^0$ (c. 1.009 in $CHCl_3$); δ ($CD_3OD$) includes 0.73-(d,3H,J=7Hz,$CH_3$), 0.82-0.94(m,6H,$CH_3$), 1.03(d,3H,J=7Hz,$CH_3$), 3.86 (d,J=7Hz,=CCHOH), 5.27(s,1H,3H), 5.78(d,1H, J=15Hz,CH=$CHCO_2$), 6.33(broad s,1H,6H), 6.84 (dd,1H,J=15,7Hz,CH=$CHCO_2$), 7.08-7.28 (m,5H,aromatic protons).

Example 2

[1S-[1α[(E),5S*],3α,4β,5α,6α(2E,4R*,6R*),7β]] 1-(3,5-Dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyl-2-octenoate)

A solution of Intermediate 6 (80mg) in anisole (0.4ml) was treated with trifluoroacetic acid (1.6ml) and the resulting mixture left to stand at room temperature for ½h. The mixture was diluted with diethyl ether

(20ml) and extracted with 1% aqueous potassium bicarbonate (2x20ml). The combined extracts were washed with diethyl ether(20ml) and acidified with 2N aqueous hydrochloric acid. The resultant mixture was extracted with ethyl acetate (2x20ml) and the summed extracts washed with water (20ml) and brine (20ml), dried over magnesium sulphate and evaporated to a gum. This was purified by preparative hplc on a Spherisorb ODS-2 (20x250mm) column eluting with 58% acetonitrile:water(95:5) containing 0.15ml/l conc. $H_2SO_4$:42% water containing 0.15ml/l conc. $H_2SO_4$. Appropriate fractions from each run were combined, the acetonitrile removed in vacuo (bath temperature <$40^0$C) and the remainder extracted with ethyl acetate. The combined extracts were dried over magnesium sulphate and evaporated to a clear gum. This was dissolved in 1,4-dioxan and freeze-dried to give the title compound (16 mg) as a white powder; $\delta$ ($CD_3OD$) includes 0.75-0.92(m,9H,$CH_3$), 1.02(d,3H,J=7Hz, $CH_3$), 3.72(sextet,1H,J=7Hz,$CH_2CH(CH_3)CO$), 5.32(broad s,1H,3H, 5.53(d,1H, J=15Hz,CH=$CHCO_2$), 6.37(d,1H,J=2Hz,6H), 6.76(dd,1H,J=15,7Hz,CH=$CHCO_2$), 7.04-7.29-(m,6H,CH=C($CH_3$)COCH and aromatic protons); analytical HPLC, retention time 6.54 min (Spherisorb ODS-2, solvent 40-70% acetonitrile -0.05M ammonium phosphate).

Example 3

[1S-[1α(5S*),3α,4β,5α,6α(2E,4R*,6R*),7β]] 1-(5-Methyl-3-methylene-4-oxo-6-phenylhexyl)-4,6,7-trihydroxy-2,8-dioxabicyclo[3.2.1]octane-3,4,5-tricarboxylic acid, 6-(4,6-dimethyl-2-octenoate)

A solution of Intermediate 5 (150mg) in anisole (0.75ml) was treated with trifluoroacetic acid (3ml) and the resulting mixture left to stand at room temperature for ½h. The mixture was concentrated in vacuo, diluted with diethyl ether (30ml) and extracted with 1% aqueous potassium bicarbonate (2x30ml). The combined extracts were washed with diethyl ether (30ml) and acidified with 2N aqueous hydrochloric acid. The resultant mixture was extracted with ethyl acetate (2x30ml) and the summed extracts washed with water (30ml) and brine (30ml), dried over magnesium sulphate and evaporated to a foam. This was purified by preparative hplc on a Spherisorb ODS-2 (20x250mm) column eluting with 54% acetonitrile:water (95:5) containing 0.15ml/l conc. $H_2SO_4$:46%A water containing 0.15ml/l conc. $H_2SO_4$. Appropriate fractions from each run were combined, the acetonitrile removed in vacuo (bath temperature <$40^0$C) and the remainder extracted with ethyl acetate. The combined extracts were dried over magnesium sulphate, and evaporated to a clear gum. This was disolved in 1,4-dioxan and freeze-dried to give the title compound (68mg) as a white powder; $\nu_{max}$ (nujol) 3700-2300(OH,carboxylic acid), 1741$cm^{-1}$(C=O); $\delta$ ($CD_3OD$) includes 0.84-0.92-(m,6H,$CH_3$), 1.04(d,3H,J=7Hz,$CH_3$), 1.08(d,3H,J=7Hz $CH_3$), 5.26(s,1H,3H), 5.87 and 6.06 (2s, 2H, C=$CH_2$), 6.29 (d, 1H, J=2Hz, 6H), 6.84(dd,1H,J=15,7Hz,CH=$CH_2CO_2$), 7.09-7.28(m,5H,aromatic protons).

Example 4

[1S-[1α[(E),4S*,5S*],3α,4β,5α,6α(2E,4R*,6R*),7β]] 1-(4-Hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate)

A solution of title Compound 2 of Intermediate 8 (29mg) in water:tetrahydrofuran (1:2) (3m1) was treated with zinc dust (72mg) then 2M aqueous hydrochloric acid until the pH remained constant at ca. 1.0. The resulting mixture was filtered in vacuo and extracted with ethyl acetate (3x2ml). The summed extracts were washed with 2M aqueous hydrochloric acid (2x2ml) and brine (2ml), dried over magnesium sulphate and evaporated to a gum. This was purified by preparative hplc on a Spherisorb ODS-2 (20x250mm) column eluting with 58% acetonitrile:water (95:5) containing 0.15ml/l conc. $H_2SO_4$: 42% water containing 0.15ml/l conc. $H_2SO_4$. Appropriate fractions from each run were combined, the acetonitrile removed in vacuo (bath temperature <$40^0$C) and the remainder extracted with ethyl acetate. The combined extracts were dried over magnesium sulphate and evaporated to a clear gum. This was disolved in 1,4-dioxan and freeze-dried to give the title compound as a white powder (8mg); $\delta$ ($CD_3OD$) includes 0.61(d,3H,J=7Hz,$CH_3$), 0.78-0.94-(m,$CH_3$), 0.96(d,3H,J=7Hz,$CH_3$), 1.68(s,3H,CH=C($CH_3$)), 5.25(s,1H,3H), 6.30(broad s,1H,6H), 6.77-(dd,1H,J=15,7Hz,CH=$CHCO_2$), 7.09-7.29(m,5H,aromatic protons); approximate molecular weight 648; -FAB mass spectrometry $[M-H]^-$ 647; -FAB mass spectrometry [M-$CO_2$H]- 603.

Example 5

[1S-[1α[(E),4R*,5S*],3α,4β,5α,6α(2E,4R*,6R*),7β]] 1-(4-Hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate)

A solution of title Compound 1 of Intermediate 8 (40mg) in water:tetrahydrofuran (1:2) (3ml) was treated with zinc dust (99mg) then 2M aqueous hydrochloric acid until the pH remained constant at ca. 1.0. The resulting mixture was filtered in vacuo and extracted with ethyl acetate (3x2ml). The summed extracts were washed with 2M aqueous hydrochloric acid (2x2ml) and brine (2ml), dried over magnesium sulphate and evaporated to a gum. This was purified by preparative hplc on a Spherisorb ODS-2 (20x250mm) column eluting with 58% acetonitrile:water (95:5) containing 0.15ml/l conc. $H_2SO_4$: 42% water containing 0.15ml/l conc. $H_2SO_4$. Appropriate fractions from each run were combined, the acetonitrile removed in vacuo (bath temperature <40°C) and the remainder extracted with ethyl acetate. The combined extracts were dried over magnesium sulphate and evaporated to a clear gum. This was disolved in 1,4-dioxan and freeze-dried to give the title compound as a white powder (6mg); δ ($CD_3OD$) includes 0.78-0.93(m,9H,$CH_3$), 0.96-(d,3H,J = 7Hz,$CH_3$), 1.67(s,3H,CH = C($CH_3$)), 5.26(s,1H,3H), 5.58(d,1H,J = 15Hz,CH = $CHCO_2$), 6.33(broad s,1H,6H), 6.77(dd,1H,J = 15,7Hz, CH = $CHCO_2$), 7.09-7.29(m,5H,aromatic protons); approximate molecular weight 648; -FAB mass spectrometry [M-H]- 647; -FAB mass spectrometry [M-$CO_2$H]- 603.

Example 6

[1S-[1α[3R*(S*),5S*],3α,4β,5α,6α(2E,4R*,6R*),7β]] 1-(3,5-Dimethyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), isomer 1 and isomer 2

A solution of Intermediate 9 (464mg) in tetrahydrofuran (20ml) and water (8ml) was treated with zinc dust (1.15g), stirred and treated dropwise over five minutes at 21°C with 2M-hydrochloric acid (7ml) until the pH remained at ca 1.5. After a further 20 minutes the mixture was filtered and the retained solid was washed with ethyl acetate (50ml). Combined filtrate and washings were further diluted with ethyl acetate (50ml) and washed with 2M- hydrochloric acid, water and brine (2x50ml each), dried ($MgSO_4$) and evaporated to a foam. The two epimers were separated by preparative HPLC using a Spherisorb ODS-2 (2x25cm) column eluting with acetonitrile : water 1:1 containing sulphuric acid (0.15ml/L), flow rate 10ml/min. The required fractions were combined, partially evaporated under reduced pressure, and extracted with ethyl acetate. The extracts were dried and evaporated to give the title compounds as white solids. Isomer 1 (72mg) δ($CDCl_3$) includes 0.8-0.9 (m,$CH_3$), 0.95-1.15 (m,$CH_3$), 2.8-3.1 (m,$CH_3CHCO$), 3.92 (bs,7H), 5.26 (bs,3H), 5.80 (d,J16Hz,OCOCH = CH), 5.90 (bs,6H), 6.90 (dd,J9 and 16Hz,OCOCH = CH), 7.1-7.3 (aromatic protons), analytical HPLC retention time 16.1min (spherisorb ODS-2, solvent acetonitrile : water 45:55 containing 0.15ml/L sulphuric acid), and Isomer 2 (136mg), δ($CDCl_3$) includes 0.75-0.95 (m,$CH_3$), 0.95-1.2 (m,$CH_3$), 2.85-3.05 (m,$CH_3CHCO$), 4.05 (bs,7H), 5.28 (bs,3H), 5.80 (d,J16Hz,OCOCH = CH), 5.93 (bs,6H), 6.88 (dd,J9 and 16Hz, OCOCH = CH), 7.1-7.3 (aromatic protons); analytical HPLC retention time 15.6min (spherisorb ODS-2,solvent acetonitrile: water 45:55 containing 0.15ml/L sulphuric acid).

Example 7

[1S-[1α[3R*(S*),4R*,5S*],3α,4β,5α,6α(2E,4R*,6R*),7β]] and [1S-[1α[3R*(S*),4S*,5S*],3α,4β,5α,6α(2E,4R*,6R*)-,7β]] 1-(4-Hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 1 and isomer 2

A solution of Example 6, isomer 1 (48mg) and sodium bicarbonate (36mg) in water (3ml) was treated with sodium borohydride (3mg) and stirred at 21°C. After 1.25h further sodium borohydride (3mg) was added and after a further 1h the solution was partitioned between ethyl acetate (10ml) and 2M-hydrochloric acid (10ml). The aqueous phase was extracted with ethyl acetate (2x5ml). Combined organic extracts were washed with water and brine (2x10ml each), dried ($MgSO_4$) and evaporated to a solid. The epimers were separated by preparative HPLC using a Spherisorb ODS-2 (2x25cm) column eluting with acetonitrile:water 60:40 containing sulphuric acid (0.15ml/L), flow rate 10ml/min. The required fractions were combined, partially evaporated under reduced pressure, and extracted with ethyl acetate. The extracts were dried and evaporated to give the title compounds as white solids. Isomer 1(11mg), δ ($CD_3OD$) includes 0.8-0.95-(m,$CH_3$), 0.95-1.1(m,$CH_3$), 3.20(t,J6Hz,CHOH), 4.04(d,J2Hz,7H), 5.25(s,3H), 5.80(d,J16Hz,OCOCH = CH), 6.30(d,J2Hz,6H), 6.84(dd,J9 and 16Hz,OCOCH = CH), 7.1-7.3(aromatic protons); analytical HPLC, retention time 2.6min (Spherisorb ODS-2, solvent acetonitrile:water 55:45 containing 0.15ml/L sulphuric acid), and Isomer 2 (7mg), δ ($CD_3OD$) includes 0.75-0.95(m,$CH_3$), 0.95-1.1(m,$CH_3$), 3.18(t,J6Hz,CHOH), 4.11-(d,J2Hz,7H), 5.25(s,3H), 5.78(d,J16Hz,OCOCH = CH), 6.30(d,J2Hz,6H), 6.82(dd,J9 and 16Hz,OCOCH = CH), 7.1-7.3(aromatic protons); analytical HPLC retention time 3.95 min (Spherisorb ODS-2, solvent acetonitrile:water 55:45 containing 0.15ml/L sulphuric acid).

Example 8

[1S-[1α[3R*(S*),4R*(S*),5S*],3α,4β,5α,6α(2E,4R*,6R*),7β]] 1-(4-Hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 3 and isomer 4

A part solution/part suspension of Example 6 isomer 2(51mg) in saturated sodium bicarbonate solution (5ml) was treated with sodium borohydride (3mg) and stirred at 21°C. After 1h further sodium borohydride (3mg) was added and after another hour the solution was partitioned between ethyl acetate (25ml) and 2M-hydrochloric acid (25ml). The aqueous phase was extracted with further ethyl acetate (2x10ml). Combined extracts were washed with water and brine (2x15ml each), dried ($MgSO_4$) and evaporated to a foam. This was combined with material prepared similarly (42mg) and the epimers were separated by preparative HPLC using a Dynamax $C_{18}$ (2x25cm) column eluting with acetonitrile:water 60:40 containing sulphuric acid (0.15ml/L), flow rate 10ml/min. The required fractions were combined, partially evaporated under reduced pressure, and extracted with ethyl acetate. The extracts were dried and evaporated to give the title compounds as white solids. Isomer 3 (20mg), δ ($CD_3OD$) includes 0.7-1.1(m,$CH_3$), 4.10(d,J2Hz,7H), 5.27-(s,3H), 5.79(d,J16Hz,OCOCH=CH), 6.32(d,J2Hz,6H), 6.82(dd,J9 and 16Hz,OCOCH=CH), 7.1-7.3(aromatic protons); analytical HPLC retention time 3.5min (Spherisorb ODS-2, solvent acetonitrile:water 55:45 containing 0.15ml/L sulphuric acid), and Isomer 4 (30mg), δ ($CD_3OD$) includes 0.75-1.0(m,$CH_3$), 1.02(d,J6Hz,$CH_3$), 3.14(dd,J2 and 9Hz,CHOH), 4.10(d,J2H,7H), 5.22(s,3H), 5.78(d,J16Hz,OCOCH=CH), 6.30(d,J2Hz,6H), 6.82-(dd,J9 and 16Hz,OCOCH=CH), 7.1-7.3(aromatic protons); analytical HPLC retention time 3.9min (Spherisorb ODS-2, solvent acetonitrile:water 55:45 containing 0.15ml/L sulphuric acid).

Example 9

[1S-[1α[(E),5R*],3α,4β,5α,6α(2E,4R*,6R*),7β]] 1-(3,5-Dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate)

Intermediate 11 (237mg) was dissolved in 3.1M HCl/dioxan (1.2ml) and the solution was stood at room temperature for 18h. The solution was evaporated and azeotroped several times with ether to give a foam. This was treated with 6.8M HCl/dioxan (0.64ml) and the resulting solution was stood at room temperature for 18h. The solution was evaporated and azeotroped several times with ether to give a foam. Preparative hplc Sphersorb ODS-2 column, 63%(95:5 MeCN:$H_2O$+0.15ml conc. $H_2SO_4$/litre) 37% $H_2O$ + 0.15ml conc. $H_2SO_4$/litre fow rate 15ml/min showed three major components to be present. Fractions which contained the first of these to be eluted were combined and the acetonitrile evaporated under reduced pressure at 30°C. The aqueous fraction was extracted with dichloromethane (4x50ml) and the organic extracts were combined and dried ($MgSO_4$). Evaporation of the solvent gave the title comund (27mg) as a cream coloured solid, δ -(DMSO-$d_6$) includes 0.91(d,J=7Hz,CH=CH.CHMe), 1.55(s,CH=C(Me)$CH_2$), 3.86(bd,J=5Hz,7-H), 4.94(s,3-H), 5.22(bt,J=6Hz,$CH_2$.CH=CMe), 5.55(d,J=15Hz,CO.CH=CH), 6.64-(dd,J=15Hz,7.5Hz,CO.CH=CH.CHMe), 7.11-7.31(m,aromatic protons); preparative HPLC, retention time 21.3min [Spherisorb ODS-2, solvent 63% (95:5) acetonitrile:water + 0.15ml conc. $H_2SO_4$/litre) 37% ($H_2O$+0.15ml conc. $H_2SO_4$/litre), flow rate 15ml/min.]

Example 10

[1S-[1α(3R*S*,5S*),3α,4β,5α,6α(2E,4R*,6R*),7β]] 1-(3-Ethyl-5-methyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate)

Intermediate 12 (139mg) was dissolved in trifluoroacetic acid (4:1ml) and anisole (1.02ml) and the solution was stood at room temperature for 90 min. The solution was evaporated and the residue was partitioned between 2% aqueous potassium bicarbonate solution (70ml) and ether (30ml). The organic phase was separated and washed with 2% aqueous potassium bicarbonate solution (30ml). The aqueous phases were combined and partitioned with ethyl acetate (20ml). The pH of the aqueous phase was adjusted to pH1 with concentrated hydrochloric acid and it was then partitioned with ethyl acetate (100ml). The organic phase was dried ($MgSO_4$) and evaporated to give a solid.

The previous organic extracts were combined and evaporated and the residue suspended in acetonitrile:water (1:3) and loaded onto a Whatman Partisil Prep 40 ODS-3 75Å silica gel column which was eluted with water:acetonitrile (3:1). Appropriate fractions were combined and the acetonitile was evaporated. The aqueous phase was freeze dried to give a solid. This was combined with the previously isolated solid and purified by preparative hplc Spherisorb ODS-2 column, eluted with 59% (MeCN:$H_2O$ + 2ml conc. $H_2SO_4$/litre) and 41% ($H_2O$ + 2ml conc. $H_2SO_4$/litre), flow rate 15ml/min. Appropriate fractions were combined and the acetonitrile was evaporated. The residual cloudy aqueous phase was extracted with ethyl acetate (3x200ml) and the organic extracts were combined, dried ($MgSO_4$) and evaporated. The residue was partially dissolved in water and subjected to freeze-drying to give the title compound (24mg) as a pale brown solid; δ (DMSO-$d_6$) includes 0.60(t,J = 6Hz,C( = 0)CH.$CH_2CH_3$), 4.97(s,3-H), 5.77-(d,J = 13Hz,CH = CH.CHMe), 6.18(bs,6-H), 6.72(dd,J = 13Hz,7Hz,CH = CH.CHMe), 7.11-7.31(m,aromatic protons); analytical HPLC, retention time 19.07 min [Spherisorb ODS-2, solvent 48% (95:5 acetonitrile:water + 0.15ml conc. $H_2SO_4$/litre) and 52% ($H_2O$ + 0.15ml conc. $H_2SO_4$/litre, flow rate 1.5ml/min)].

Example 11

[1S-[1α[(E),4R*,5S*],3α,4β,5α,6α(4S*,6R*),7β]] 1-(4-Acetyloxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyloctanoate)

A solution of freeze dried Compound A of Intermediate 1 (1g) in ethyl acetate (30ml) was hydrogenated for 1h using 10% Pd/C (120mg) as catalyst. The catalyst was removed by filtration through Kieselguhr and the filtrate was evaporated to dryness to give a gum.

Four 20mg portions of the gum were dissolved in acetonitrile/water 1:1 (2ml) and loaded onto a preparative HPLC column (20 mm x 250 mm) of Spherisorb ODS-2. The column was developed with acetonitrile/water 3:2 containing sulphuric acid (0.15 ml/L), flow rate 10 ml/min. Appropriate fractions from each run were combined, the acetonitrile was removed by evaporation under reduced pressure (bath temperature <40°C) and the aqueous solutions were extracted with ethyl acetate (x3), washed with water (x2), dried ($MgSO_4$) and evaporated to dryness. The first component eluted was the title compound (4mg); δ ($CD_3OD$) includes 0.80 - 0.90 (m, $CH_3$ 9 protons), 1.28 (s, -C($CH_3$ = CH), 2.06 (s, $OCOCH_3$, 4.07 (s, broad 7H), 5.25 (s, 3H), 5.61 (t, 12.5 Hz, $CH_2$CH), 6.23 (broad s, 6H), 7.3 (m, aromatic protons); analytical HPLC retention time 6.7 min. (Spherisorb ODS C-2, solvent acetonitrile - water 55:45 containing 0.15 ml sulphuric acid/L).

Example 12

[1S-[1α(3R*S*,4S*,5S*),3α,4β,5α,6α(4S*,6R*),7β]] 1-(4-Acetyloxy-3,5-dimethyl-6-phenylhexyl)-4,6,7-trihydroxy-2,8-dioxabicyclo[3.2.1]octane-3,4,5-tricarboxylic acid, 6-(4,6-dimethyloctanoate) [Compound 3] and [1S-[1α-(3R*S*,5R*), 3α,4β,5α,6α(4S*,6R*),7β]] 1-(3,5-dimethyl-6-phenylhexyl)-4,6,7-trihydroxy-2,8-dioxabicyclo[3.2.1]-octane-3,4,5-tricarboxylic acid, 6-(4,6-dimethyloctanoate) [Compound 4]

A solution of freeze dried Compound A of Intermediate 1 (300mg) was hydrogenated for 6 days using 10% Pd/C (150 mg) as catalyst. The catalyst was removed by filtration through Kieselguhr and the filtrate was evaporated to give a gum. Four portions (20 mg, 80 mg, 70 mg, 70 mg) of the gum were dissolved in acetonitrile/water 2:1 and loaded onto a preparative HPLC column (20 mm x 50 mm) of Spherisorb ODS-2. The column was developed each time with acetonitrile/water 65:35 containing sulphuric acid (0.15 ml/L), flow rate 10 ml/min. Appropriate fractions from each run were combined and the acetonitrile was removed by evaporation under reduced pressure (bath temperature <40°C). The aqueous solutions were extracted with ethyl acetate (x3), washed with water (x2), dried ($MgSO_4$) and evaporated to dryness. The first component eluted from the column was title Compound 3 (25mg); δ (DMSO-$d_6$) includes 0.75-0.90(m, $CH_3$, 2.05 (s, $OCOCH_3$), 4.96 and 4.97 (2s, 3H), 6.15 (broad s, 6H), 7.10 - 7.35 (aromatic protons); approximate molecular weight 694.78; -FAB mass spectrometry [M-H]-693; -FAB mass spectrometry [M-$CO_2$H]- 649. The second compound eluted from the column was title Compound 4 (40mg); δ ($CD_3OD$) includes 0.90 - 0.80 (m, $CH_3$), 4.02 (m, 7H), 5.23 (s, 3H), 6.25 (broad s, 6H), 7.1 - 7.4 (aromatic protons); approximate molecular weight 636.75; [M + H] + 637.74.

Example 13

[1S-[1α(3R*S*,4S*,5S*),3α,4β,5α,6α(4S*,6R*),7β]] 1-(3,5-Dimethyl-4-hydroxy-6-phenylhexyl)-4,6,7-trihydroxy-2,8-dioxabicyclo[3.2.1]octane-3,4,5-tricarboxylic acid, 6-(4,6-dimethyloctanoate)

A solution of freeze dried Compound B of Intermediate 1 in ethyl acetate (30ml) was hydrogenated for 3 days using 10% Pd/C (150mg) as catalyst. The catalyst was removed by filtration through Kieselguhr and the filtrate was evaporated to dryness to give a gum. Four 57mg portions of the gum were dissolved in acetonitrile/water 1:1 (1ml) and loaded onto a preparative HPLC column of Spherisorb ODS-2 (20mmx250mm). The column was eluted with acetonitrile-water (50:50) followed by acetonitrile-water (55:45) and acetonitrile-water (60:40), all containing sulphuric acid (0.15ml/L). Appropriate fractions were combined, the acetonitrile was removed by evaporation under reduced presure (bath temp <40$^0$C) and the aqueous solutions were extracted with ethyl acetate (x3), washed with water (x2), dried ($MgSO_4$) and evaporated to dryness to give the title compound (35mg); δ(DMSOd$_6$) includes 0.8-1.0(m,$CH_3$), 5.0(s,3H), 6.18 (broad singlet,6H), 7.15-7.35 (aromatic protons); approximate molecular weight 652.75; -FAB mass spectrometry [M-H]- 651; -FAB mass spectrometry [M-$CO_2$H]- 607.

Example 14

[1S-[1α[(E),4R*,5S*],3α,4β,5α,6α(4S*,6R*),7β]] 1-(3,5-Dimethyl-4-hydroxy-6-phenyl-2-hexenyl)-4,6,7-trihydroxy-2,8-dioxabicyclo[3.2.1]octane-3,4,5-tricarboxylic acid, 6-(4,6-dimethyloctanoate)

A solution of freeze dried Compound B of Intermediate 1 (250mg) in ethyl alcohol (25ml) containing triethylamine (1.25ml) was added to a suspension of Pd-C (10%,100mg) suspended in dichloromethane (2.5ml). The reaction mixture was stirred with hydrogen for 3h, the reaction mixture was acidified with 2N hydrochloric acid and the catalyst removed by filtration. The filtrate was evaporated to low volume, ethyl acetate (100ml) was added and the solution was washed with water (x3), dried $MgSO_4$ and evaporated to dryness. Four 50mg portions of the resultant were dissolved in acetonitrile/water (1:1) containing sulphuric acid (0.15ml/L) and loaded onto a preparative HPLC column of Sphenisorb ODS-2 (20mmx250mm). The column was eluted with acetonitrile-water (1:1) containing sulphuric acid (0.15ml/L). Appropriate fractions were combined, the acetonitrile was removed by evaporation under reduced pressure (bath temp <40$^0$C) and the aqueous solutions were extracted with ethyl acetate (x3), washed with water (x2), dried ($MgSO_4$) and evaporated to dryness to give the title compound (34mg); δ(DMSO-d$_6$) values include 0.65-0.88(m $CH_3$), 1.58(s,$CH_3$), 3.91(broad s,7H), 5.0 (s,3H), 5.51 (t,13H$_3$,CH-$CH_2$-), 6.17(broad s,6H), 7.15-7.32-(m,aromatic protons); approximate molecular weight 650.73; -FAB mass spectrometry [M-H]- 649; - FAB mass spectrometry [M-$CO_2$H]- 605.

Example 15

[1S-[1α[(Z/E),5S*],3α,4β,5α,6α(4S*6R*),7β]] 1-(3,5-Dimethyl-6-phenyl-3-hexenyl)-4,6,7-trihydroxy-2,8-dioxabicyclo[3.2.1]octane-3,4,5-tricarboxylic acid, 6-(4,6-dimethyloctanoate)

Intermediate 13 (0.635g) was dissolved in tetrahydrofuran (8ml) and water (32ml) and stirred with zinc (1.64g) at room temperature. Hydrochloric acid (2N) was added dropwise over a 5 minute period until a pH1 was obtained. The mixture was then stirred for an hour and filtered through Kieselguhr. The bed was washed with ethyl acetate (200ml) and the combined filtrates washed with 2N hydrochloric acid (3x25ml), dried and evaporated to yield a gum. A portion (100mg) of this mixture was purified by preparative h.p.l.c (Spherisorb ODS-2 (20x250mm)) eluting with acetonitrile : water (70:30) containing sulphuric acid (0.15ml/l) (flow rate 15ml/min). Appropriate fractions were combined from each run, the acetonitrile was removed by evaporation under reduced pressure (bath temp <40$^0$C) and the aqueous solution was extracted with ethyl acetate (x3), dried and evaporated to yield a gum. This was freeze dried from aqueous dioxan to give the title compound (76mg) as a white solid; δ($CD_3OD$) includes 1.66 (d,J=2Hz,$CH_2C(CH_3)$=CH.$CHCH_3$), 2.74-2.89 (m,C($CH_3$)=CH.CH.$CH_3$), 3.95 and 4.04 (2d,7H,E/Z isomers), 4.97 and 5.05 (2bd,J=10Hz,C($CH_3$)-=CH.$CHCH_3$, E/Z isomers), 5.23 (s,3-H), 6.25 and 6.27 (2d,J=ca.2Hz,6-H), 7.04-7.27 (m, aromatic protons); approximate molecular weight 634; -FAB mass spectrometry [M-H]- 633.

Example 16

[1S-[1α(3R*(S*),5S*),3α,4β,5α,6α(4S*,6R*),7β]] 1-(3,5-Dimethyl-4-oxo-6-phenylhexyl)-4,6,7-trihydroxy-2,8-dioxabicyclo[3.2.1]octane-3,4,5-tricarboxylic acid, 6-(4,6-dimethyloctanoate), 3,4,5-tripotassium salt, isomers 1 and 2

A solution of Intermediate 15 (396mg) in tetrahydrofuran (20ml) and water (8ml) was treated with zinc

dust (1g) followed by dropwise addition of hydrochloric acid (2M; 4ml) until the pH of the solution was stable at 1. The excess zinc was collected by filtration, washed with ethyl acetate and the combined filtrate and washings were washed with 2MHCl (x3), brine, dried ($MgSO_4$) and evaporated to give of a gum. The residue was purified by reverse-phase HPLC (1 inch Spherisorb ODS-2, flow rate 10ml/min eluting with 55% acetonitrile-water containing 0.15ml concentrated sulphuric acid/litre). Appropriate fractions from each run were combined, the acetonitrite was removed by evaporation under reduced pressure (bath temperature <40°C) and the aqueous solutions were extracted with ethyl acetate (x3), washed with water (x2) dried ($MgSO_4$) and evaporated to dryness.

The first component eluted (analytical HPLC retention time 15.5min, Spherisorb ODS-2, solvent 45% acetonitrite-water containing 0.15ml concentrated sulphuric acid/L) (41mg) in dioxan (1ml) was treated with potassium hydroxide solution (0.1M; 1.89ml) at 20°C. The solution was diluted with water(10ml) and freeze-dried to give isomer 1 of the title compound (48mg) as a white solid; $\delta(D_2O)$ includes 0.79-0.89(m,$CH_3$), 1.05 and 1.09(2d,J7Hz,$CH_3CHCO$), 2.40(t,J8Hz,$OCOCH_2CH_2$), 3.83(broad singlet,7H), 4.87(s,3H), 6.13(broad singlet,6H), 7.23-7.4(aromatic); -FAB mass spectrometry [M-H]- 763.

The second component eluted (analytical HPLC retention time 17.1min) (35mg) in dioxan (2ml) was treated with potassium hydroxide solution (0.1M; 1.61ml) at 20°C. The solution was diluted with water (10ml) and freeze-dried to give isomer 2 of the title compound (40mg) as a white solid; $\delta(D_2O)$ includes 0.75-0.85-(m,$CH_3$), 1.10(d,J7Hz,$CH_3CHCO$), 2.39(t,J7Hz,$OCOCH_2CH_2$), 3.95(broad singlet,7H), 4.88(s,3H), 6.13(broad singlet,6H), 7.2-7.4(aromatic protons); - FAB mass spectrometry [M-H]- 763.

Example 17

[1S-[1α(4R*,5S*),3α,4β,5α,6α(4S*,6R*),7β]] 1-(4-Hydroxy-5-methyl-3-oxo-6-phenylhexyl)-4,6,7-trihydroxy-2,8-dioxobicyclo[3.2.1]octane-3,4,5-tricarboxylic acid, 6-(4,6-dimethyloctanoate)

A solution of Intermediate 17 (123mg) was hydrogenated over 10% palladium-on-carbon (20mg) in ethanol (10ml) for 1.33h. The catalyst was collected by filtration and washed with ethanol. The combined filtrate and washings were evaporated to dryness and the residue was partitioned between ether (30ml) and water (30ml) containing 0.88 ammonia (1ml). The aqueous layer was extracted with ether (x3), acidified to pH 1 with 2MHCl, and extracted with ethyl acetate. The organic solution was washed with water (x2), dried ($MgSO_4$), evaporated and the residue was freeze-dried from water (15ml) to give the title compound (65mg) as a white solid; $\delta(CD_3OD)$ includes 0.75-0.9(m,$CH_3$), 4.0(d,J2Hz,7H), 4.09(d,J3Hz,CH(OH)CO), 5.22(s,3H), 6.26(d,J2Hz,6H), 7.13-7.30(aromatic protons); approximate molecular weight 652.7; -FAB mass spectrometry [M-H]-651;-FAB mass spectrometry [M-$CO_2$H]- 607.

Example 18

[1S-[1α(3R*S*,4R*,5S*),3α,4β,5α,6α(4S*,6R*),7β]] 1-(3,4-Dihydroxy-5-methyl-6-phenylhexyl)-4,6,7-trihydroxy-2,8-dioxabicyclo[3.2.1]octane-3,4,5-tricarboxylic acid, 6-(4,6-dimethyloctanoate)

A solution of Intermediate 18 (52mg) in ethanol (6ml) was hydrogenated over 10% palladium-on-carbon (6mg) for 1h. The catalyst was collected by filtration and washed with ethanol. The combined filtrate and washings were evaporated under reduced pressure. The residue was partitioned between ether (15ml) and water (15ml) containing 0.88 ammonia (1ml). The aqueous layer was extracted with ether (x3), acidified with 2MHCl and extracted with ethyl acetate. The organic solution was washed with brine, dried ($MgSO_4$) and purified by reverse-phase HPLC (1 inch Spherisorb ODS-2, flow rate 10ml/min eluting with 50% acetonitrile-water containing 0.15ml concentrated sulphuric acid/L). Appropriate fractions were combined, the acetonitrile was removed by evaporation under reduced pressure (bath temperature <40°C) and the aqueous solution was extracted with ethyl acetate (x3), washed with brine, dried ($MgSO_4$) and evaporated to dryness. The residue was freeze-dried to give the title compound (24mg); $\delta(CD_3OD)$ includes 0.8-0.9(m,$CH_3$), 4.02-(broad singlet,7H), 5.23(s,3H), 6.27(broad singlet,6H), 7.1-7.38(m,aromatic protons); approximate molecular weight 654.7; -FAB mass spectrometry [M-H]- 653;-FAB mass spectrometry [M-$CO_2$H]- 609.

Example 19

[1S-[1α,3α,4β,5α,6α(4S*,6R*),7β]] 1-(4-Oxo-6-phenylhexyl)-4,6,7-trihydroxy-2,8-dioxabicyclo[3.2.1]octane-3,4,5-tricarboxylic acid, 6-(4,6-dimethyloctanoate)

A solution of Intermediate 21 (206mg) in ethanol (30ml) was hydrogenated over 10% palladium-on-carbon (30mg) for 3h. The catalyst was collected by filtration and washed with ethanol. The combined filtrate and washings were evaporated under reduced pressure. The residue was partitioned between ether (30ml) containing 0.88 ammonia (2ml). The aqueous layer was extracted with ether (x3), acidified with 2M HCl and extracted with ethyl acetate. The organic solution was washed with brine, dried ($MgSO_4$) and purified by reverse-phase HPLC (1 inch Spherisorb ODS-2, flow rate 15ml/min eluting with 50% acetonitrile-water containing 0.15ml concentrated sulphuric acid/L). Appropriate fractions were combined, the acetonitrile was removed by evaporation under reduced pressure and the aqueous solution was extracted with ethyl acetate (x3), washed with brine, dried and evaporated to dryness to give the title compound (90mg); δ-($CD_3OD$) includes 0.82-0.91 (m,$CH_3$), 2.30(t,J=7Hz,$CH_2CO_2$), 4.02 (d,J=2Hz,7-H), 5.21 (s,3-H), 6.28 (br.s,6-H), 7.12-7.28 ($C_6H_5$); approximate molecular weight 622.6;-FAB mass spectrometry [M-H]- 621.

Example 20

[1S-[1α(5R*S*),3α,4β,5α,6α(4S*,6R*),7β]] 1-(5-Methyl-4-oxo-6-phenylhexyl)-4,6,7-trihydroxy-2,8-dioxabicyclo-[3.2.1]octane-3,4,5-tricarboxylic acid, 6-(4,6-dimethyloctanoate)

A solution of Intermediate 22 (288mg) in ethanol (30ml) was hydrogenated over 10% palladium-on-carbon (50mg) for 18h. The catalyst was collected by filtration and washed with ethanol. The combined filtrate and washings were evaporated under reduced pressure. The residue was partitioned between ether (30ml) and water (40ml) containing 0.88 ammonia (2ml). The aqueous layer was extracted with ether (x3), acidified with 2M HCl, and extracted with ethyl acetate (x4). The organic phase was washed with brine, dried and purified by reverse-phase HPLC (1 inch Spherisorb ODS-2 flow rate 15ml/min eluting with 50% increasing to 90% acetonitrile-water containing 0.15ml concentrated sulphuric acid/L). Appropriate fractions were combined, the acetonitrile was removed by evaporation under reduced pressure and the aqueous solution was extracted with ethyl acetate, washed with brine, dried, and evaporated to dryness to give the title compound (104.8mg); δ($CD_3OD$) includes 0.82-0.92 (m,$CH_3$), 1.05 (d,J=7Hz,$COCHCH_3$), 2.30 (t,J=7Hz,$CH_2CO_2$), 4.03 (br.s,7-H), 5.23 (s,3-H), 6.26 (br s,6-H), 7.12-7.30($C_6H_5$); approximate molecular weight 636.7; -FAB mass spectrometry [M-H]-635.

Example 21

[1S-[1α[3R*(S*)],3α,4β,5α,6α(4S*,6R*),7β]] 1-(3-Methyl-4-oxo-6-phenylhexyl)-4,6,7-trihydroxy-2,8-dioxabicyclo[3.2.1]octane-3,4,5-tricarboxylic acid, 6-(4,6-dimethyloctanoate)

A solution of Intermediate 23 (107mg) in ethanol (20ml) was stirred with 10% palladium on charcoal catalyst (70mg) and hydrogenated for 24h at room temperature and atmospheric pressure. The catalyst was removed by filtration and the filter pad was washed with ethanol. The washings and filtrate were combined and evaporated and the residue was dissolved in water (30ml) containing 0.88 ammonia solution (2ml) and partitioned with diethyl ether (8x30ml). The aqueous phase was acidified to pH1 with 2N hydrochloric acid and extracted with ethyl acetate (2x80ml). The organic extracts were combined, dried ($MgSO_4$) and evaporated to give the title compound (60mg); δ ($CD_3OD$) includes 1.05 and 1.02(2d,J=2.5Hz,$CH_2CH(CH_3)$-C(=0)), 2.30(t,J=7.5Hz,$OC(0)CH_2CH_2$),2.67-2.87(m,$CH_2CH_2CH(CH_3)C(0)$,$CH_2CH_2CH(CH_3)C(0)$ and $CH_2CH_2CH(CH_3)C(0)$, 4.00(d,J<2Hz,7-H), 5.22(s,3-H), 6.24(d,J<2Hz,6-H),7.08-7.28(m,aromatic protons); approximate molecular weight 636.7; thermospray [M-H]- 635.

Example 22

[1S-[1α,3α,4β,5α,6α(4S*,6R*),7β]] 1-(4-Oxo-7-phenylheptyl)-4,6,7-trihydroxy-2,8-dioxabicyclo[3.2.1]octane-3,4,5-tricarboxylic acid, 6-(4,6-dimethyloctanoate)

A solution of Intermediate 24 (177mg) in ethanol (25ml) was hydrogenated over 10% palladium-on-carbon (25mg) for 3h. The catalyst was collected by filtration and washed with ethanol. The combined filtrate and washings were evaporated under reduced pressure. The residue was partitioned between ether (30ml) and water (40ml) containing 0.88 ammonia (3ml). The aqueous layer was extracted with ether (x3), acidified with 2M HCl, and extracted with ethyl acetate. The organic phase was washed with brine, dried and purified by reverse-phase HPLC (1 inch Spherisorb ODS-2 flow rate 15ml/min) eluting with 60% increasing to 95% acetonitrile-water containing 0.15ml concentrated sulphuric acid/L. Appropriate fractions

were combined, the acetonitrile was removed by evaporation under reduced pressure and the aqueous solution was extracted with ethyl acetate, washed with brine, dried and evaporated to dryness to give the title compound (68mg); δ($CD_3OD$) includes 0.8-0.9 (m,$CH_3$), 2.29 (t,J = 7Hz,$CH_2CO_2$), 4.04 (d,J = 2Hz,7-OH), 5.22 (s,3-H), 6.24 (d,J = 2Hz,6-H), 7.09-7.29 (m,$C_6H_5$); analytical HPLC retention time 4.0min (Spherisorb ODS-2, solvent 50→90% acetonitrile - $H_2O$ containing 0.15ml concentrated sulphuric acid/L).

Example 23

[1S-[1α,3α,4β,5α,6α(4S*,6R*),7β]] 1-(4-Oxo-7-phenylheptyl)-4,6,7-trihydroxy-2,8-dioxabicyclo[3.2.1]octane-3,4,5-tricarboxylic acid, 6-(4,6-dimethyloctanoate), 3,4,5-tripotassium salt

A solution of Example 22 (63mg) in acetone (2ml) was added to a solution of potassium bicarbonate (30mg) in water(15ml) dropwise with stirring and warming until the mixture became homogeneous. The solution was then freeze-dried to give the title compound (74mg); δ($D_2O$) includes 0.75-0.85 (m,$CH_3$), 2.39 (t,J = 7Hz,$CH_2CO_2$), 4.00 (d,J = 2Hz, 7-OH), 4.89 (s,3-H), 6.15 (d,J = 2Hz,6-H), 7.2-7.4. (m,$C_6H_5$); analysis found: C,45.7;H,5.63, $C_{32}H_{41}K_3O_{13}.5H_2O$(841.1) requires C,45.7;H6.1%; analytical HPLC retention time 3.82 min (Spherisorb ODS-2, solvent 50→90% acetonitrile - $H_2O$ containing 0.15ml concentrated sulphuric acid/L).

Example 24

[1S-[1α(4R*,5S*),3α,4β,5α,6α(2E,4R*,6R*),7β]] 7-Acetyloxy-1-(4-acetyloxy-5-methyl-3-methylene-6-phenylhexyl)-4,6-dihydroxy-2,8-dioxabicyclo[3.2.1]octane-3,4,5-tricarboxylic acid, 6-(4,6-dimethyl-2-octenoate)

Intermediate 32 (0.050g) was treated with a solution of hydrogen chloride in dioxan (6.9M, 1ml) and the resulting solution left to stand at room temperature for 19h then evaporated to give a gum. This material was chromatographed using a 'Baker'-10 SPE octadecyl ($C_{18}$) disposable column eluting with acetonitrile-water (4:1) and appropriate fractions were combined and evaporated to give a white solid. This material was further purified by preparative HPLC chromatography using a Dynamax $C_{18}$ column eluting with acetonitrile-0.15% v/v aqueous trifluoroacetic acid (3:2) and appropriate fractions were combined and freeze-dried to give the title compound (0.005g) as a white solid; δ ($CD_3OD$) values include 2.09 and 2.17 (2s,$CH_3CO_2$), 2.69 (dd,J14 and 6Hz,CHPh), 5.08 and 5.20 (2d,J5 and J2Hz,$CH_3CO_2CH$), 5.80 (d,J16Hz,$O_2CCH$ = CH), 6.47 (bs,$CHO_2CCH$ = CH), 6.83 (dd, J16 and 8Hz,$O_2CCH$ = CH); analytical HPLC, retention time 11.42 min (Dynamax C18, solvent 60% acetonitrile-water containing 0.15ml trifluoroacetic acid/L).

Example 25

[1S-[1α(4R*,5S*),3α,4β,5α,6α(2E,4R*,6R*),7β]] 1-(4-Acetyloxy-5-methyl-3-methylene-6-phenylhexyl)-4,7-diacetyloxy-6-hydroxy-2,8-dioxabicyclo[3.2.1]octane-3,4,5-tricarboxylic acid, 6-(4,6-dimethyl-2-octenoate)

A solution of Intermediate 34 (160mg) in tetrahydrofuran (5ml) and water (2ml) was stirred with zinc dust (380mg) at 21°C and treated dropwise with 2M-hydrochloric acid (2ml) over 20 minutes. Further acid (1.5ml) was added and after an additional 10 minutes the mixture was filtered. The retained solid was washed with ethyl acetate (25ml). Combined filtrate and washings were diluted with ethyl acetate (25ml) and 2M-hydrochloric acid (25ml). The organic phase was washed with water and brine (2x25ml each), dried ($MgSO_4$) and evaporated to a solid. This was purified by preparative HPLC using a Spherisorb ODS-2 column (2x25cm) eluting with acetonitrile:water 65:35 containing sulphuric acid (0.15ml/L), flow rate 10ml/min. The required fractions were combined, partially evaporated under reduced pressure, and extracted with ethyl acetate. The extracts were dried and evaporated to the title compound as a pale yellow solid (70mg); δ ($CD_3OD$) includes 0.8-1.0 (m,$CH_3$), 1.05 (d,J6Hz,$CH_3CH$ = CH), 2.05,2.10 and 2.16 (s,$OCOCH_3$), 4.99 (d,J9Hz,C = $CH_2$), 5.02- 5.20 (3H,7H, CHOAc), 5.80 (dd,J16Hz,OCOCH = CH), 6.43 (d,J2Hz,6H), 6.86 (dd,J9 and 16Hz,OCOCH = CH), 7.1-7.3 (aromatic protons); approximate molecular weight 774.8; -FAB mass spectrometry $[M-H]^-$773; -FAB mass spectrometry $[M-CH_3CO]^-$731.

Example 26

[1S-[1α(4R*,5S*),3α,4β,5α,6α(2E,4R*,6R*),7β]] 1-(4-Acetyloxy-5-methyl-3-methylene-6-phenylhexyl)-4,6-dihydroxy-7-[(methoxycarbonyl)oxy]-2,8-dioxabicyclo[3.2.1]octane-3,4,5-tricarboxylic acid, 6-(4,6-dimethyl-2-octenoate).

To a solution of Intermediate 35 (359 mg) in formic acid (11 ml) was added water (2.4ml) with stirring. The resulting reaction mixture was stirred at 60°C for 1 hour, after which time it was separated between ethyl acetate (80ml) and water (30 ml). The organic phase was washed with saturated brine (2 x 30 ml) and dried over $MgSO_4$. The ethyl acetate was then removed under reduced pressure to yield a colourless oil. This was dissolved in 1:1 acetonitrile/water (40 ml) and loaded in 5 ml aliquots onto an HPLC column (250 mm x 20 mm id, Spherisorb ODS-2). The column was eluted with 40%-60% acetonitrile/water with 150 µl/L conc. $H_2SO_4$, flowrate 15 ml/min. Appropriate fractions from each run were combined and the acetonitrile removed under reduced pressure to leave an acidic, aqueous solution of the title compound. The aqueous solution was extracted with ethyl acetate (150 ml) and the organic phase washed with water (2 x 50 ml) and saturated brine (50 ml) before being dried over $MgSO_4$. The solvent was then removed under reduced pressure yielding a white foam. This foam was then dissolved in water (5 ml) and freeze-dried to give a white, solid sample of the title compound (159.6 mg); δ ($CD_3OD$) includes 0.8-0.93 (m, $CH_3$, 9 protons), 1.03 (d, 1 = 7 Hz, $CH_3$), 2.09 (s, $OCOCH_3$) 4.98,5.02 (2S, = $CH_2$), 5.07 (m, 3H, 7H), 5.10 (d, J = 5 Hz, CHOAc), 5.81 (d, J = 16 Hz, OCOCH = CH), 6.47 (s, broad, 6H), 6.86 (dd, J = 16 Hz, 8.5 Hz, CH = CHCH(Me)), 7.1-7.3 (m, aromatic protons);
Analysis Found: C, 56.66%; H,6.43%,
$C_{37}H_{48}O_{16}.2H_2O$ (784.82) requires C, 56.63%; H, 6.68%.
Approximate molecular weight 748.8; thermospray negative $[M-H]^-$747; $[M-CO_2H]^-$703; $[M-OCOCH_3]^-$689.

Example 27

[1S-[1α(4R*,5S*),3α,4β,5α,6α,7β]] 1-(4-Acetyloxy-5-methyl-3-methylene-6-phenylhexyl)-4,6-dihydroxy-7-[-(methoxycarbonyl)oxy]-2,8-dioxabicyclo[3.2.1]octane-3,4,5-tricarboxylic acid.

To a solution of Intermediate 36 (299.9 mg) in formic acid (10 ml) was added, water (2 ml) with vigorous stirring. The resulting reaction mixture was then stirred at room temperature for 2 hours. The reaction mixture was separated between ethyl acetate (75 ml) and water (50ml), and washed with both water (50ml) and saturated brine (50ml) before being dried over $MgSO_4$. The solvent was then removed under reduced pressure to yield a colourless gum. This was dissolved in 1:1 acetonitrile/water (40 ml) and loaded in 5 ml aliquots onto a preparative HPLC column (250 mm x 20 mm id, Spherisorb ODS-2). The column was eluted with 50%-80% acetonitrile/water with 150 µl/L conc. $H_2SO_4$, flow rate 15 ml/min. Appropriate fractions from each run were combined and the acetonitrile removed under reduced pressure to leave an acidic, aqueous solution of the title compound. The aqueous solution was extracted with ethyl acetate (100 ml) and the organic phase washed with water (50 ml) and brine (50 ml). The solution was then dried over $MgSO_4$ and the solvent removed under reduced pressure to yield a colourless gum. This gum was then suspended in water (7 ml) and freeze-dried to give the title compound as a white solid (74.4mg); δ ($CD_3OD$) includes 0.84 (d, J = 7 Hz, - $CH_3$), 2.11 (s, $OCOCH_3$),4.94 (s, 3H), 4.99, 5.03 (2s, = $CH_2$), 5.08 (d,J = 1.5 Hz, 7H), 5.11 (d, J = 4.5 Hz, CHOAc), 5.34 (s, broad, 6H), 7.12-7.31 (m, aromatic protons);
Analysis Found: C, 46.32%; H, 5.52%,
$C_{27}H_{32}O_{15}.3H_2O.0.5H_2SO_4$ (699.64), requires C, 46.35%; H, 5.62%.

Example 28

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-acetate [Compound 1] and 6,7-diacetate [Compound 2]

To a stirred solution of Intermediate 38 (0.70g) in dry dichloromethane (2ml) at 0°C, 4-N,N-dimethylaminopyridine (213mg) was added followed by acetyl bromide (64µl). The mixture was stirred at 0°C for 20min. Water (10ml) and ethyl acetate (10ml) were added, and after extraction the aqueous was re-extracted with ethyl acetate (2x10ml). The combined organics were dried and evaporated to give an oil which was subjected to flash chromatography (5% methanol in chloroform). Combination of the required fractions gave a yellow oil which was dissolved in dichloromethane (2ml) and stirred at 0°C. Trifluoroacetic acid (2ml) was added and after 16h at 3°C the volatiles were evaporated. The residue was partitioned between water (20ml) and ethyl acetate (20ml) and after separation the aqueous was re-extracted with ethyl acetate (2x20ml). The combined organics were washed (water 50ml, brine 50ml) and dried ($MgSO_4$). Evaporation of the solvent gave a yellow foam. This was purified by HPLC using acetonitrile-water mixtures

on a Spherisorb ODS-2 column (PLC 474). Appropriate fractions were combined and the acetonitrile evaporated. The aqueous was extracted with ethyl acetate (3 times), the combined organics dried ($MgSO_4$) and the solvent evaporated. The residues were taken up in water and lyophilised and dried over phosphorus pentoxide at 50°C in vacuo to give as white solids Compound 1 (21mg); δ($CD_3OD$) includes 0.85 (d,J = 7Hz,3H), 2.03 (3H,s,OAc), 2,10(3H,s,OAc), 4.03 (1H,d,J = 2Hz,7-H),4.98,5.01 (2H,2s, = $CH_2$), 5.08 (1H,d,J = 5Hz,CHOAc), 5.26 (1H,s,3H) 6.28 (1H,d,J = 2Hz,6-H), 7.12-7.30 (5H,m,Ph); approximate molecular weight 580.5; -FAB mass spectrometry $[M-H]^-$579; + FAB mass spectrometry $[M+Na]^+$601;
Analysis Found: C,52.84%,H,5.51%,
$C_{27}H_{32}O_4$. $2H_2O$(620.5) requires C,52.60%;H,5.89% and Compound 2 (85mg); δ ($CD_3OD$) includes 0.84 (3H,d,J = 7Hz,3H), 2.02 (3H,s,OAc), 2.10 (3H,s,OAc), 2.14 (3H,s,OAc), 4.96,5.00 (2H,2s, = $CH_2$), 5.04 (1H,s,3-H), 5.07 (1H,d,J = 6Hz,CHOAc), 5.18 (1H,d,J = 2Hz,7-H), 6.51 (1H,brs,6-H), 7.12-7.30 (5H,m,Ph); -FAB mass spectrometry $[M-H]^-$621; -FAB mass spectrometry $[M-CO_2H]^-$577; + FAB mass spectrometry $[M+Na]^+$643;

## Example 29

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6,7-bisbutanoate

To a stirred solution of Intermediate 38 (0.70g) in dichloromethane (2ml) at 0°C, 4-N,N-dimethylaminopyridine (213mg) was added followed by n-butyryl chloride (90μl). After 12h at 30°C, a further portion of n-butyryl chloride (30μl) was added and after a further 30min, water (20ml) was added. Extraction with ethyl acetate (3x20ml), drying of the combined organics ($MgSO_4$) and evaporation of the solvent gave a yellow oil which was subjected to flash chromatography (3%, methanol in chloroform). Combination of the required fractions gave a clear colourless oil which was dissolved in dichloromethane (2ml) at 0°C and trifluoroacetic acid (2ml) was added. The solution was stored at 3°C for 16h and the solvent removed to give a pale yellow oil which was purified by HPLC using acetonitrile water mixtures containing 150μl/L of sulphuric acid on a spherisorb ODS-2 column (PLC 476). Appropriate fractions was combined, the acetonitrile was removed by evaporation under reduced pressure and the aqueous was extracted with ethyl acetate (3 times), the combined organics dried ($MgSO_4$) and the solvent evaporated. The residue was taken up in water and lyophilised and dried over $P_2O_5$ at 50°C in vacuo to give the title compound (60mg) as a white solid; δ($CD_3OD$) includes 0.91 (3H,d,J = 7Hz,$CH_3$CH), 1.02 (3H,t,J = 7Hz, $CHCH_2$), 1.05 (3H,t,J = 7Hz,$CH_3CH_2$), 1.72 (4H,m,2x$CH_3CH_2$), 2.18 (3H,s,OAc), 5.05,5.09 (2H,2s, = $CH_2$), 5.15 (1H,d,J = 5Hz,CHOAc), 5.18 (1H,s,3-H), 5.27 (1H,d,J = 2Hz,7-H), 6.50 (1H,d,J = 2Hz,6-H), 7.19-7.38 (5H,m,Ph); -FAB mass spectrometry $[M-H]^-$677; -FAB mass spectrometry $[M-CO_2H]^-$633; approximate molecular weight 678.7;
Analysis Found: C,56.46%;H,6.27%;
$C_{33}H_{42}O_{15}$. $H_2O$ requires C,56.89%;H,6.37%.

## Example 30

[1S-[1α(4R*,5S*),3α,4β,5α,6α,7β]] 1-(4-Acetyloxy-5-methyl-3-methylene-6-phenylhexyl)4,6,7-trihydroxy-2,8-dioxabicyclo [3.2.1]octane-3,4,5-tricarboxylic acid, 6- and 7-decanoate

To a solution of Intermediate 38 (680mg) and 4-N,N-dimethylaminopyridine (160mg) in dichloromethane (1ml) at 0°C under dry $N_2$, n-decanoyl chloride (124mg) was added. After 20min, ethyl acetate (5ml) was added and the mixture was washed with water (5ml). The aqueous was re-extracted with ethyl acetate (5ml) and the combined organics washed with 2M hydrochloric acid (2x5ml) and brine (2x5ml). The organics were dried ($MgSO_4$) and reduced to a white foam which was subjected to flash chromatography (20% ethyl acetate, petrol). Combination of the required fractions gave a white solid which was suspended in a mixture of formic acid (4ml) and water (1ml) and stirred at 50-60°C for 4h. Water (5ml) was added and the mixture extracted with ethyl acetate (3x5ml). The organics were dried ($MgSO_4$) and the solvent evaporated to give an oil which was purified by HPLC in 55-85% acetonitrile, water mixtures containing 150μl/L of sulphuric acid, on a 1 in spherisorb ODS-2 column (PLC 489). Appropriate fraction were combined, the acetonitrile was removed by evaporation under reduced pressure and the aqueous was extracted with ethyl acetate (3 times), the combined organics dried ($MgSO_4$) and the solvent evaporated. The residues were taken up in water and lyophilised to give [1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-decanoate (25mg); δ-

($CD_3OD$) includes 0.86 (6H,m,2x$CH_3$), 1.30 (12H,brs,$CH_2$), 2.11 (3H,s,OAc), 4.02 (1H,d,J=2Hz,7-H), 4.98,5.03 (2H,2s,=$CH_2$), 5.08 (1H,d,J=5Hz,CHOAc), 5.25 (1H,s,3-H), 6.29 (1H,d,J=2Hz,6-H), 7.10-7.31 (5H,m,Ph); thermospray negative [M-H]$^-$691; thermospray negative [M-$CH_3CO_2H$]$^-$632;
Analysis Found: C, 59.00%;H,7.07%,
$C_{35}H_{48}O_{15}$.$H_2O$ requires C,59.14%,H,7.09%, and [1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 7-decanoate (25mg); δ($CD_3OD$) includes 0.85 (6H,m,2x$CH_3$), 1.30 (12H,brs,$CH_2$), 2.11 (3H,s,OAc), 4.98,5.03 (2H,2s,=$CH_2$), 5.03(1H,s,7-H), 5.11 (1H,d,J=5Hz,CHOAc), 5.25 (1H,d,J=2Hz,6-H), 5.31 (1H,s,3-H), 7.10-7.31 (5H,m,Ph); -FAB mass spectrometry [M-H]$^-$691, [M-$CO_2H$]$^-$647, [M-$CH_3CO_2$]$^-$633;
Analysis Found: C,59.86%; H,6.85%,
$C_{35}H_{48}O_{14}$. 0.5$H_2O$ requires C,59.90%;H,7.04%.

Example 31

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-octanoate

To a stirred solution of Intermediate 38 (900mg) in dry dichloromethane (3ml) at 0°C, 4-N,N-dimethylaminopyridine (282mg) was added, followed by n-octanoyl chloride (0.19ml). After 20min, water (20ml) and ethyl acetate (20ml) were added and the layers were separated. Extraction of the aqueous with ethyl acetate (3x20ml), drying of the combined organics ($MgSO_4$) and evaporation of the solvent gave a yellow oil which was subjected to flash chromatography (3% methanol in chloroform). Combination of the required fractions gave an oil which was dissolved in methylene chloride (0.5ml) at 0°C and trifluoroacetic acid (0.5ml) was added dropwise. The mixture was stirred for 45min at 0°C and kept at 3°C for 4h. Water was added and the volume reduced dryness. The residue was purified by HPLC using acetonitrile water mixtures containing 150μl/L of sulphuric acid, on a spherisorb ODS-2 column (PLC 464). Appropriate fractions were combined, the acetonitrile was removed by evaporation under reduced pressure and the aqueous extracted with ethyl acetate (3 times). The combined organics were dried ($MgSO_4$) and the solvent evaporated. The residue was taken up in water and lyophilised followed by drying over $P_2O_5$ at 50°C in vacuo to give the title compound (25mg) as a white solid; δ($CD_3OD$) includes 0.95 (6H,m,2x$CH_3$),4.00 (1H,s,7H),4.95,5.05 (2H,2s,=$CH_2$), 5.10 (1H,d,J=5Hz,CHOAc), 5.20 (1H,brs,3-H), 6.35 (1H,brs,6-H), 7.10-7.30 (5H,m,Ph); approximate molecular weight 664.7; -FAB mass spectrometry [M-H]$^-$664; -FAB mass spectrometry [M-CO$(CH_2)_6CH_3$]$^-$538.

Example 32

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6,7-bis-octanoate

A solution of Compound 1 of Intermediate 40 (300mg) in trifluoroacetic acid (9ml) and water (1ml) was stirred at 20°C for 30 minutes, then diluted with water (5ml), and evaporated to dryness, and residual solvents were removed by addition and evaporation of toluene (3x10ml). The residue was purified by preparative HPLC (Spherisorb ODS-2, development with 60 to 90% acetonitrile in water containing sulphuric acid ((0.15ml/L), flow rate 10ml/min). The appropriate fractions were combined, the acetonitrile evaporated off, and the residual aqueous solution (ca 20ml) extracted with ethyl acetate (2x50ml). The extract was washed with water, and brine (25ml of each), and dried (magnesium sulphate), and evaporated to dryness to give the title compound (45mg) as a colourless solid; δ($CD_3OD$) includes 0.8-0.9 (m,$CH_3$,9 protons), 1.2-1.4 (m,OCO$CH_2CH_2(CH_2)_4$Me), 1.55-1.7 (m,OCO$CH_2CH_2(CH_2)_4$Me), 2.10 (s,OCO$CH_3$), 5.00 (s), 5.03 (s), (=$CH_2$), 5.09 (d,J7hz,CHOAc), 5.11 (s,3H), 5.22 (d,J2Hz,7H), 6.40 (d,J2Hz,6H), 7.1-7.3 (m,aromatic protons);
Analysis Found: C,60.8;H,7.1,
$C_{41}H_{58}O_{15}$. $H_2O$ requires C,60.9;H,7.5%.

Example 33

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-octanoate

A solution of Compound 2 of Intermediate 40 (180mg) in formic acid (5ml) and water (1ml) was heated

to 50 to 60°C for 2 hours, and then evaporated to dryness, and residual solvents were removed by addition and evapoation of toluene (3x10ml). The residue was dissolved in ether (20ml), and the solution extracted with 1%-aqueous potassium bicarbonate (2x20ml). The extract was washed with ether (20ml), then covered with ethyl acetate (40ml), and 2 N-hydrochloric acid was added, with stirring, to pH1. The organic phase was washed with water, and brine (20ml of each), and dried (magnesium sulphate), and evaporated to an oily solid. A portion (81mg) of this material was purified by preparative HPLC (PLC 509, Spherisorb ODS-2, development with 60% acetonitrile in water containing sulphuric acid (0.15ml/L)). Fractions containing the major component were combined, the acetonitrile evaporated off, and the residual aqueous solution (ca 25ml) extracted with ethyl acetate (3x25ml). The extract was washed with water, and brine (25ml of each), and dried (magnesium sulphate), and evaporated to dryness to give the title compound (50mg) as a colourless solid; δ($CD_3OD$) includes 0.8-0.9 (m,$CH_3$,6 protons), 1.2- 1.4 (m,$OCOCH_2CH_2(CH_2)_4Me$), 1.6- 1.7 (m,$OCOCH_2CH_2(CH_2)_4Me$), 2.45 (t,J7Hz,$OCOCH_2(CH_2)_5Me$), 2.10 (s,$OCOCH_3$), 5.00 (s,3H), 5.05 (broad s,$=CH_2$), 5.12 (d,J5Hz,CHOAc), 5.25 (d,J2Hz), 5.32 (d,J2Hz), (6H,7H), 7.1-7.3 (m,aromatic protons); Analysis Found: C,58.4;H,6.7,

$C_{33}H_{34}O_{14}$. $0.75H_2O$ requires C,58.4;H,6.8%.

## Example 34

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-octanoate

A solution of Intermediate 41 (120mg) in trifluoroacetic acid (4.5ml) and water (0.5ml) was stirred at 20°C for 30 minutes, then diluted with water (5ml), and evaporated to dryness, and residual solvents were removed by addition and evaporation of toluene (3x10ml). The residue was dissolved in ether (20ml), and the solution extracted twice with 1% aqueous potassium bicarbonate (20ml, 10ml). The combined extract was washed with ether (10ml), then covered with ethyl acetate (30ml), and 2N-hydrochloric acid was added, with stirring, to pH1. The organic phase was washed with water, and brine (20ml of each), and dried (magnesium sulphate), and evaporated to dryness to give the title compound (60mg) as a gum. A portion of this material (42mg) was further purified by preparative HPLC (PLC 498,Spherisorb ODS-2, development with 50-85% acetonitrile in water containing sulphuric acid ((0.15ml/L), flow rate 10ml/min). The appropriate fractions were combined, the acetonitrile evaporated off and the residual aqueous solution (ca 20ml) extracted with ethyl acetate (3x25ml). The extract was washed with water, and brine (25ml of each), and dried (magnesium sulphate), and evaporated to dryness to give the title compound (3mg); having spectral characteristics corresponding to an authentic sample prepared in Example 31 above.

## Example 35

[1S-[1α(4R*,5S*),3α,4β,5α,6α(2E,4R*,6R*),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid 6-(4,6-dimethyl-2-octenoate), 7-octanoate

A solution of Intermediate 43 (140mg) in formic acid (5ml) and water(1ml) was heated to 50 to 60°C for 2 hours, then evaporated to dryness, and residual solvents were removed by addition and evaporation of toluene (3x10ml). The residue was purified by preparative HPLC (PLC 522, Spherisorb ODS-2, development with 68 to 90% acetonitrile in water containing sulphuric acid (0.15ml/L), flow rate 10ml/min). The appropriate fractions were combined, the acetonitrile evaporated off, and the residual aqueous solution (ca 20ml) extracted with ethyl acetate (3x50ml). The extract was washed with water, and brine (25ml of each), and dried (magnesium sulphate), and evaporated to dryness to give the title compound (20mg) as a colourless solid; δ($CD_3OD$) includes 0.8-0.9 (m,12 protons) and 1.02 (d,J7Hz,3 protons), ($CH_3$), 1.25-1.4 (m,$OCOCH_2CH_2(CH_2)_4Me$), 1.6 to 1.7 (m,$OCOCH_2CH_2(CH_2)_4Me$), 2.45 (m,$OCOCH_2(CH_2)_5Me$), 2.10 (s,$OCOCH_3$), 4.97 (s) and 5.02 (s), ($=CH_2$), 5.10 (d,J4Hz CHOAc), 5.15 (s,3H), 5.25 (d,J2Hz,7H), 5.80 (d,J16Hz,OCOCH = CH), 6.44 (d,J2Hz,6H), 6.83 (dd,J7,16Hz,OCOCH = CHCH(Me)), 7.1-7.4 (m,aromatic protons); Analysis Found: C,63.1;H,7.4, $C_{43}H_{60}O_{15}$ (816.95) requires C,63.2;H,7.4%.

## Example 36

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 7-acetate

A solution of Intermediate 44 (220mg) in formic acid (5ml) and water(1ml) was heated to 50 to 60°C for 2hours, then evaporated to dryness, and residual solvents were removed by addition and evaporation of toluene (3x10ml). The residue was dissolved in ether (20ml), and the solution extracted with 1% aqueous potassium bicarbonate (20ml, 10ml). The extract was washed with ether (10ml), then covered with ethyl acetate (30ml), and 2N-hydrochloric acid was added, with stirring, to pH1. The organic phase was washed with water and brine (20ml of each), and dried (magnesium sulphate), and evaporated to a foam. A portion of this material (83mg) was purified by preparative HPLC (Spherisorb ODS-2, development with 35% acetonitrile in water containing sulphuric acid (0.15ml/L), flow rate 10ml/min). Fractions containing the major component were combined, the acetonitrile evaporated off, and the residual aqueous solution (ca 20ml) extracted with ethyl acetate (3x50ml). The extract was washed with water and brine (50ml of each), and dried (magnesium sulphate), and evaporated to dryness to give the title compound (45mg) as a colourless solid; δ($CD_3OD$) includes 0.83 (d,J8Hz,$CH_3$), 2.10 (s), 2.16 (s), ($OCOCH_3$), 4.98 (s,3H), 5.02 (s,=$CH_2$), 5.10 (d,J5Hz,CHOAc), 5.24 (d,J2Hz), 5.28 (d,J5Hz), (6H,7H), 7.1-7.3 (m,aromatic protons);
Analysis Found: C,53.7;H,5.6,
$C_{27}H_{32}O_{14}$. 1.25 $H_2O$ (603.1) requires C,53.8;H,5.8%.

## Example 37

[1S-[1α(4R*,5S*)3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-pentanoate

Intermediate 46 (447mg) was heated (4h) at 65°C in formic acid (12.5ml) and water (3.9ml). The reaction mixture was evaporated to dryness and the residue partitioned between water (30ml) containing 0.880 ammonia soln (3ml) and diethylether (30ml). The aqueous layer was washed with more diethylether (2x30ml) and then acidified with 2N hydrochloric acid to pH1. This was then extracted with ethyl acetate (2x100ml) and the organic ectracts dried over sodium sulphate and evaporated to give a residue. This material was purified by preparative h.p.l.c. (5ODS-2 column) eluting with 60% (95% acetonitrile, 5% water with 0.15ml/litre conc. sulsphuric acid) in water also containing 0.15ml/litre conc. sulphuric acid. Appropriate fractions were combined and the acetonitrile evaporated. The resultant aqueous solution was extracted with ethyl acetate (3x) and the organic phase dried over sodium sulphate and evaporated to give the title compound (81mg) as a gum; δ($CDCl_3$) includes 0.75-0.92 (m,-$CH_2CH_3$ and -$CHCH_3$), 2.05 (s,-$OCOCH_3$), 4.03 (s,7-H), 4.94 and 4.98 (2s,C=$CH_2$), 5.06 (d,J=4Hz,-CHOAc), 5.28 (s,3-H), 5.94 (s,6-H) and 7.05-7.30 (m,aromatic protons); HPLC S5ODS-2 column, 50% MeCN/$H_2O$ containing 150µl/L $H_2SO_4$ gave a retention time of 4.11min.

## Example 38

[1S-[1α(4R*,5S*)3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-pentanoate, tripotassium salt

Example 37 (81mg) in water (30ml) was treated with potassium hydrogen carbonate (38mg) and stirred for 0.5h. The aqueous solution was then washed with diethyl ether (80ml) and freeze dried overnight to give the title compound (93mg) as a white solid. HPLC S5ODS-2 column, 50% MeCN/$H_2O$ containing 150µl/L $H_2SO_4$ gave a retention time of 11.18min; δ($D_2O$) includes 0.66-0.82 (m,-$CH_2CH_3$ and -$CHCH_3$), 2.04 (s,-$COCH_3$), 3.79 (d,J=2Hz,7-H), 4.74 (s,3-H), 4.78 (d,J=5Hz,-$CHCOCH_3$), 4.85 and 4.92 (2s,-C=$CH_2$), 6.04 (d,J=1Hz,6-H) and 7.06-7.26 (m,aromatic protons).

## Example 39

[1S-[1α(4R*,5S*)3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(7-phenylheptanoate)

Intermediate 47 (533mg) was heated (2h) at 65°C in formic acid (14.9ml) and water (4.6ml). The reaction mixture was evaporated to dryness and the residue partitioned between water (30ml) containing 0.880 ammonia soln (3ml) and diethyl ether (30ml). The aqueous layer was washed with more diethyl ether (2x30ml) and then acidified with 2N hydrochloric acid to pH1. This was then extracted with ethyl acetate (2x100ml) and the organic extracts dried over sodium sulphate and evaporated to give a residue. This material was purified by preparative h.p.l.c, (5ODS-2 column) eluting with 70% (95% acetonitrile, 5% water

with 0.15ml/litre conc. sulphuric acid) in water also containing 0.15ml/litre conc sulphuric acid. Appropriate fractions were combined and acetonitrile evaporated. The resultant aqueous solution was extracted with ethyl acetate (3x) and the organic phase dried over sodium sulphate to give the title compound (102mg); δ-($CD_3OD$) includes 0.82-0.94 (m,-$CHCH_3$ and -$CH_2CH_3$), 2.02 (s,-$COCH_3$), 4.02 (d,J = 2Hz,7-H), 6.28 (d,J = 2Hz,6-H), 7.08-7.30 (m,aromatic protons). HPLC S5ODS-2 column, 60% $MeCN/H_2O$ containing 150µl/L $H_2SO_4$ gave a retention time of 7.29 min.

Example 40

[1S-[1α(4R*,5S*)3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(7-phenylheptanoate), tripotassium salt

Example 39 (102mg) in water (30ml) was treated with potassium hydrogen carbonate (41.4mg) and stirred for 0.5h. The solution was then washed with diethylether (100ml) and freeze dried overnight to give the title compound (151mg) as a white solid; HPLC S5ODS-2 column, 50% $MeCN/H_2O$ containing 150µl/L $H_2SO$ gave a retention time of 12.18min; δ($D_2O$) includes 0.62-0.68 (m,2x-$CH_2CH_3$), 1.96 (s,-$COCH_3$), 3.74 (d,J = 2Hz,7-H), 5.96 (s,6-H), 6.96-7.21 (m,aromatic protons).

Example 41

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4-cyclohexylbutanoate)

To a solution of Intermediate 48 (628mg) in 98-100% formic acid (17.1ml) was added water (5.4ml) and the mixture stirred at 65°C for 1.5h. The reaction was evaporated to a gum and partitioned between diethyl ether (150ml) and water (150ml) containing 0.88 ammonia (10ml). The aqueous layer was washed with diethyl ether (3x50ml), acidified to pH1 with 2M aqueous hydrochloric acid and extracted with ethyl acetate (2x75ml). The combined organic extracts were washed with brine (50ml),dried ($MgSO_4$) and evaporated to a gum. This was purified by preparative hplc on a Spherisorb ODS-2 (20x250mm) column eluting with 60% (acetonitrile:water (95:5) containing 0.15ml/l conc. $H_2SO_4$):40% (water containing 0.15ml/1 conc. $H_2SO_4$). Appropriate fractions from each run were combined, the acetonitrile removed in vacuo (bath temperature <40°C) and the remainder saturated with sodium chloride and extracted with ethyl acetate. The combined extracts were washed with brine, dried ($MgSO_4$) and evaporated to give the title compound (146mg) as a clear, colourless oil; δ ($CD_3OD$) includes 0.87(d,3H,J = 7Hz,$CH_3$),2.10(s,3H,$O_2CCH_3$), 4.03(d,1H,J = 2Hz,7H), 4.98 and 5.04(2s,2H,C = $CH_2$), 5.08(d,1H,J = 7Hz,$CHO_2CCH_3$), 5.27(s,1H,3H), 6.27(d,1H,J = 2Hz,6H), 7.12-7.31(m,5H,aromatic protons); analytical hplc (Spherisorb ODS-2, solvent 50% acetonitrile:water containing 0.15ml/l conc.$H_2SO_4$) gave a retention time of 10.57min.

Example 42

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(4-cyclohexylbutanoate), tripotassium salt

To a mixture of Example 41 (143mg) and water (50ml) was added potassium bicarbonate (61mg) and the reaction stirred vigorously at room temperature for 0.5h and then sonicated for 15min. The mixture was washed with diethyl ether (25ml) and the aqueous layer freeze-dried to give the title compound (165mg) as a white powder; δ ($D_2O$) includes 0.91(d,3H,J = 7Hz,$CH_3$), 2.18(s,3H,$O_2CCH_3$), 3.97(broad s,1H,7H), 6.19-(broad s,1H,6H), 7.23-7.42(m,5H,aromatic protons); analytical hplc (Spherisorb ODS-2, solvent 50% acetonitrile:water containing 0.15ml/l conc. $H_2SO_4$) gave a retention time of 10.40min.

Example 43

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(cyclohexanecarboxylate)

To a solution of Intermediate 49 (92mg) in 98-100% formic acid (2.6ml) was added water (0.81ml) and the mixture stirred at 65°C for 1h. The reaction was evaporated to a gum and partitioned between diethyl ether (30ml) and water (30ml) containing 0.88 ammonia (2ml). The aqueous layer was washed with diethyl

ether (3x10ml), acidified to pH1 with 2M aqueous hydrochloric acid and extracted with ethyl acetate (2x15ml). The combined organic extracts were washed with brine (10ml), dried ($MgSO_4$) and evaporated to a gum. This was purified by preparative hplc on a Spherisorb ODS-2 (20x250mm) column eluting with 48% (acetonitrile:water (95:5) containing 0.15ml/l conc. $H_2SO_4$):52% (water containing 0.15ml/l conc. $H_2SO_4$). Appropriate fractions from each run were combined, the acetonitrile removed in vacuo (bath temperature <40°C) and the remainder saturated with sodium chloride and extracted with ethyl acetate. The combined extracts were washed with brine, dried ($MgSO_4$) and evaporated to give the title compound (32mg) as an opaque white gum; δ ($CD_3OD$) includes 0.87(d,3H,J=7Hz,$CH_3$), 2.09(s,3H,$O_2CCH_3$), 4.00(d,1H,J=2Hz,7H), 4.98 and 5.04(2s,2H,C=$CH_2$), 5.07(d,1H,J=7Hz,$CHO_2CCH_3$), 5.27(s,1H,3H), 6.28(d,1H,J=2Hz,6H), 7.07-7.42(m,5H,aromatic protons); approximate molecular weight 648; -FAB mass spectrometry $[M-H]^-$ 647.

Example 44

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(cyclohexanecarboxylate), tripotassium salt

To a mixture of Example 43 (26mg) and water (15ml) was added potassium bicarbonate (11.9mg) and the reaction stirred vigorously at room temperature for 0.5h. The mixture was washed with diethyl ether (15ml) and the aqueous layer freeze-dried to give the title compound (29mg) as a white powder; δ ($D_2O$) includes 0.93(d,3H,J=7Hz,$CH_3$), 2.21(s,3H,$O_2CCH_3$), 3.94(s,1H,7H), 6.17(s,1H,6H), 7.18-7.44(m,5H,aromatic protons); analytical hplc (Spherisorb ODS-2, solvent 40% acetonitrile:water containing 0.15ml/l conc. $H_2SO_4$) gave a retention time of 11.70min.

Example 45

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-[1-(tricyclo[3.3.1.1$^{3,7}$]decyl)acetate]

To a solution of Intermediate 50 (375mg) in 98-100% formic acid (10.1ml) was added water (3.2ml) and the mixture stirred at 65°C for 2.5h. The reaction was evaporated to a gum and partitioned between diethyl ether (75ml) and water (75ml) containing 0.88 ammonia (5ml). The aqueous layer was washed with diethyl ether (3x25ml), acidified to pH1 with 2M aqueous hydrochloric acid and extracted with ethyl acetate (2x35ml). The combined organic extracts were washed with brine (25ml), dried ($MgSO_4$) and evaporated to gum. This was purified by preparative hplc on a Spherisorb ODS-2 (20x250mm) column eluting with 61% (acetonitrile:water (95:5) containing 0.15ml/l conc. $H_2SO_4$):39% (water containing 0.15ml/l conc. $H_2SO_4$). Appropriate fractions from each run were combined, the acetonitrile removed in vacuo (bath temperature<40°C) and the remainder saturated with sodium chloride and extracted with ethyl acetate. The combined extracts were washed with brine, dried ($MgSO_4$) and evaporated to give the title compound - (107mg) as an opaque, white gum; δ ($CD_3OD$) includes 0.86(d,3H,J=7Hz,$CH_3$), 2.10(s,3H,$O_2CCH_3$), 4.04-(d,1H,J=2Hz,7H), 4.99 and 5.04(2s,2H,C=$CH_2$), 5.07(d,1H,J=7Hz $CHO_2CCH_3$), 5.27(s,1H,3H), 6.23-(d,1H,J=2Hz,6H), 7.12-7.33(m,5H,aromatic protons); analytical hplc (Spherisorb ODS-2, solvent 50% acetonitrile:water containing 0.15ml/l conc. $H_2SO_4$) gave a retention time of 8.40min.

Example 46

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-[1-(tricyclo[3.3.1.1$^{3,7}$]decyl)acetate], tripotassium salt

To a mixture of Example 45 (105mg) and water (30ml) was added potassium bicarbonate (43mg) and the reaction stirred vigorously at room temperature for 10min and then sonicated for 15min. The mixture was washed with diethyl ether (15ml) and the aqueous layer freeze-dried to give the title compound - (118mg) as a white powder; δ ($D_2O$) includes 0.88(d,3H,J=7Hz,$CH_3$), 2.17(s,3H,$O_2CCH_3$), 4.07(broad s,1H,7H), 6.15(broad s,1H,6H), 7.19-7.43(m,5H,aromatic protons); analytical hplc (Spherisorb ODS-2, solvent 50% acetonitrile:water containing 0.15ml/l conc. $H_2SO_4$) gave a retention time of 8.50min.

Example 47

[1S-[4α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-benzoate

To a solution of Intermediate 51 (570mg) in 98-100% formic acid (16.2ml) was added water (5.1ml) and the mixture stirred at 65°C for 2.5h. The reaction was evaporated to an oil then partitioned between diethyl ether (150ml) and water (150ml) containing 0.88 ammonia (10ml). The aqueous layer was washed with diethyl ether (3x50ml), acidified to pH1 with 2N aqueous hydrochloric acid and extracted with ethyl acetate (2x75ml). The combined organic extracts were washed with brine (50ml), dried ($MgSO_4$) and evaporated to a gum. This was purified by preparative hplc on a Spherisorb ODS-2 (20x250mm) column eluting with 50% (acetonitrile:water (95:5) containing 0.15ml/l conc. $H_2SO_4$):50% (water containing 0.15ml/l conc. $H_2SO_4$). Appropriate fractions from each run were combined, the acetonitrile removed in vacuo (bath temperature <40°C) and the remainder saturated with sodium chloride and extracted with ethyl acetate. The combined extracts were washed with brine, dried ($MgSO_4$) and evaporated to give the title compound (182mg) as a clear, colourless gum; analytical HPLC (Spherisorb ODS-2, solvent 45-60% (over 22 min) acetonitrile/water containing 0.15ml/L concentrated sulphuric acid) gave a retention time of 7.14min; -FAB mass spectrometry $[M-H]^-$641.

Example 48

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-benzoate, tripotassium salt

To a mixture of Example 47 (182mg) and water (45ml) was added potassium bicarbonate (84mg) and the reaction stirred vigorously at room temperature for 0.5h. The mixture was washed with diethyl ether (25ml) and the aqueous layer freeze-dried to give the title compound (208mg) as a white powder; δ ($D_2O$) includes 0.88(d,3H,J=7Hz,$CH_3$), 2.15(s,3H,$O_2CCH_3$), 4.07(d,1H,J=2Hz,7H), 6.39(broad s,1H,6H),7.13-8.08-(4m,10H,aromatic protons); analytical hplc (Spherisorb ODS-2, solvent 50% acetonitrile:water containing 0.15ml/l conc. $H_2SO_4$) gave a retention time of 4.10min.

Example 49

[1S-[1α(4R*,5S*),3α,4β,5α,6α(2E),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(3-phenyl-2-propenoate)

To a solution of Intermediate 52 (612mg) in 98-100% formic acid (17.1ml) was added water (5.4ml) and the mixture stirred at 65°C for 1.5h. The reaction was evaporated to a gum and partitioned between diethyl ether (150ml) and water (150ml) containing 0.88 ammonia (10ml). The aqueous layer was washed with diethyl ether (3x50ml), acidified to pH1 with 2M aqueous hydrochloric acid and extracted with ethyl acetate (2x75ml). The combined organic extracts were washed with brine (50ml), dried ($MgSO_4$) and evaporated to a foam. This was purified by preparative hplc on a Spherisorb ODS-2 (20x250mm) column eluting with 50% (acetonitrile:water (95:5) containing 0.15ml/l conc. $H_2SO_4$):50% (water containing 0.15ml/l conc. $H_2SO_4$). Appropriate fractions from each run were combined, the acetonitrile removed in vacuo (bath temperature<40°C) and the remainder saturated with sodium chloride and extracted with ethyl acetate. The combined extracts were washed with brine, dried ($MgSO_4$) and evaporated to give the title compound - (188mg) as a white foam/gum; δ ($CD_3OD$) includes 0.87(d,3H,J=7Hz,$CH_3$), 2.10(s,3H,$O_2CCH_3$), 4.13(broad s,1H,7H), 4.98 and 5.04(2s,2H,C=$CH_2$), 5.09(d,1H,J=7Hz,$CHO_2CCH_3$), 5.31(s,1H,3H), 6.40(broad s, 1H,6H), 6.51(d,1H,J=15Hz,CH=CHPh), 7.04-7.63(3m,10H,aromatic protons), 7.72(d,1H,J=15Hz,CH=CHPh); analytical hplc, [Spherisorb ODS-2, solvents B (acetonitrile:water(95:5) containing 0.15ml/l conc. $H_2SO_4$) and A (water containing 0.15ml/l conc. $H_2SO_4$) in a gradient of 45-60% B over 20min] gave a retention time of 10.66min.

Example 50

[1S-[1α(4R*,5S*),3α,4β,5α,6α(2E),7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(3-phenyl-2-propenoate), tripotassium salt

To a mixture of Example 49 (181mg) and water (45ml) was added potassium bicarbonate (80mg) and the reaction stirred vigorously at room temperature for 0.5h. The mixture was washed with diethyl ether (25ml) and the aqueous layer freeze-dried to give the title compound (214mg) as a white powder; δ ($D_2O$)

includes 0.88(d,3H,J = 7Hz,$CH_3$), 2.17(s,3H,$O_2CH_3$), 4.06(s,1H,7H), 6.30(broad s,1H,6H), 6.58-(d,1H,J = 15Hz,CH = CHPh), 7.15-7.74(3m,10H,aromatic protons), 7.77(d,1H,J = 15Hz,CH = CHPh; analytical hplc (Spherisorb ODS-2, solvent 40% acetonitrile:water containing 0.15ml/l conc. $H_2SO_4$) gave a retention time of 13.48 min.

Example 51

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(5-oxo-hexanoate)

Intermediate 53 (600mg) was dissolved in formic acid (16ml) and water (4ml). The solution was heated at 65° for 110min, it was then evaporated to dryness and the mixture was partitioned between ether (100ml) and ammonium hydroxide (100ml, containing 3.3ml of 0.88 ammonia). The aqueous layer was washed with ether (2x50ml) and the ether layer was discarded. The aqueous layer was acidified with 4M hydrochloric acid (15ml) and the required acid was extracted into ethyl acetate (1x100ml), (2x50ml). The ethyl acetate extract was washed with brine (2x50ml), dried ($MgSO_4$) and evaporated to dryness to give a foam. This material was purified by preparative hplc using acetonitrile-water 2:3 containing sulphuric acid 0.15ml/L and a Spherisorb 5-ODS-2 column (25cmx20mm). Appropriate fractions were combined and the acetonitrile was removed by evaporation under reduced pressure (bath temp. 40°C) and the aqueous solution was extracted with ethyl acetate (50mlx3). The ethyl acetate extract was washed with water(25mlx3), dried ($MgSO_4$) and evaporated to dryness to give the title compound (100mg); δ ($CD_3OD$) includes 0.85(d,J = 6Hz,$CH_3$), 2.1 and 2.12(2s,$COCH_3$ and $OCOCH_3$), 4.03(s,7H), 4.98,5.03(2s, = $CH_2$), 5.1(d,J = 4Hz,CHOAc), 5.26(s,3H), 6.3(s,6H)-,7.14-7.3(aromatic protons); analysis found: C,54.8; H,6.2, $C_{31}H_{38}O_{15}$. 1.5$H_2O$ requires C,54.9, H,6.1%.

Example 52

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(5-oxo-hexanoate), tripotassium salt

Example 51 (83mg) was stirred with potassium hydrogen carbonate (36mg) in water (50ml) at room temperature for 1h. It was then extracted with diethyl ether (50ml), the ether layer was discarded and the aqueous layer was freeze-dried to give the title compound (83mg); δ ($D_2O$) includes 0.91(d,6Hz,CH-$CH_3$), 2.20,2.21(2s,$OCH_3$ and $COCH_3$), 4.0(s,7H), 4.9(s,3H), 4.92(d,J = 4Hz,CHOAc), 5.08,5.01(2s, = $CH_2$), 6.21-(s,6H), 7.24-7.41(aromatic protons), Hplc (15cm, 5ODS-2 analytical column solvent acetonitrile-water containing 0.15ml $H_2SO_4$/L, gradient 45-60% acetonitrile over 20min) gave a retention time of 5.06 min.

Example 53

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(6-oxo-heptanoate)

Intermediate 54 (530mg) was dissolved in formic acid (12ml) and water (4ml). The solution was heated at 65°C for 2h and then evaporated to dryness. It was then dissolved in acetonitrile-water 2:3 containing sulphuric acid (1ml/l, 20ml) and chromatographed on P40 silica (100ml) using acetonitrile-water 2:3 containing sulphuric acid (1ml/L) as solvent. Appropriate fractions were combined, the acetonitrile was removed by evaporation under reduced pressure and the aqueous mixture was extracted with ethyl acetate (100mlx3). The extract was washed with water, dried ($MgSO_4$) and evaporated to dryness to give the title compound (110mg); δ ($CD_3OD$) includes 0.86(d,J = 6Hz,$CH_3$), 2.1 and 2.11(2s,$COCH_3$ and $OCOCH_3$), 4.03-(s,7H), 4.98 and 5.03(2s, = $CH_2$), 5.1(d,J = 3Hz,CHOAc), 5.17(s,3H), 6.3(s,6H), 7.15-7.3(aromatic protons); analysis found: C,55.6; H,6.0; $H_2O$; 3.8%, $C_{32}H_{40}O_{15}$ 1.5 $H_2O$ requires C,55.6; H,6.3; $H_2O$ 3.9%.

Example 54

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-(6-oxo-heptanoate), tripotassium salt

Example 53 (94mg) was stirred with potassium hydrogen carbonate(40mg) in water (50ml) for 30min. It was then extracted with ether (50ml), the ether layer was discarded and the aqueous layer was freeze-dried

to give the title compound (86mg); δ ($D_2O$) includes 0.91 (d,J=7Hz,CH-$CH_3$), 2.19,2.20(2s,$OCH_3$ and $COCH_3$), 3.98(s,7H), 4.9(s,3H), 5.02,5.08(2s,=$CH_2$), 6.2(s,6H), 7.22-7.43(aromatic protons); Hplc (15cm, 5ODS-2 analytical column solvent acetonitrile-water containing 0.15ml sulphuric acid/L, gradient 45-60% acetonitrile over 20min) gave a retention time of 5.4 min.

Example 55

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-acetate, tripotassium salt

Compound 1 of Example 28 (160mg) was stirred with potassium hydrogen carbonate (78mg) in water-(50ml) at room temperature for 15min. The mixture was then extracted with diethyl ether (50ml), the ether layer was discarded and the aqueous layer was freeze-dried to give the title compound (174mg); δ ($D_2O$) includes 0.90(d,6Hz,CH.$CH_3$), 2.1(s,6-$OCOCH_3$), 2.2(s,$OCOCH_3$), 4.04(s,7H), 4.9(s,3H), 4.94-(d,J=6Hz,CHOAc), 5.0 and 5.07(2s,=$CH_2$), 6.22(s,6H), 7.26-7.41(aromatic protons) Hplc (15cm, 5ODS-2 analytical column solvent acetonitrile-water containing 0.15ml $H_2SO_4$/L gradient 45-60° acetonitrile over 20min) gave a retention time of 4.37 min.

Example 56

[1S-[1α(4R*,5S*),3α,4β,5α,6α,7β]] 1-(4-Acetyloxy-5-methyl-3-methylene-6-phenylhexyl)-4,7-dihydroxy-6-[-(methoxycarbonyl)oxy]-2,8-dioxabicyclo[3.2.1]octane-3,4,5-tricarboxylic acid.

To a stirred solution of Intermediate 55 (398.6mg) in formic acid (14 ml) was added water (2.8 ml). The resulting solution was stirred at 60°C for 5 hours after which time it was separated between ethyl acetate (80 ml) and water (30 ml). The organic phase was then dried with saturated brine (2 x 30 ml) and $MgSO_4$. The solvent was then removed under reduced pressure to yield a colourless gum. This was dissolved in 1:1 acetonitrile/water (40 ml) and 5 ml aliquots of the resulting solution were applied to a preparative HPLC column (250 mm x 20 mm id, Spherisorb ODS-2) and eluted with 40%-60% acetonitrile/water with 150 µl/L conc. $H_2SO_4$, flow rate 15 ml/min. Appropriate fractions from each run were combined and the acetonitrile was removed by evaporation at reduced pressure. The remaining aqueous solution was extracted with ethyl acetate (80 ml) and the organic phase washed with water (40 ml) and saturated brine (30 ml). It was then dried over $MgSO_4$ and evaporated under reduced pressure to a colourless gum which was suspended in water (5ml) and freeze dried to a white, solid sample of the title compound (63.2mg); δ ($CD_3OD$) includes 0.85 (d, 3=6.5 Hz, $CH_3$), 2.11 (s, $OCOCH_3$), 3.78 (s, $OCO_2CH_3$), 4.07 (d, J=2Hz, 7H), 4.96, 5.02 (2s, =$CH_2$), 5.09 (d, J=5 Hz, CHOAc), 5.21 (s, 3H), 6.23 (d, J=2 Hz, 6H), 7.1-7.3 (m, aromatic protons); -FAB mass spectrometry 595 $[M-H]^-$, 551 $[M-CO_2H]^-$, 475 $[M-CO_2H-OCO_2CH_3]^-$; approximate molecular weight 596.6.

Analysis Found: C,49.59; H,5.53; N,1.93;

$C_{27}H_{32}O_{15}.3.5H_2O.0.9CH_3CN$ requires C,49.66; H,6.03; N,1.81%.

Example 57

[1S-[1α(4R*,5S*),3α,4β.5α,6α,7β]] 1-(4-Acetyloxy-5-methyl-3-methylene-6-phenylhexyl)-4,7-dihydroxy-6-[-(nonoxycarbonyl)oxy]-2,8-dioxabicyclo[3.2.1]octane-3,4,5-tricarboxylic acid.

To a solution of Intermediate 56 (325.1mg) in formic acid (10.6 ml) was added water (2.1 ml) with stirring. The resulting reaction mixture was stirred at 60°C for 5 hours after which time it was separated between water (30ml) and ethyl acetate (80 ml). The organic phase was washed with water (25 ml) and saturated brine (25 ml), and dried over $MgSO_4$. The solvent was then removed under reduced pressure to yield a colourless gum. This was dissolved in 1:1 acetonitrile/water (40 ml) and loaded in 5 ml aliquots onto an HPLC column (250 mm x 20 mm id, Spherisorb ODS-2). The column was eluted with 40%-60% acetonitrile/water with 150 µl/L conc. $H_2SO_4$, flow rate 15 ml/min. Appropriate fractions from each run were combined and the acetonitrile removed under reduced pressure to leave an acidic, aqueous solution of the title compound. The aqueous solution was extracted with ethyl acetate (100ml) and the organic phase washed with water (2 x 50ml) and saturated brine (50 ml) before being dried over $MgSO_4$. The solvent was then removed under reduced pressure yielding a colourless gum. This gum was suspended in water (5 ml) and freeze dried to a white, solid sample of the title compound (54.4mg); δ ($CD_3OD$) includes 0.86 (d, J=7

Hz, $CH_3$) 0.89 (t, J=6.5 Hz, $OCO_2(CH_2)_8CH_3$), 1.29 (s, broad, $OCO_2CH_2CH_2(CH_2)_6CH_3$), 1.65 (t, J=7 Hz. $OCO_2CH_2CH_2(CH_2)_6CH_3$), 2.10 (s, $OCOCH_3$), 4.07 (s, 7H), 4.14 (t, J=6 Hz, $OCO_2CH_2(CH_2)_7CH_3$), 4.96,5.02 (2s, $=CH_2$), 5.08 (d, J=5 Hz, CHOAc), 5.22 (s, 3H), 6.20 (s, broad, 6H), 7.1-7.4 (m, aromatic protons); approximate molecular weight 708.8; thermospray (TSP) negative, 707 $[M-H]^-$ 537, $[M-CO_2(CH_2)_8CH_3]^-$,519 $[M-OCO_2(CH_2)_8CH_3]^-$.
Analysis Found: 59.16; H,6.82;
$C_{35}H_{48}O_{15}$ requires : 59.31; H,6.83%.

Example 58

[1S-[1α(4R*,5S*),3α,4β,5α,6α,7β]] 1-(4-Acetyloxy-5-methyl-3-methylene-6-phenylhexyl)-4,7-dihydroxy-6-[-(methoxycarbonyl)oxy]-2,8-dioxabicyclo[3.2.1]octane-3,4,5-tricarboxylic acid, tripotassium salt

The product of Example 56 (148mg) was dissolved in water (50ml) containing potassium hydrogen carbonate (73mg). The resulting solution was extracted with diethyl ether (50ml) and the aqueous phase was freeze-dried to give the title compound (161mg) δ($D_2O$) includes 0.73(d,J=6Hz,$CH_3CH$), 2.01(s,$CH_3C$-(O)-O), 3.62(s,$CH_3OC$-(O)-O), 3.93(d,J=2Hz,7-H),4.69(s,3-H),4.75(d,J=3Hz,$CH_3C(O)$ - O-CH), 4.81,4.88-(2s,$C=CH_2$), 6.00(d,J=2Hz,6-H) and 7.03-7.24(m,aromatic protons); hplc retention time 4.15min (Spherisorb ODS-2 column, 35% acetonitrile/water + 0.15ml $H_2SO_4$/litre: 65%$H_2O$ + 0.15ml$H_2SO_4$/litre).

Example 59

[1S-[1α(4R*,5S*),3α,4β,5α,6α,7β]] 1-(4-Acetyloxy-5-methyl-3-methylene-6-phenylhexyl)-4,7-dihydroxy-6-[-(nonoxycarbonyl)oxy]-2,8-dioxabicyclo[3.2.1]octane-3,4,5-tricarboxylic acid, tripotassium salt

The product of Example 57 (100mg) was suspended in water (75ml) containing potassium hydrogen carbonate (42mg) and sonicated for 30min. The resulting cloudy solution was extracted with diethyl ether (50ml) and the clear aqueous phase was freeze-dried to give the title compound (96mg); δ ($D_2O$) includes 1.96(s,$CH_3C(O)$-O-), 3.82(s,7-H), 3.89-4.11(m,-O-C(O)-O-$CH_2CH_2$), 5.98(s,6-H) and 6.93-7.17(m,aromatic protons), hplc retention time 5.87min (Spherisorb ODS-2, 60% acetonitrile/water + 0.15ml $H_2SO_4$/litre : 40% $H_2O$ + 0.15ml $H_2SO_4$/litre).

Example 60

[1S-[1α(4R*,5S*)3α,4β,5α,6α,7β]] 1-(4-Acetyloxy-5-methyl-3-methylene-6-phenylhexyl)-6-[(butoxycarbonyl)-oxy]-4,7-dihydroxy-2,8-dioxabicyclo[3.2.1]octane-3,4,5-tricarboxylic acid

Intermediate 57 (520mg) was heated(3h) at 65°C in formic acid (14.5ml) and water (4.5ml). The reaction mixture was evaporated to dryness and the residue partitioned between water (30ml) containing 0.880 ammonia soln (3ml) and diethyl ether (30ml). The aqueous layer was washed with more diethyl ether (2x30ml) and then acidified with 2N hydrochloric acid to pH1. This was then extracted with ethyl acetate (2x100ml) and the organic extracts dried over sodium sulphate and evaporated to give a residue. This material was purified by preparative h.p.l.c. (5ODS-2 column) eluting with 60% (95% acetonitrile, 5% water with 0.15ml/litre conc. sulphuric acid) in water also containing 0.15ml/litre conc. sulphuric acid. Appropriate fractions were combined and acetonitrile evaporated. The resultant aqueous solution was extracted with ethyl acetate (3x) and the orgainic phase dried over sodium sulphate to give the title compound (122mg) as a foam; δ($CD_3OD$) includes 0.82-0.98 (m,-$CH_2CH_3$ and -$CHCH_3$), 2.10 (s,-$OCOCH_3$), 4.09 (s,7-H), 4.96 and 5.02 (2s,-$C=CH_2$), 5.08 (d,J=5Hz,-CH-OAc), 5.24 (s,3-H), 6.18 (s,6-H) and 7.05-7.35 (m,aromatic protons). Analysis found C54.0%,H6.0%,$H_2O$ 3.5%;$C_{30}H_{38}O_{15}$. 3/2$H_2O$ requires C54.1%,H6.2%,$H_2O$4.1%.

Example 61

[1S-[1α(4R*,5S*)3α,4β,5α,6α,7β]] 1-(4-Acetyloxy-5-methyl-3-methylene-6-phenylhexyl)-6-[(butoxycarbonyl)-oxy]-4,7-dihydroxy-2,8-dioxabicyclo[3.2.1]octane-3,4,5-tricarboxylic acid, tripotassium salt

The product of Example 60 (102mg) in water (20ml) was treated with potassium hydrogen carbonate (47.2mg) and stirred for 0.5h. The aqueous solution was then washed with diethyl ether (100ml) and freeze dried overnight to give the title compound (113mg) as a white solid. HPLC S5ODS-2 column, 40%

MeCN/$H_2O$ containing 150μl/L $H_2SO_4$ gave a retention time of 8.12min; δ($D_2O$) includes 0.87-0.98 (m,-$CH_2CH_3$ and -$CHCH_3$), 2.22 (s,-$COCH_3$), 4.08 (d,J = 2Hz,7-H), 4.22 (t,J = 6Hz,-$OCO_2CH_2CH_2$-), 4.98 and 5.06 (2s,-C = $CH_2$), 6.14 (d,J = 1Hz,6-H) and 7.24-7.42 (m,aromatic protons).

Example 62

[1S-[1α(4R*,5S*),3α,4β,5α,6α,7β]] 1-(4-Acetyloxy-5-methyl-3-methylene-6-phenylhexyl)-4,7-dihydroxy-6-methoxy-2,8-dioxabicyclo[3.2.1]octane-3,4,5-tricarboxylic acid

Intermediate 60 (140mg) was dissolved in formic acid (98-100%, 3.52ml) and the solution was stirred at room temperature for 30 minutes. Water (0.70ml) was added and stirring continued at 55°C for 7 hours and at room temperature for 16 hours. The solvent was removed under reduced pressure to give a yellow oil. The residue was taken up in 3% $KHCO_3$ (50ml) and washed with diethyl ether (2x120ml). The aqueous phase was acidified with 2N HCl to pH1-2, and was saturated with solid NaCl. This saturated aqueous layer was now extracted with ethyl acetate (2x150ml). The combined ethyl acetate fractions were dried over anhydrous $MgSO_4$ and the solvent was removed under reduced pressure to give a beige coloured foam, (61mg).

The foam was dissolved in 1:1 acetonitrile/water (5ml) and loaded onto a preparative HPLC column (250mmx20mm, Spherisorb ODS-2). The column was eluted with 35% acetonitrile/water containing 150μl/L conc $H_2SO_4$ at a flow rate of 15ml/min. Appropriate fractions were combined and the acetonitrile removed under reduced pressure to leave an acidic, aqueous solution. The solution was saturated with sodium chloride solution and then extracted with ethyl acetate (3x50ml). The combined organic layers were dried (anhydrous $MgSO_4$) and the solvent was removed under reduced pressure to yield a colourless glass. The material was taken up in water (5ml) and freeze-dried to yield the title compound as a white foam (15mg); δ ($CD_3OD$) includes 0.85 (d,J6.25Hz,3H,$CH_3$), 2.11 (s,3H,$OCOCH_3$), 3.44 (s,3H,6$OCH_3$, 4.05 (brs,1H,7CH), 7.10-7.30ppm (m,5H, aromatic protons); - FAB mass spectrometry $[M-H]^-$ m/z 551.7; $\nu_{max}$ (Nujol) 1711$cm^{-1}$ (C = 0).

Example 63

[1S-[1α(4R*,5S*),3α,4β,5α,6α,7β]] 1-(4-Acetyloxy-5-methyl-3-methylene-6-phenylhexyl)-6-[(aminocarbonyl)-oxy]-4,7-dihydroxy-2,8-dioxabicyclo[3.2.1]octane-3,4,5-tricarboxylic acid

A solution of Intermediate 61 (321mg) was dissolved in absolute formic acid (10ml) and diluted dropwise with water (3ml). The solution was heated for 1h at 70°C. The mixture was concentrated under reduced pressure and partitioned between ether (30ml) and water containing 0.880 ammonia (2ml). The aqueous phase was extracted with ether (3x15ml), acidified with 2MHCl (20ml) and extracted with ethyl acetate (100ml). The organic phase was washed with 2MHCl, brine, dried, and evaporated. The residue was purified by reverse-phase HPLC (1 inch Spherisorb ODS-2, flow rate 15ml/min eluting with 35% increasing to 95% acetonitrile-water containing 0.15ml concentrated sulphuric acid/L). Appropriate fractions were combined and the acetonitrile was removed under reduced pressure. The aqueous phase was extracted with ethyl acetate (x3), and the organic phase was washed with brine (x2), dried, and evaporated to give the title compound (46mg); $\nu_{max}$ (nujol) 3472,3366 (N-H), 3600-2300 (O-H,carboxylic acid), 1726$cm^{-1}$ (C = 0 ester, carbamate, carboxylic acids), δ($CD_3OD$) includes 0.85 (d,J = 7Hz,$CH_3CH$), 2.10 (s,AcO), 4.07 (d,J = 2Hz,7-H), 4.98 and 5.02 (2s,C = $CH_2$), 5.08 (d,J = 5Hz,CHOAc), 5.25 (s,3-H), 6.15 (d,J = 2Hz,6-H), 7.12-7.3 (m,$C_6H_5$).

Example 64

[1S-[1α(4R*,5S*),3α,4β,5α,6α,7β]] 1-(4-Acetyloxy-5-methyl-3-methylene-6-phenylhexyl)-4,6,7-trihydroxy-2,8-dioxabicyclo[3.2.1]octane-3,4,5-tricarboxylic acid, 6-[(E/Z)-3-octenoate]

A solution of Intermediate 62 (470mg) was dissolved in warm formic acid (20ml). Water (6ml) was added and the resulting solution was stirred at 65°C for 2h. The solution was evaporated and the residue partitioned between ether (50ml) and water (50ml, containing 5ml of 0.880 ammonia solution). The aqueous phase was separated, extracted with ether (3x50ml), acidified to pH1 with concentrated hydrochloric acid and extracted with ethyl acetate (4x50ml). The organic extracts were combined, washed with water(50ml), dried ($MgSO_4$) and evaporated to give a pale brown solid. This material was purified by preparative hplc

[Spherisorb ODS-2 column, flow rate 15ml/min, 45% (95:5 MeCN:$H_2O$ containing 0.15ml conc. $H_2SO_4$/L] 55% $H_2O$ containing 0.15ml conc. $H_2SO_4$/L). Appropriate fractions were combined and the acetonitrile evaporated. The cloudy aqueous phase was extracted with ethyl acetate (4x100ml) and the extracts were combined, dried ($MgSO_4$) and evaporated to give the title compounds as a cream coloured solid (59mg); δ-($CD_3OD$) includes 0.82-0.95 (m,$CH_3$ groups), 2.10 (s,$OCCH_3$), 3.02 (bd,J=5Hz 1 of,$OCOCH_2CH=CH$), 3.09 (bt,J~5Hz,1 of $OCOCH_2CH=CH$), 4.02 (s,fine splitting,7-H), 4.97 and 5.02 (2s,=$CH_2$), 5.08 (d,J=5Hz,$CHOCOCH_3$), 5.25 (s,3-H), 5.41-5.65 (m,CH=CH) 6.92 (s,fine splitting,6-H), 7.10-7.31 (m,aromatic protons); Mass spectrum 680 ($MNH_4^+$) implies $M^+$=662.

Example 65

[1S-[1α(3R*S*,4S*,5S*),3α,4β,5α,6α,7β]] 1-(4-Acetyloxy-3,5-dimethyl-6-phenylhexyl)-4,7-dihydroxy-6-[[-(methoxycarbonyl]oxy]-2,8-dioxabicyclo[3.2.1]octane-3,4,5-tricarboxylic acid (I) and [1S-[1α(3R*S*,5R*)-,3α,4β,5α,6α,7β]] 1-(3,5-dimethyl-6-phenylhexyl)-4,7-dihydroxy-6-[[(methoxycarbonyl]oxy]-2,8-dioxabicyclo-[3.2.1]octane-3,4,5-tricarboxylic acid (II)

A solution of Example 56 (500mg) in ethanol (20ml) was hydrogenated at room temperature and atmospheric pressure for 6.5 days using 20% palladium on charcoal catalyst (200mg). The catalyst was removed by filtration through kieselguhr and the filtrate was evaporated to give a gum. This material (438mg) was purified portionwise by hplc [ODS-2 column:eluent 40% (acetonitrile:water 95:5 containing 0.15ml/litre conc sulphuric acid)]. Appropriate fractions were combined, acetonitrile evaporated and the aqueous residue extracted with ethyl acetate (3x100ml). Organic extracts were dried ($MgSO_4$) and evaporated to yield the title compound (I) (64mg) as a white solid; δ ($CD_3OD$) includes 2.08 and 2.12-(2s,$OCOCH_3$), 3.78(broad s,$OCO_2CH_3$), 5.19 and 5.22(2s,3H), 6.16(s,6H), 7.08-7.30(m,aromatic protons);
Analysis Found: C,51.38%; H,6.06%; $H_2O$ 5.5%.
$C_{27}H_{34}O_{15}.2H_2O$ requires: C,51.1%; H,6.04%,$H_2O$ 5.67%.

The slower running component was extracted similarly to give the title compound (II) (103mg) as a white waxy solid; δ ($CD_3OD$) includes 3.76 and 3.78(2s,$OCO_2CH_3$), 4.10(d,J=3Hz,7H), 4.15(d,J=3Hz,7H), 5.20 and 5.22(2s,3H), 6.12 and 6.18(2s,6H), 7.08-7.28(m,aromatic protons.
Analysis Found: C,53.42%; H,6.53%; $H_2O$, 3.22%.
$C_{25}H_{32}O_{13}.H_2O$ requires C,53.76%; H,6.14%; $H_2O$, 3.90%.

Example 66

[1S-[1α(4R*,5S*),3α,4β,5α,6α,7β]] 1-(4-Acetyloxy-5-methyl-3-methylene-6-phenylhexyl)-6-[[(ethylamino)-carbonyl]oxy]4,7-dihydroxy-2,8-dioxabicyclo[3.2.1]octane-3,4,5-tricarboxylic acid

A solution of Intermediate 63 (230mg) in absolute formic acid (15ml) was heated to 80°C diluted with water (4ml) and stirred at 80°C for 2h. The mixture was then allowed to cool to 20°C and then evaporated to dryness under reduced pressure. The residue was purified by reverse-phase hplc on a Spherisorb ODS-2 (25cmx2cm id) column eluting with 45% acetonitrile:water (95:5) containing 0.15ml/L conc $H_2SO_4$:55% water containing 0.15ml/L conc $H_2SO_4$. Appropriate fractions were combined, and the acetonitrile removed under reduced pressure. The aqueous phase was extracted with ethyl acetate (x3), and the organic phase was washed with brine (x2), dried, evaporated to dryness. The residue was dissolved in water and freeze-dried to give the title compound (94mg); δ ($CD_3OD$) includes O.85(d,J7Hz,$CHCH_3$), 1.09(t,J7Hz,$NCH_2CH_3$), 2.10(s,AcO), 4.05(br s,7-H), 4.99 and 5.02(2s,=$CH_2$), 5.08(d,J5Hz,CHOAc), 5.25(s,3-H), 6.18(br s,6-H), 7.1-7.3(m,$C_6H_5$);
Analysis found: C, 50.66; H, 5.83; N, 2.02%.
$C_{28}H_{35}NO_{14}$. $3H_2O$ requires: C, 50.67; H, 6.23; N, 2.11%.

Example 67

[1S-[1α(4R*,5S*),3α,4β,5α,6α,7β]] 1-(4-Acetyloxy-5-methyl-3-methylene-6-phenylhexyl)-4,7-dihydroxy-6-[[-(methylamino)carbonyl]oxy]-2,8-dioxabicyclo[3.2.1]octane-3,4,5-tricarboxylic acid

A solution of Intermediate 64 (180mg) in absolute formic (5ml) was heated to 70°C diluted with water (1.7ml) and stirred at 70°C for 2h. The mixture was allowed to cool to 20°C and the formic acid was removed under reduced pressure. The residue was partitioned between water (30ml), ammonia (0.88; 3ml)

and ether (30ml). The aqueous layer was extracted with ether (x3), acidified with 2M HCl to pH 1 and extracted with ethyl acetate. The organic solution was washed with brine, dried, and evaporated to dryness. The residue was purified by reverse-phase hplc on a Spherisorb ODS-2 (25cmx2cm id) column eluting with 45% acetonitrile:water (95:5) containing 0.15ml/L conc $H_2SO_4$:55% water containing 0.15ml/L conc $H_2SO_4$. Appropriate fractions were combined, and the acetonitrile removed under reduced pressure. The aqueous phase was extracted with ethyl acetate (x3), and the organic phase was washed with brine (x2), dried and evaporated to dryness. The residue was dissolved in water and freeze-dried to give the title compound - (25mg); δ ($CD_3OD$) includes 0.86(d,J7Hz,$CHCH_3$), 2.10(s,AcO), 2.70(s,$NCH_3$), 4.05(br s,7-H),4.98 and 5.02-(2s,=$CH_2$), 5.08(d,J5Hz,CHOAc), 5.26(s,3-H), 6.17(br s,6-H), 7.1-7.3(m,$C_6H_5$);
Analysis found: C, 50.47; H, 5.48; N, 2.15%.
$C_{27}H_{33}NO_{14}$. 2.5$H_2O$ requires: C, 50.62; H, 5.98; N, 2.19%.

Example 68

Characteristics of IMI 332962

After 2-3 weeks growth at 25°C on oatmeal agar the colonies were smoke grey to mouse grey in colour (Rayner's Mycological Colour Chart, 1970; published by the Commonwealth Agricultural Bureaux) on both the surface and reverse of the colony.

Morphological observations of the strain grown at 25°C on oatmeal agar were made under an optical microscope. The fungus had no sexual reproductive stage but formed pycnidia, thereby placing it in the class Coelomycetes. The fungus produced rostrate pycnidia with loose hyphae and both aseptate and one-septate conidia. The conidia were approximately 5-9 x 1.5-3μM in dimensions (usually 7-9 x 1.502.5μM). Numerous multiseptate/multicellular, globose structures resembling chlamydospores or pycnidial initials were also observed. Distinct dictyochlamydospores were absent.

The isolate has been identified as a species of the genus Phoma, and the identity confirmed by the CAB International Mycological Institute.

Example 69

IN VITRO RESULTS

(1) The ability of compounds of the invention to inhibit the enzyme squalene synthase was demonstrated using [2-$^{14}$C] farnesylpyrophosphate as substrate under assay conditions similar to those described by S. A. Biller et al in J Medicinal Chemistry 31(10), 1869-1871(1988). [$^{14}$C] Squalene was separated from unreacted substrate on thin layer chromatography plates and determined by liquid scintillation counting. Inhibition of squalene synthase was quantified by incubating rat liver homogenate with various concentrations of the test compound in the presence of [2-$^{14}$C] farnesylpyrophosphate. The concentration of compound giving 50% inhibition of [$^{14}$C] squalene production in a 30 minute assay was taken as the $IC_{50}$ value.

In this test the title compounds in Examples 1, 2, 3, 5, 6, 7, 8, 9, 10, 11, 12, 13, 14, 15, 17, 18, 19, 20, 21, 24, 25, 26, 29, 31, 37, 39, 41, 43, 45, 47, 49, 51, 53, 56, 57, 60, 64, 66, 67, isomer 1 in Example 16, Compounds 1 and 2 in Example 28, the 6-decanoate in Example 30 and Compound (I) in Example 65 had $IC_{50}$ values of less than 100nM.

(2) The in vitro evaluation of the antifungal activity of compounds of the invention was performed by determining the minimum concentration (MIC) of the test compound at which growth of the particular microorganism in a suitable medium failed to occur. In practice, a series of agar plates, each having the test compound incorporated at a particular concentration, was inoculated with a standard culture of a clinically relevant pathogen, for example Candida albicans, and each plate was then incubated at 37°C for 24 to 48 hours depending on the pathogen. The plates were then examined for the presence or absence of growth of the fungus and the appropriate MIC was noted.

In this test the title compounds in Examples 1, 7, 11, 12, 13, 18, 24, 31, 39, 41, 45, 57, 64 and the 6-decanoate in Example 30 had MICs in the range of 0.1 to 31 μg/ml against a variety of clinically relevant pathogens.

Pharmaceutical Examples

In the following examples the term 'Active Ingredient' refers to a compound of the present invention, for

example a compound described in the Examples hereinabove.

Example 1 - Tablets

a)

| | |
|---|---|
| Active Ingredient | 5.0mg |
| Lactose | 95.0mg |
| Microcrystalline Cellulose | 90.0mg |
| Cross-linked Polyvinylpyrrolidone | 8.0mg |
| Magnesium Stearate | 2.0mg |
| Compression Weight | 200.0mg |

The active ingredient, microcrystalline cellulose, lactose and cross-linked polyvinylpyrrolidone are sieved through a 500 micron sieve and blended in a suitable mixer. The magnesium stearate is sieved though a 250 micron sieve and blended with the active blend. The blend is compressed into tablets using suitable punches.

b)

| | |
|---|---|
| Active Ingredient | 5.0mg |
| Lactose | 165.0mg |
| Pregelatinised Starch | 20.0mg |
| Cross-linked Polyvinylpyrrolidone | 8.0mg |
| Magnesium Stearate | 2.0mg |
| Compression weight | 200.0mg |

The active ingredient, lactose and pregelatinised starch are blended together and granulated with water. The wet mass is dried and milled. The magnesium stearate and cross-linked polyvinylpyrrolidone are screened through a 250 micron sieve and blended with the granule. The resultant blend is compressed using suitable tablet punches.

Example 2 - Capsules

a)

| | |
|---|---|
| Active Ingredient | 5.0mg |
| Pregelatinised Starch | 193.0mg |
| Magnesium Stearate | 2.0mg |
| Fill weight | 200.0mg |

The active ingredient and pregelatinised starch are screened through a 500 micron mesh sieve, blended together and lubricated with magnesium stearate (meshed through a 250 micron sieve). The blend is filled into hard gelatin capsules of a suitable size.

b)

| | |
|---|---|
| Active Ingredient | 5.0mg |
| Lactose | 177.0mg |
| Polyvinypyrrolidone | 8.0mg |
| Cross-linked Polyvinylpyrrolidone | 8.0mg |
| Magnesium Stearate | 2.0mg |
| Fill weight | 200.0mg |

The active ingredient and lactose are blended together and granulated with a solution of polyvinylpyrrolidone. The wet mass is dried and milled. The magnesium stearate and cross-linked polyvinylpyrrolidone are screened through a 250 micron sieve and blended with the granule. The resultant blend is filled into hard gelatin capsules of a suitable size.

Example 3 - Syrup

a)

| | |
|---|---|
| Active Ingredient | 5.0mg |
| Hydroxypropyl Methylcellulose | 45.0mg |
| Propyl Hydroxybenzoate | 1.5mg |
| Butyl Hydroxybenzoate | 0.75mg |
| Saccharin Sodium | 5.0mg |
| Sorbitol Solution | 1.0ml |
| Suitable Buffers | qs |
| Suitable Flavours | qs |
| Purified Water to | 10.0ml |

The hydroxypropyl methylcellulose is dispersed in a portion of hot purified water together with the hydroxybenzoates and the solution is allowed to cool to room temperature. The saccharin sodium, flavours and sorbitol solution are added to the bulk solution. The active ingredient is dissolved in a portion of the remaining water and added to the bulk solution. Suitable buffers may be added to control the pH in the region of maximum stability. The solution is made up to volume, filtered and filled into suitable containers.

Example 4 - Intranasal Solution

a) Preserved solution

| | % w/w |
|---|---|
| Active Ingredient | 0.1 |
| Dextrose (Anhydrous) | 5.0 |
| Benzalkonium Chloride | 0.02 |
| Suitable buffers | qs |
| Purified Water to | 100 |

The active ingredient and dextrose are dissolved in a portion of the bulk solution. Suitable buffers may be added to control the pH in the region of maximum stability. The solution is made up to volume, filtered and filled into suitable containers.

Alternatively, the solution may be provided as a sterile unit dose presentation such that the preservatives are omitted from the formulation.

b) Unpreserved sterile solution

| | % w/w |
|---|---|
| Active Ingredient | 0.1 |
| Dextrose (Anhydrous) | 5.0 |
| Suitable Buffers | qs |
| Purified Water to | 100 |

**Claims**

**1.** Compounds having the formula (I)

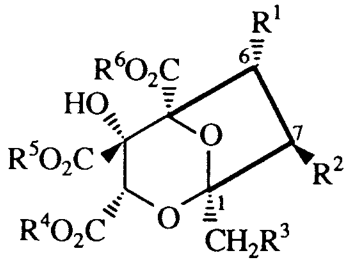

(I)

wherein $R^1$ represents a hydrogen atom or a hydroxyl, acyloxy, carbonate, carbamate or ether group;
$R^2$ represents a hydrogen atom, a hydroxyl group, a group $-OCOR^7$ or a group $-OCO_2R^7$ (where $R^7$ is a group selected from $C_{1-8}$alkyl, aryl, aryl$C_{1-4}$alkyl and $C_{3-8}$cycloalkyl);
$R^3$ represents a group selected from

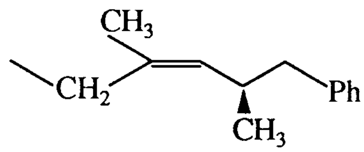

,

$-CH{-}{-}CR^8CR^9R^{10}CHR^{11}(CH_2)_mPh$,
$CH_2\overline{C}R^{\underline{12}}CR^{11}CR^9R^{10}CHR^{11}(CH_2)_nPh$,

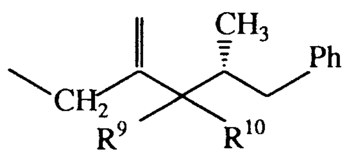

$-CH_2C(CH_3)\overset{E}{=}CHCH(CH_2R^{13})CH_2Ph$, $-CH_2C(CH_2OH)\overset{Z}{=}CHCH(CH_3)CH_2Ph$,
$-CH_2C(=CH_2)CH(OH)CH(CH_2OH)CH_2Ph$,
$-CH_2C(=CH_2)CH(NHCOCH_3)CH(CH_3)CH_2Ph$,
$-CH_2C(CH_2NHCOCH_3)\overset{E}{=}CHCH(CH_3)CH_2Ph$ and

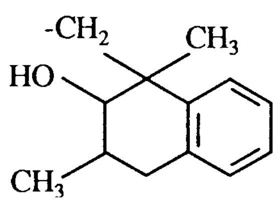

(where the dotted line represents the absence or presence of a single bond, $R^8$ represents a hydrogen atom or a hydroxyl, acyloxy, $C_{1-6}$alkoxy or $C_{1-4}$alkyl group, $R^9$ represents a hydrogen atom and $R^{10}$ represents a hydrogen atom or a hydroxyl, $C_{1-6}$alkoxy or acyloxy group or $CR^9R^{10}$ forms a group $C=0$, $R^{11}$ represents a hydrogen atom or a $C_{1-4}$alkyl group, $R^{12}$ represents a hydrogen atom or a methyl group, $R^{13}$ represents a hydrogen atom or a hydroxyl group, m represents 1 or 2 and n represents zero or 1);

$R^4$, $R^5$ and $R^6$ may each independently represent a hydrogen atom or a methyl group; and salts thereof;

With the provisos that (1) $R^1$ and $R^2$ cannot both represent hydrogen atoms and (2) when $R_1$ represents a hydrogen atom, a hydroxyl group or an acyloxy group selected from $-OCOCH\overset{E}{=}CHCH(CH_3)(CH_2)_3CH_3$, $-OCOCH\overset{E}{=}CHC(CH_3)\overset{E}{=}CHCH(CH_3)-CH_2CH_3$, or $-OCO-X-CH_2CH(CH_3)CH_2CH_3$ -

[where X is $-CH\overset{E}{=}CHCH(CH_3)-$, $-CH_2CH(OH)CH(CH_3)-$, $-CH\overset{E}{=}CHC(OH)(CH_3)-$, $-CH_2CH(OH)CH_2-$ or $-CH_2CH_2CH(CH_3)-$)] and $R_2$ represents a hydrogen atom or a hydroxyl group then $R_3$ cannot represent

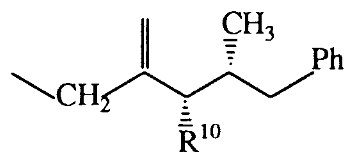

(where $R^{10}$ is a hydroxyl or an acetoxy group),
$-CH_2C(CH_3)\overset{E}{=}CHCH(CH_2R^{13})CH_2Ph$, $-CH_2C(CH_2OH)\overset{Z}{=}CHCH(CH_3)CH_2Ph$,
$-CH_2C(=CH_2)CH(OH)CH(CH_2OH)CH_2Ph$,
$-CH_2C(=CH_2)CH(NHCOCH_3)CH(CH_3)CH_2Ph$,
$-CH_2C(CH_2NHCOCH_3)\overset{E}{=}CHCH(CH_3)CH_2Ph$ or

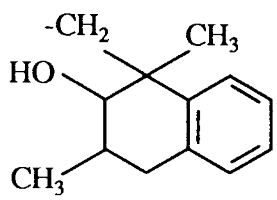
;

and salts thereof.

2. Compounds according to Clalm 1 in which $R^4$, $R^5$ and $R^6$ represent hydrogen atoms.

3. Compounds according to Claim 1 or Claim 2 in which $R^1$ represents an acyloxy, carbonate or ether group.

4. Compound according to Claim 1 or Claim 2 in which $R^1$ represents an acyloxy group.

5. Compounds according to Claim 4 in which $R^1$ represents a group selected from

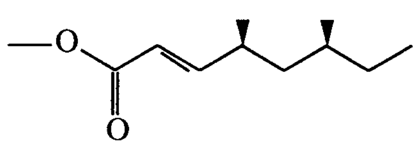 , 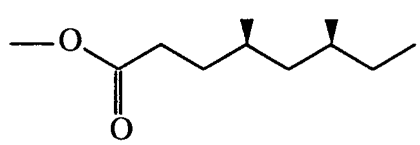 ,

alkanoyloxy, alkenoyloxy, -OCOPh, phenylalkanoyloxy, phenylalkenoyloxy, cycloalkanoyloxy and cycloalkylalkanoyloxy.

6. Compounds according to any preceding claim in which $R^2$ represents a hydroxyl, acetoxy or $-OCO_2CH_3$ group.

7. Compounds according to any preceding claim in which $R^3$ represents $-CH\underline{--}CR^8CR^9R^{10}CHR^{11}CH_2Ph$ or

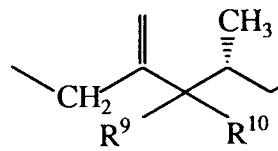

8. Compounds according to any preceding claim in which within $R^3$ the group -CH--$CR^8$- represents -CH=C($CH_3$)-, -$CH_2$CH($CH_3$)-, -$CH_2$CH(OH)-, -$CH_2$$CH_2$-or -$CH_2$CH($CH_2$$CH_3$)-.

9. Compounds according to any preceding claim in which within $R^3$ the group -$CR^9R^{10}$- represents -CH-(OH)-, -C(=0)-, -$CH_2$- or -CH($OCOCH_3$)-.

10. Particularly preferred compounds of the invention are:
[1S-[1α(4S*,5S*),3α,4β,5α,6α(2E,4R*,6R*),7β]] 1-(4-hydroxy-5-methyl-3-methylene-6-phenylhexyl)-4,6,7-trihydroxy-2,8-dioxabicyclo[3.2.1] octane-3,4,5-tricarboxylic acid, 6-(4,6-dimethyl-2-octenoate);
[1S-[1α[(E),5S*],3α,4β,5α,6α(2E,4R*,6R*),7β]] 1-(3,5-dimethyl-4-oxo-6-phenyl-2-hexenyl)-4,6,7-trihydroxy-2,8-dioxabicyclo[3.2.1]octane-3,4,5-tricarboxylic acid, 6-(4,6-dimethyl-2-octenoate);
[1S-[1α[(E),4R*,5S*],3α,4β,5α,6α(2E,4R*,6R*),7β]] 1-(4-hydroxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyl-2-octenoate);
[1S-[1α[3R*(S*),5S*],3α,4β,5α,6α(2E,4R*,6R*),7β]] 1-(3,5-dimethyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate), isomer 1;
[1S-[1α[3R*(S*),4R*,5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 1;
[1S-[1α[3R*(S*),4S*,5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 2;
[1S-[1α[3R*(S*),4R*(S*),5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 3;
[1S-[1α[3R*(S*),4R*(S*),5S*],3α,4β,5α,6α(2E,4R*,6R*),7β]] 1-(4-hydroxy-3,5-dimethyl-6-phenylhexyl)-4,6,7-trihydroxy-2,8-dioxabicyclo[3.2.1]octane-3,4,5-tricarboxylic acid, 6-(4,6-dimethyl-2-octenoate), isomer 4;
[1S-[1α(3R*S*,5S*),3α,4β,5α,6α(2E,4R*,6R*),7β]] 1-(3-ethyl-5-methyl-4-oxo-6-phenylhexyl)-4,6,7-trihydroxy-2,8-dioxabicyclo[3.2.1]octane-3,4,5-tricarboxylic acid, 6-(4,6-dimethyl-2-octenoate);
[1S-[1α[(E),4R*,5S*],3α,4β,5α,6α(4S*,6R*),7β]] 1-(4-acetyloxy-3,5-dimethyl-6-phenyl-2-hexenyl)-4,6,7-trihydroxy-2,8-dioxabicyclo[3.2.1]octane-3,4,5-tricarboxylic acid, 6-(4,6-dimethyloctanoate);
[1S-[1α(3R*S*,4S*,5S*),3α,4β,5α,6α(4S*,6R*),7β]] 1-(4-acetyloxy-3,5-dimethyl-6-phenylhexyl)-4,6,7-trihydroxy-2,8-dioxabicyclo[3.2.1]octane-3,4,5-tricarboxylic acid, 6-(4,6-dimethyloctanoate);
[1S-[1α(3R*S*,5R*), 3α,4β,5α,6α(4S*,6R*),7β]] 1-(3,5-dimethyl-6-phenylhexyl)-4,6,7-trihydroxy-2,8-dioxabicyclo[3.2.1]octane-3,4,5-tricarboxylic acid, 6-(4,6-dimethyloctanoate);
[1S-[1α(3R*S*,4S*,5S*),3α,4β,5α,6α(4S*,6R*),7β]] 1-(3,5-dimethyl-4-hydroxy-6-phenylhexyl)-4,6,7-trihydroxy-2,8-dioxabicyclo[3.2.1]octane-3,4,5-tricarboxylic acid, 6-(4,6-dimethyloctanoate);
[1S-[1α[(E),4R*,5S*],3α,4β,5α,6α(4S*,6R*),7β]] 1-(3,5-dimethyl-4-hydroxy-6-phenyl-2-hexenyl)-4,6,7-trihydroxy-2,8-dioxabicyclo[3.2.1]octane-3,4,5-tricarboxylic acid, 6-(4,6-dimethyloctanoate);
[1S-[1α(3R*(S*),5S*),3α,4β,5α,6α(4S*,6R*),7β]] 1-(3,5-dimethyl-4-oxo-6-phenylhexyl)-4,6,7-trihydroxy-2,8-dioxabicyclo[3.2.1]octane-3,4,5-tricarboxylic acid, 6-(4,6-dimethyloctanoate), 3,4,5-tripotassium salt, isomer 1;
[1S-[1α(4R*,5S*),3α,4β,5α,6α(4S*,6R*),7β]] 1-(4-hydroxy-5-methyl-3-oxo-6-phenylhexyl)-4,6,7-trihydroxy-2,8-dioxobicyclo[3.2.1]octane-3,4,5-tricarboxylic acid, 6-(4,6-dimethyloctanoate);
[1S-[1α(3R*S*,4R*,5S*),3α,4β,5α,6α(4S*,6R*),7β]] 1-(3,4-dihydroxy-5-methyl-6-phenylhexyl)-4,6,7-trihydroxy-2,8-dioxabicyclo[3.2.1]octane-3,4,5-tricarboxylic acid, 6-(4,6-dimethyloctanoate);
[1S-[1α(5R*S*),3α,4β,5α,6α(4S*,6R*),7β]] 1-(5-methyl-4-oxo-6-phenylhexyl)-4,6,7-trihydroxy-2,8-dioxabicyclo[3.2.1]octane-3,4,5-tricarboxylic acid, 6-(4,6-dimethyloctanoate);
[1S-[1α[3R*(S*)],3α,4β,5α,6α(4S*,6R*),7β]] 1-(3-methyl-4-oxo-6-phenylhexyl)-4,6,7-trihydroxy-2,8-dioxabicyclo[3.2.1]octane-3,4,5-tricarboxylic acid, 6-(4,6-dimethyloctanoate);
[1S-[1α(4R*,5S*),3α,4β,5α,6α(2E,4R*,6R*),7β]] 7-acetyloxy-1-(4-acetyloxy-5-methyl-3-methylene-6-phenylhexyl)-4,6-dihydroxy-2,8-dioxabicyclo[3.2.1]octane-3,4,5-tricarboxylic acid, 6-(4,6-dimethyl-2-octenoate);

[1S-[1α(4R*,5S*),3α,4β,5α,6α(2E,4R*,6R*),7β]] 1-(4-acetyloxy-5-methyl-3-methylene-6-phenylhexyl)-4,6-dihydroxy-7-[(methoxycarbonyl)oxy]-2,8-dioxabicyclo[3.2.1]octane-3,4,5-tricarboxylic acid, 6-(4,6-dimethyl-2-octenoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-acetate;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-decanoate;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-octanoate;
[1S-[1α(4R*,5S*)3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-pentanoate;
[1S-[1α(4R*,5S*)3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(7-phenylheptanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(4-cyclohexylbutanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(cyclohexanecarboxylate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-[1-(tricyclo[3.3.1.$1^{3,7}$]decyl)acetate];
[1S-[4α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-benzoate;
[1S-[1α(4R*,5S*),3α,4β,5α,6α(2E),7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(3-phenyl-2-propenoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(5-oxo-hexanoate);
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-(6-oxo-heptanoate);
[1S-[1α(4R*,5S*),3α,4β,5α,6α,7β]] 1-(4-acetyloxy-5-methyl-3-methylene-6-phenylhexyl)-4,7-dihydroxy-6-methoxy-2,8-dioxabicyclo[3.2.1]octane-3,4,5-tricarboxylic acid;
[1S-[1α(4R*,5S*),3α,4β,5α,6α,7β]] 1-(4-acetyloxy-5-methyl-3-methylene-6-phenylhexyl)-4,6,7-trihydroxy-2,8-dioxabicyclo[3.2.1]octane-3,4,5-tricarboxylic acid, 6-[(E/Z)-3-octenoate];
and physiologically acceptable salts thereof.

**11.** [1S-[1α(4R*,5S*),3α,4β.5α,6α,7β]] 1-(4-acetyloxy-5-methyl-3-methylene-6-phenylhexyl)-4,7-dihydroxy-6-[(nonoxycarbonyl)oxy]-2,8-dioxabicyclo[3.2.1]octane-3,4,5-tricarboxylic acid; [1S-[1α(4R*,5S*)-3α,4β,5α,6α,7β]] 1-(4-acetyloxy-5-methyl-3-methylene-6-phenylhexyl)-6-[(butoxycarbonyl)oxy]-4,7-dihydroxy-2,8-dioxabicyclo[3.2.1]octane-3,4,5-tricarboxylic acid; and physiologically acceptable salts thereof.

**12.** A compound according to any preceding claim for use in therapy.

**13.** A compound according to any preceding claim for use in the treatment of conditions where a lowering of the level of blood plasma cholesterol in animals, including humans, would be beneficial.

**14.** A compound according to any of Claims 1 to 12 for use in the treatment of fungal infections in a human or non-human animal patient.

**15.** A method of treatment of the human or non-human animal body to combat diseases associated with hypercholesterolemia and/or hyperlipoproteinemia or to combat fungal diseases, which method comprises administering to said body an effective amount of a compound as claimed in any of Claims 1 to 12 which inhibits squalene synthase.

**16.** A pharmaceutical composition comprising a compound according to any of Claims 1 to 12 together with one or more carriers and/or excipients.

**17.** A pharmaceutical composition comprising an active amount of a compound as claimed in any of Claims 1 to 12 for use in the treatment of conditions where a lowering of the level of blood plasma cholesterol in animals, including humans, would be beneficial.

**18.** A pharmaceutical composition comprising an active amount of a compound as claimed in any of Claims 1 to 12 for use in the treatment of fungal infections in a human or non-human animal patient.

**19.** A pharmaceutical composition according to any one of Claims 16 to 18 in a form suitable for oral, buccal, topical, parenteral, implant, rectal, ophthalmic or genito-urinary administration or in a form suitable for administration by inhalation or insufflation.

**20.** A pharmaceutical composition according to any one of Claims 16 to 19 in unit dosage form.

**21.** Use of a compound according to any of Claims 1 to 12 in the manufacture of a medicament for the treatment of hypercholesterolemia and/or hyperlipoproteinemia in a human or non-human animal patient.

**22.** Use of a compound according to any of Claims 1 to 12 in the manufacture of a medicament for the treatment of fungal infections in a human or non-human animal patient.

**23.** A process for the preparation of a compound as claimed in Claim 1 which comprises :

(A) (in the preparation of compounds of formula (I) in which $R^3$ represents a group $-CH=CR^8CR^9R^{10}CHR^{11}(CH_2)_mPh$ or a group $-CH_2CR^{12}=CR^{11}CR^9R^{10}CHR^{11}(CH_2)_nPh$ where $R^8$ is hydrogen or $C_{1-4}$alkyl) reacting a compound of formula (II)

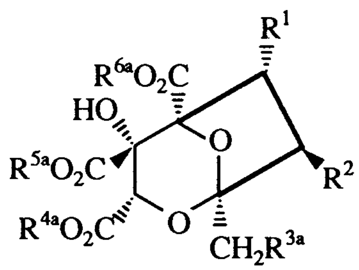

(II)

(wherein $R^{3a}$ represents CHO or $CH_2COR^{12}$ as appropriate, $R^1$ and $R^2$ are as defined in Claim 1 and $R^{4a}$, $R^{5a}$ and $R^{6a}$ are as defined for $R^4$, $R^5$ and $R^6$ in Claim 1 or are protecting groups) with a compound of formula (IIIa) or (IIIb)

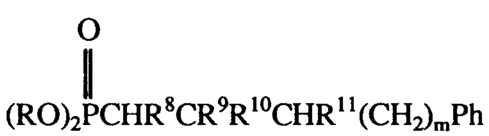

(IIIa)

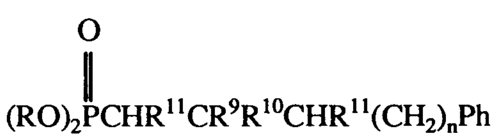

(IIIb)

as appropriate (wherein $R^{11}$ is as defined in Claim 1, $R^8$ is hydrogen or $C_{1-4}$alkyl, $CR^9R^{10}$ forms a group C=0 and R represents a $C_{1-6}$alkyl group or an aryl group), and thereafter removing any protecting groups present;

(B) converting a compound of formula (I) or a protected derivative thereof to a different compound of formula (I) or a protected derivative thereof, followed, if necessary, by the removal of any protecting groups present;

(C) (in the preparation of compounds of formula (I) in which $R^2$ represents a group $-OCOR^7$ or $-OCO_2R^7$) reacting a compound of formula (IX)

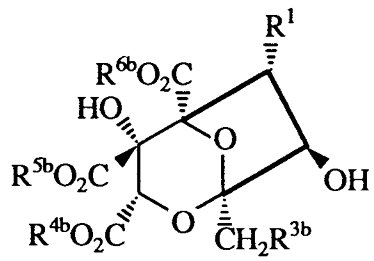

(IX)

(wherein $R^1$ is as defined in Claim 1, $R^{3b}$ is as defined above and $R^{4b}$, $R^{5b}$ and $R^{6b}$ are protecting groups) under conditions for ester or carbonate formation, followed by removal of the protecting groups present; or

(D) (in the preparation of compounds of formula (I) in which $R^1$ represents an acyloxy, carbonate, carbamate or ether group) reacting a compound of formula (XI)

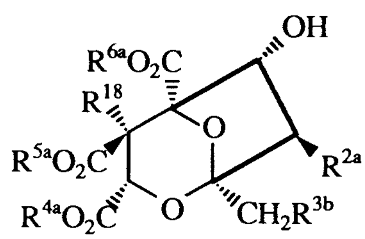

(XI)

(wherein $R^{2a}$ is as defined for $R^2$ in Claim 1 or is a protected hydroxyl group, $R^{3b}$ is as defined for $R^3$ in Claim 1 or is a protected derivative thereof, $R^{4a}$-$R^{6a}$ are as defined above and $R^{18}$ is a hydroxyl group or a protected hydroxyl group) to convert the 6 position hydroxyl group to an acyloxy, carbonate, carbamate or ether group, followed by removal of any protecting groups present.

**24.** Compounds of formulae (II), (IV), (V), IX) and (XV).

**25.** Compounds according to any of Claims 1 to 12 substantially as herein described.

**26.** Compositions according to any one of Claims 16 to 20 substantially as herein described.

European Patent Office

# PARTIAL EUROPEAN SEARCH REPORT

which under Rule 45 of the European Patent Convention shall be considered, for the purposes of subsequent proceedings, as the European search report

Application Number

EP 92 10 0095

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | CH-A- 606 071 (CIBA-GEIGY)(13-10-1978) * Column 1 - column 2, line 23 * --- | 1,13,14,21 | C 07 D 493/08<br>A 61 K 31/34<br>C 12 P 17/18<br>C 07 H 19/01<br>C 12 P 19/02<br>C 07 C 57/03<br>A 61 K 31/71 //<br>(C 07 D 493/08<br>C 07 D 319:00<br>C 07 D 307:00 ) |
| A | EP-A-0 259 087 (MERCK)(09-03-1988) * Claims 1,9 * --- | 1,13,14,21 | |
| P,A | US-A-5 026 554 (K.F. BARTIZAL et al.)(25-06-1991) * Column 1, line 50 - column 4, line 19 * ----- | 1,13,14,21 | |

TECHNICAL FIELDS SEARCHED (Int. Cl.5)

C 07 D 493/00
C 12 P 17/00
C 07 H 19/00
C 12 P 19/00

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

Remark: Although claim 15 is directed to a method of treatment of the human/animal body (Art. 52(4) EPC) the search has been carried out and based on the alleged effects of the compound/composition.

| Place of search | Date of completion of the search | Examiner |
|---|---|---|
| THE HAGUE | 30-03-1992 | HEYWOOD C.J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0407)